(19) Europäisches Patentamt
European Patent Office
Office européen des brevets

(11) EP 0 868 202 B1

(12) EUROPÄISCHE PATENTSCHRIFT

(45) Veröffentlichungstag und Bekanntmachung des Hinweises auf die Patenterteilung:
**28.08.2002 Patentblatt 2002/35**

(21) Anmeldenummer: **96941055.4**

(22) Anmeldetag: **29.11.1996**

(51) Int Cl.[7]: **A61K 49/00**, A61K 49/04

(86) Internationale Anmeldenummer:
**PCT/EP96/05315**

(87) Internationale Veröffentlichungsnummer:
**WO 97/023245 (03.07.1997 Gazette 1997/29)**

(54) **KASKADEN-POLYMER-KOMPLEXE, VERFAHREN ZU IHRER HERSTELLUNG UND DIESE ENTHALTENDE PHARMAZEUTISCHE MITTEL**

CASCADE POLYMER COMPLEXES, PROCESS FOR PRODUCING THE SAME AND PHARMACEUTICALS CONTAINING THE SAME

COMPLEXES POLYMERES EN CASCADE, LEUR PROCEDE DE PREPARATION ET AGENTS PHARMACEUTIQUES LES CONTENANT

(84) Benannte Vertragsstaaten:
**AT BE CH DE DK ES FI FR GB GR IE IT LI LU MC NL PT SE**

(30) Priorität: **22.12.1995 DE 19549286**

(43) Veröffentlichungstag der Anmeldung:
**07.10.1998 Patentblatt 1998/41**

(73) Patentinhaber: **SCHERING AKTIENGESELLSCHAFT**
**13353 Berlin (DE)**

(72) Erfinder:
- **SCHMITT-WILLICH, Heribert**
  **D-12161 Berlin (DE)**
- **PLATZEK, Johannes**
  **D-12621 Berlin (DE)**
- **RADÜCHEL, Bernd**
  **D-13465 Berlin (DE)**
- **WEINMANN, Hanns-Joachim**
  **D-14129 Berlin (DE)**
- **EBERT, Wolfgang**
  **D-12203 Berlin (DE)**
- **MISSELWITZ, Bernd**
  **D-13439 Berlin (DE)**
- **MÜHLER, Andreas**
  **D-15366 Neuenhagen (DE)**
- **FRENZEL, Thomas**
  **D-12247 Berlin (DE)**

(56) Entgegenhaltungen:

| | |
|---|---|
| **EP-A- 0 430 863** | **WO-A-95/24225** |
| **WO-A-95/28966** | **DE-A- 4 344 460** |
| **DE-A- 4 425 857** | **US-A- 5 527 524** |

- **MAGNETIC RESONANCE IN MEDICINE, Bd. 31, Nr. 1, 1.Januar 1994, Seiten 1-8, XP000423671 WIENER E C ET AL: "DENDRIMER-BASED METAL CHELATES: A NEW CLASS OF MAGNETIC RESONANCE IMAGING CONTRAST AGENTS"**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft den in den Patentansprüchen gekennzeichneten Gegenstand, das heißt neue Kaskaden-Polymer-Komplexe, diese Verbindungen enthaltende Mittel, die Verwendung der Komplexe in der Diagnostik und Therapie sowie Verfahren zur Herstellung dieser Verbindungen und Mittel.

Die zur Zeit klinisch eingesetzten Kontrastmittel für die modernen bildgebenden Verfahren Kernspintomographie (MRI) und Computertomographie (CT) [Magnevist ®, Pro Hance ®, Ultravist® und Omniscan ®] verteilen sich im gesamten extrazellulären Raum des Körpers (Intravasalraum und Interstitium). Dieser Verteilungsraum umfaßt etwa 20 % des Körpervolumens.

Extrazelluläre MRI-Kontrastmittel sind klinisch zuerst erfolgreich bei der Diagnostik von zerebralen und spinalen Krankheitsprozessen eingesetzt worden, da sich hier eine ganz besondere Situation hinsichtlich des regionalen Verteilungsraumes ergibt. Im Gehirn und im Rückenmark können extrazelluläre Kontrastmittel im gesunden Gewebe aufgrund der Blut-Hirn-Schranke nicht den Intravasalraum verlassen. Bei krankhaften Prozessen mit Störung der Blut-Hirn-Schranke (z.B. maligne Tumoren, Entzündungen, demyelinisierende Erkrankungen etc.) entstehen innerhalb des Hirns dann Regionen mit erhöhter Blutgefäß-Durchlässigkeit (Permeabilität) für diese extrazellulären Kontrastmittel (Schmiedl et al., MRI of blood-brain barrier permeability in astrocytic gliomas: application of small and large molecular weight contrast media, Magn. Reson. Med. 22: 288, 1991). Durch das Ausnutzen dieser Störung der Gefäßpermeabilität kann erkranktes Gewebe mit hohem Kontrast gegenüber dem gesunden Gewebe erkannt werden.

Außerhalb des Gehirns und des Rückenmarkes gibt es allerdings eine solche Permeabilitätsbarriere für die oben genannten Kontrastmittel nicht (Canty et al., First-pass entry of nonionic contrast agent into the myocardial extravascular space. Effects on radiographic estimate of transit time and blood volume. Circulation 84: 2071, 1991). Damit ist die Anreicherung des Kontrastmittels nicht mehr abhängig von der Gefäßpermeabilität, sondern nur noch von der Größe des extrazellulären Raumes im entsprechenden Gewebe. Eine Abgrenzung der Gefäße gegenüber dem umliegenden interstitiellen Raum bei Anwendung dieser Kontrastmittel ist nicht möglich.

Besonders für die Darstellung von Gefäßen wäre ein Kontrastmittel wünschenswert, das sich ausschließlich im vasalen Raum (Gefäßraum) verteilt. Ein solches blood-pool-agent soll es ermöglichen, mit Hilfe der Kernspintomographie gut durchblutetes von schlecht durchblutetem Gewebe abzugrenzen und somit eine Ischämie zu diagnostizieren. Auch infarziertes Gewebe ließe sich aufgrund seiner Anämie vom umliegenden gesunden oder ischämischen Gewebe abgrenzen, wenn ein vasales Kontrastmittel angewandt wird. Dies ist von besonderer Bedeutung, wenn es z.B. darum geht, einen Herzinfarkt von einer Ischämie zu unterscheiden.

Bisher müssen sich die meisten der Patienten, bei denen Verdacht auf eine kardiovaskuläre Erkrankung besteht (diese Erkrankung ist die häufigste Todesursache in den westlichen Industrieländern), invasiven diagnostischen Untersuchungen unterziehen. In der Angiographie wird zur Zeit vor allem die Röntgen-Diagnostik mit Hilfe von jodhaltigen Kontrastmitteln angewandt. Diese Untersuchungen sind mit verschiedenen Nachteilen behaftet: sie sind mit dem Risiko der Strahlenbelastung verbunden, sowie mit Unannehmlichkeiten und Belastungen, die vor allem daher kommen, daß die jodhaltigen Kontrastmittel, verglichen mit NMR-Kontrastmitteln, in sehr viel höherer Konzentration angewandt werden müssen.

Es besteht daher ein Bedarf an NMR-Kontrastmitteln, die den vasalen Raum markieren können (blood-pool-agent). Diese Verbindungen sollen sich durch eine gute Verträglichkeit und durch eine hohe Wirksamkeit (hohe Steigerung der Signalintensität bei MRI) auszeichnen.

Der Ansatz, zumindest einen Teil dieser Probleme durch Verwendung von Komplexbildnern, die an Makro- oder Biomoleküle gebunden sind, zu lösen, war bisher nur sehr begrenzt erfolgreich.

So ist beispielsweise die Anzahl paramagnetischer Zentren in den Komplexen, die in den Europäischen Patentanmeldungen Nr. 0 088 695 und Nr. 0 150 844 beschrieben sind, für eine zufriedenstellende Bildgebung nicht ausreichend.

Erhöht man die Anzahl der benötigten Metallionen durch mehrfache Einführung komplexierender Einheiten in ein makromolekulares Biomolekül, so ist das mit einer nicht tolerierbaren Beeinträchtigung der Affinität und/oder Spezifizität dieses Biomoleküls verbunden [J. Nucl. Med. 24, 1158 (1983)].

Makromoleküle können generell als Kontrastmittel für die Angiographie geeignet sein. Albumin-GdDTPA (Radiology 1987; 162: 205) z.B. zeigt jedoch 24 Stunden nach intravenöser Injektion bei der Ratte eine Anreicherung im Lebergewebe, die fast 30 % der Dosis ausmacht. Außerdem werden in 24 Stunden nur 20 % der Dosis eliminiert.

Das Makromolekül Polylysin-GdDTPA (Europäische Patentanmeldung, Publikations-Nr. 0 233 619) erwies sich ebenfalls geeignet als blood-pool-agent. Diese Verbindung besteht jedoch herstellungsbedingt aus einem Gemisch von Molekülen verschiedener Größe. Bei Ausscheidungsversuchen bei der Ratte konnte gezeigt werden, daß dieses Makromolekül unverändert durch glomeruläre Filtration über die Niere ausgeschieden wird. Synthesebedingt kann Polylysin-GdDTPA aber auch Makromoleküle enthalten, die so groß sind, daß sie bei der glomerulären Filtration die Kapillaren der Niere nicht passieren können und somit im Körper zurückbleiben.

Auch makromolekulare Kontrastmittel auf der Basis von Kohlenhydraten, z.B. Dextran, sind beschrieben worden (Europäische Patentanmeldung, Publikations-Nr. 0 326 226). Der Nachteil dieser Verbindungen liegt darin, daß diese in der Regel nur ca. 5 % des signalverstärkenden paramagnetischen Kations tragen.

Die in der Europäischen Patentanmeldung Nr. 0 430 863 beschriebenen Polymere stellen bereits einen Schritt auf dem Wege zu blood-pool-agents dar, da sie nicht mehr die für die vorher erwähnten Polymere charakteristische Heterogenität bezüglich Größe und Molmasse aufweisen. Sie lassen jedoch immer noch Wünsche im Hinblick auf vollständige Ausscheidung, Verträglichkeit und/oder Wirksamkeit offen.

Es bestand daher die Aufgabe, neue diagnostische Mittel vor allem zur Erkennung und Lokalisierung von Gefäßkrankheiten, die die genannten Nachteile nicht besitzen, zur Verfügung zu stellen. Diese Aufgabe wird durch die vorliegende Erfindung gelöst.

Es wurde gefunden, daß sich Komplexe, die aus stickstoffhaltigen, mit komplexbildenden Liganden versehenen Kaskaden-Polymeren, mindestens 16 Ionen eines Elements der Ordnungszahlen 20 - 29, 39, 42, 44 oder 57 - 83 sowie gegebenenfalls Kationen anorganischer und/oder organischer Basen, Aminosäuren oder Aminosäureamide bestehen und gegebenenfalls acylierte Aminogruppen enthalten, überraschenderweise hervorragend zur Herstellung von NMR- und Röntgen-Diagnostika ohne die genannten Nachteile aufzuweisen, eignen.

Die erfindungsgemäßen komplexbildenden Kaskaden-Polymere lassen sich durch die allgemeine Formel I beschreiben

$$A\text{-}\{X\text{-}[Y\text{-}(Z\text{-}\langle W\text{-}K_w\rangle_z)_y]_x\}_a \qquad (I),$$

worin

| | |
|---|---|
| A | für einen stickstoffhaltigen Kaskadenkern der Basismultiplizität a, |
| X und Y | unabhängig voneinander fiir eine direkte Bindung oder eine Kaskadenreproduktionseinheit der Reproduktionsmultiplizität x bzw. y, |
| Z und W | unabhängig voneinander für eine Kaskadenreproduktionseinheit der Reproduktionsmultiplizität z bzw. w, |
| K | für den Rest eines Komplexbildners, |
| a | für die Ziffern 2 bis 12, |
| x, y, z und w | unabhängig voneinander für die Ziffern 1 bis 4 stehen. mit der Maßgabe, daß mindestens zwei Reproduktionseinheiten unterschiedlich sind, daß für das Produkt der Multiplizitäten gilt $16 \leq a \cdot x \cdot y \cdot z \cdot w \leq 64$ und daß mindestens eine der Kaskadenreproduktionseinheiten X, Y, Z, W für eine 1,4,7,10-Tetraazacyclododecan- oder 1,4,8,11-Tetraazacyclotetradecan-Reproduktionseinheit steht. |

Als Kaskadenkern A sind geeignet:
Stickstoffatom,

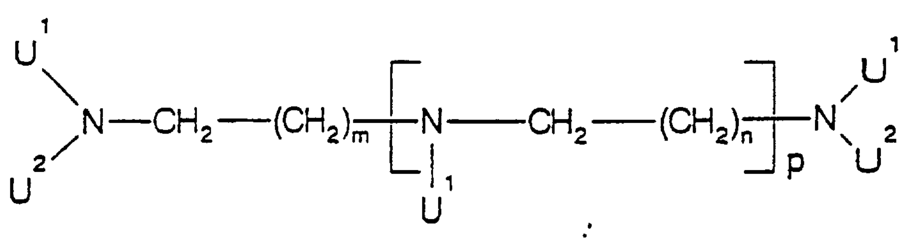

,

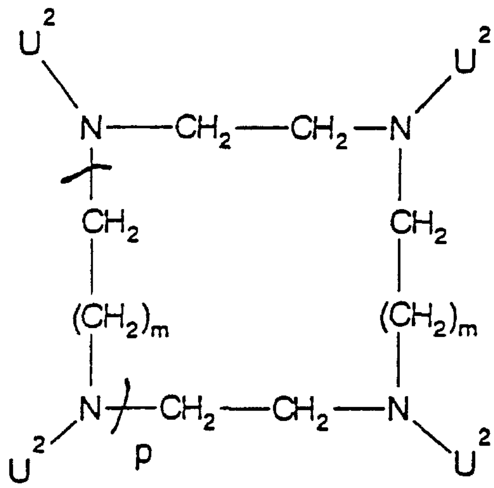

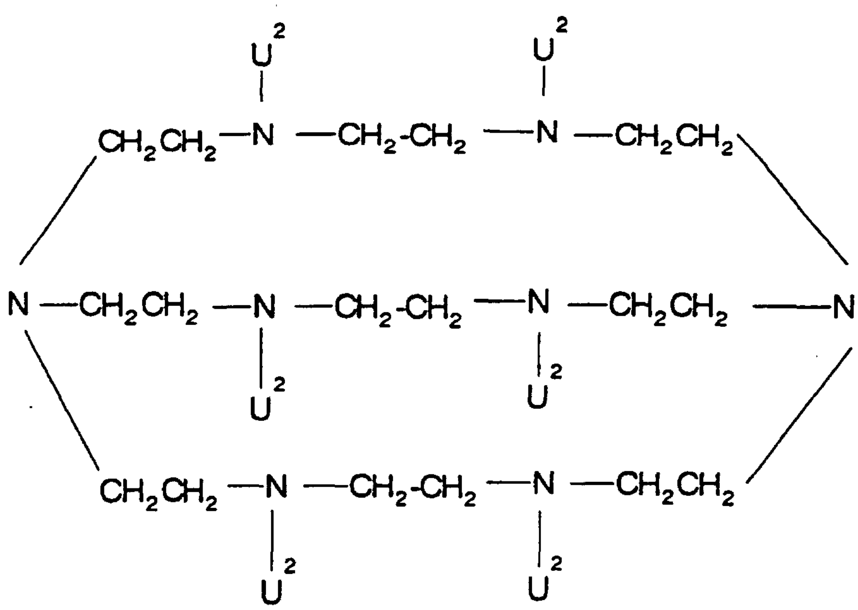

,

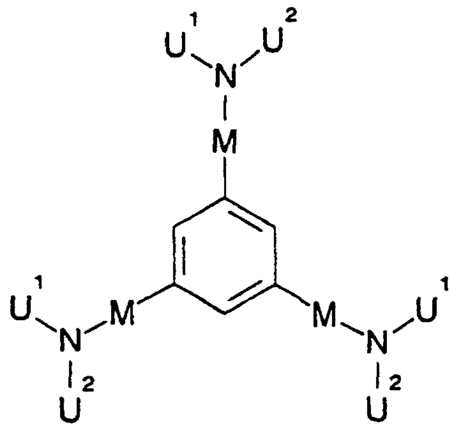

,

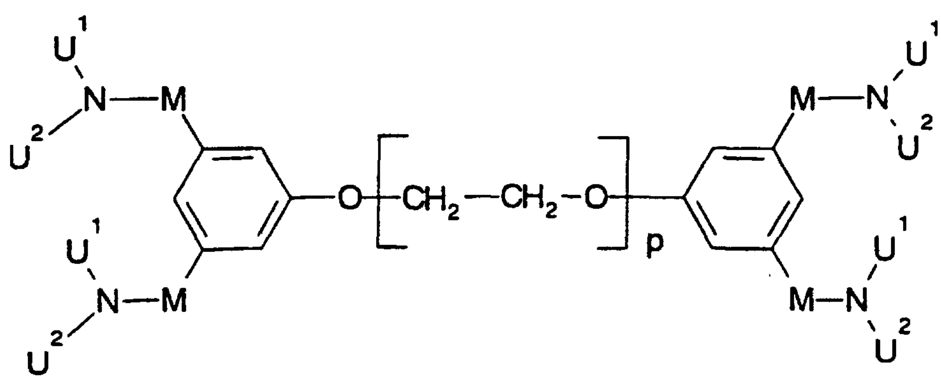

,

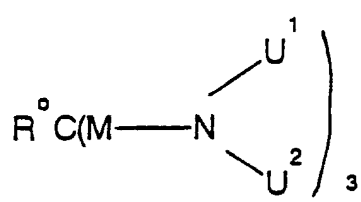

,

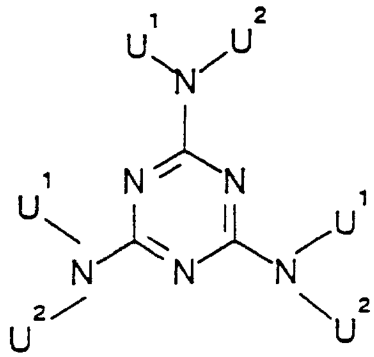

,

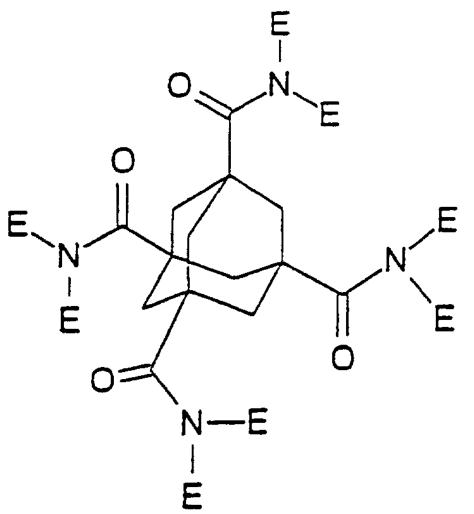

,

worin

| | |
|---|---|
| m und n | für die Ziffern 1 bis 10, |
| p | für die Ziffern 0 bis 10, |
| $U^1$ | für $Q^1$ oder E, |
| $U^2$ | für $Q^2$ oder E mit |

E in der Bedeutung der Gruppe

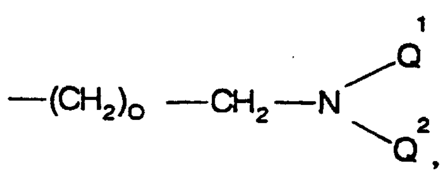

,

wobei

| | |
|---|---|
| o | für die Ziffern 1 bis 6, |
| $Q^1$ | für ein Wasserstoffatom oder $Q^2$ und |

$Q^2$ für eine direkte Bindung

M für eine $C_1$-$C_{10}$-Alkylenkette, die gegebenenfalls durch 1 bis 3 Sauerstoffatome unterbrochen ist und/oder gegebenenfalls mit 1 bis 2 Oxogruppen substituiert ist,

$R^o$ für einen verzweigten oder unverzweigten $C_1$-$C_{10}$-Alkylrest, eine Nitro-, Amino-, Carbonsäuregruppe oder für

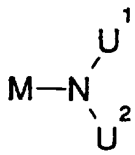

stehen,

wobei die Anzahl $Q^2$ der Basismultiplizität a entspricht.

Den einfachsten Fall eines Kaskadenkerns stellt das Stickstoffatom dar, dessen drei Bindungen (Basismultiplizität a = 3) in einer ersten "inneren Schicht" (Generation 1) von drei Reproduktionseinheiten X bzw. Y (wenn X für eine direkte Bindung steht) bzw. Z (wenn X und Y jeweils für eine direkte Bindung stehen) besetzt sind; anders formuliert: die drei Wasserstoffatome des zugrundeliegenden Kaskadenstarters Ammoniak $A(H)_a = NH_3$ sind durch drei Reproduktionseinheiten X bzw. Y bzw. Z substituiert worden. Die im Kaskadenkem A enthaltene Anzahl $Q^2$ gibt dabei die Basismulitplizität a wieder.

Die Reproduktionseinheiten X, Y, Z und W enthalten $-NQ^1Q^2$-Gruppen, worin $Q^1$ ein Wasserstoffatom oder $Q^2$ und $Q^2$ eine direkte Bindung bedeuten. Die in der jeweiligen Reproduktionseinheit (z.B. X) enthaltene Anzahl $Q^2$ entspricht der Reproduktionsmultiplizität dieser Einheit (z.B. x im Falle von X). Das Produkt aller Multiplizitäten $a \cdot x \cdot y \cdot z \cdot w$ gibt die Anzahl der im Kaskadenpolymeren gebundenen Komplexbildner-Reste K an. Die erfindungsgemäßen Polymere enthalten mindestens 16 und höchstens 64 Reste K im Molekül, die jeweils ein bis maximal drei (im Falle von zweiwertigen Ionen), vorzugsweise ein Ion, eines Elements der oben genannten Ordnungszahlen binden können.

Die letzte Generation, d.h. die an die Komplexbildner-Reste K gebundene Reproduktionseinheit W ist über NH-Gruppen ($-NQ^1Q^2$ mit Q1 in der Bedeutung eines Wasserstoffatoms und $Q^2$ = direkte Bindung) an K gebunden, während die vorangehenden Reproduktionseinheiten sowohl über $NHQ^2$-Gruppen (z.B. durch Acylierungsreaktionen) als auch über $NQ^2Q^2$-Gruppen (z.B. durch Alkylierungsreaktionen) miteinander verknüpft sein können.

Die erfindungsgemäßen Kaskaden-Polymer-Komplexe weisen maximal 10 Generationen auf (d.h. es können auch mehr als jeweils nur eine der Reproduktionseinheiten X, Y und Z im Molekül vorhanden sein), vorzugsweise jedoch 2 bis 4 Generationen, wobei mindestens zwei der Reproduktionseinheiten im Molekül unterschiedlich sind.

Als bevorzugte Kaskadenkerne A seien diejenigen angeführt, die unter die oben genannten allgemeinen Formeln fallen, wenn

m für die Ziffern 1 - 3, besonders bevorzugt für die Ziffer 1,
n für die Ziffern 1 - 3, besonders bevorzugt für die Ziffer 1,
p für die Ziffern 0 - 3, besonders bevorzugt für die Ziffern 1 und 3,
o für die Ziffern 1 - 2, besonders bevorzugt für die Ziffer 1,
M für eine $-CH_2$-, -CO- oder $-CH_2CO$-Gruppe und
$R^o$ für eine $-CH_2NU^1U^2$-, $CH_3$- oder $NO_2$-Gruppe

steht.

Bevorzugt sind weiterhin Kaskadenkerne A, die unter die zweite und vierte der angegebenen acht der allgemeinen Formeln fallen, besonders diejenige der allgemeinen Formel

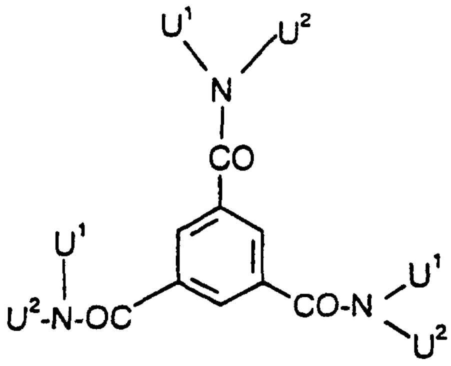 ,

worin

$U^1$ und $U^2$ für die Gruppe E mit o in der Bedeutung der Ziffern 1 oder 2 stehen.

Als weitere bevorzugte Kaskadenstarter $A(H)_a$ seien z.B. aufgeführt:
(In der Klammer wird die Basismultiplizität a angegeben für den Fall der zum Aufbau der nächsten Generation dienenden nachfolgenden Mono- bzw. Disubstitution)

| | |
|---|---|
| Tris(aminoethyl)amin | (a = 6 bzw. 3); |
| Tris(aminopropyl)amin | (a = 6 bzw. 3); |
| Diethylentriamin | (a = 5 bzw. 3); |
| Triethylentetramin | (a = 6 bzw. 4); |
| Tetraethylenpentamin | (a = 7 bzw. 5); |
| 1,3,5-Tris(aminomethyl)benzol | (a = 6 bzw. 3); |
| Trimesinsäuretriamid | (a = 6 bzw. 3); |
| 1,4,7-Triazacyclononan | (a = 3); |
| 1,4,7,10-Tetraazacyclododecan | (a = 4); |
| 1,4,7,10,13-Pentaazacyclopentadecan | (a = 5); |
| 1,4,8,11-Tetraazacyclotetradecan | (a = 4); |
| 1,4,7,10,13,16-Hexaazacyclooctadecan | (a = 6); |
| 1,4,7,10,13,16,19,22,25,28-Decaazacyclotriacontan | (a = 10); |
| Tetrakis(aminomethyl)methan | (a = 8 bzw. 4); |
| 1,1,1-Tris(aminomethyl)ethan | (a = 6 bzw. 3); |
| Tris(aminopropyl)-nitromethan | (a = 6 bzw. 3); |
| 2,4,6-Triamino-1,3,5-triazin | (a = 6 bzw. 3); |
| 1,3,5,7-Adamantantetracarbonsäureamid | (a = 8 bzw. 4); |
| 3,3',5,5'-Diphenylether-tetracarbonsäureamid | (a = 8 bzw. 4); |
| 1,2-Bis[Phenoxyethan]-3',3",5',5"-tetracarbonsäureamid | (a = 8 bzw. 4); |
| 1,4,7,10,13,16,21,24-Octaazabicyclo[8.8.8.]hexacosan | (a = 6). |

Es sei darauf hingewiesen, daß die Definition als Kaskadenkern A und damit die Trennung von Kaskadenkern und erster Reproduktionseinheit rein formal und damit unabhängig von dem tatsächlichen synthetischen Aufbau der gewünschten Kaskaden-Polymer-Komplexe gewählt werden kann. So kann man z.B. das in Beispiel 4 verwendete Tris(aminoethyl)-amin sowohl selbst als Kaskadenkern A (vergleiche die erste für A angegebene allgemeine Formel mit m = n = p = 1, $U^1$ = E mit o in der Bedeutung der Ziffer 1 und $U^1$ = $U^2$ = $Q^2$) aber auch als Stickstoffatom (= Kaskadenkern A), das als erste Generation drei Reproduktionseinheiten

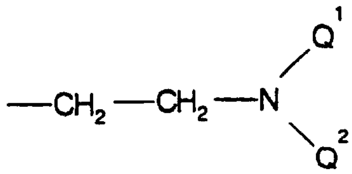

(vergleiche die Definition von E) aufweist, ansehen.

Die Kaskadenreproduktionseinheiten X, Y, Z und W werden unabhängig voneinander durch

E ,

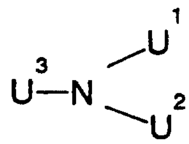 ,

bevorzugt

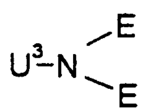 ,

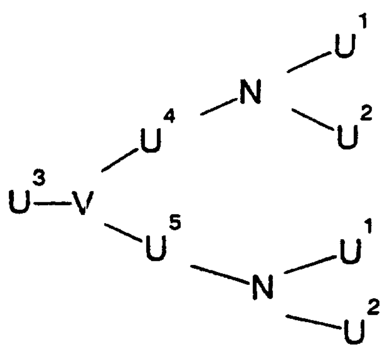 ,

bevotzugt

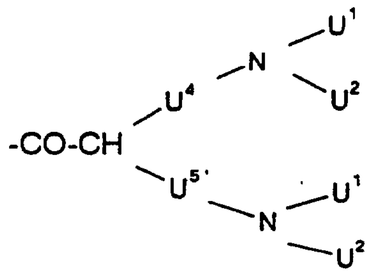

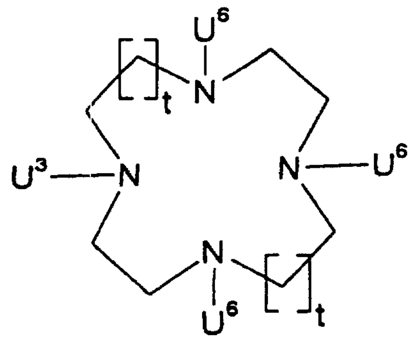

mit t in der Bedeutung der Ziffern 1 oder 2,

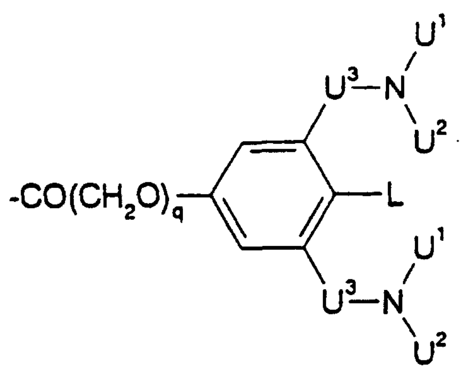

,

bevorzugt

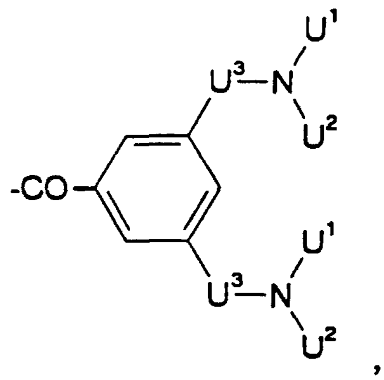

bestimmt,
worin

$U^1$ für $Q^1$ oder E,
$U^2$ für $Q^2$ oder E mit

E in der Bedeutung der Gruppe

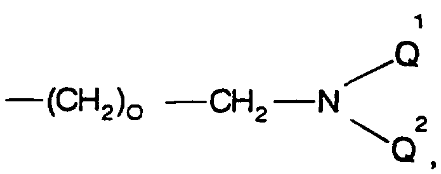

wobei

o für die Ziffern 1 bis 6, bevorzugt 1 bis 2,
$Q^1$ für ein Wasserstoffatom oder $Q^2$,
$Q^2$ für eine direkte Bindung,

$U^3$ für eine -NHCO-$(CH_2)_o$-Kette oder eine $C_1$-$C_{20}$-, bevorzugt $C_{1-10}$ Alkylenkette, die gegebenenfalls durch 1 bis 10, bevorzugt 1 bis 2, Sauerstoffatome und/oder 1 bis 2 -$N(CO)_q$-$R^2$-, 1 bis 2 Phenylen- und/oder 1 bis 2 Phenylenoxyreste unterbrochen ist und/oder gegebenenfalls durch 1 bis 2 Oxo-, Thioxo-, Carboxy-, $C_1$-$C_5$-Alkylcarboxy-, $C_1$-$C_5$-Alkoxy-, Hydroxy-, $C_1$-$C_5$-alkylgruppen substituiert ist, wobei

q für die Ziffern 0 oder 1 und
$R^2$ für ein Wasserstoffatom, einen Methyl- oder einen Ethylrest, der gegebenenfalls mit 1 - 2 Hydroxy- oder 1 Carboxygruppe(n) substituiert ist,
L für ein Wasserstoffatom oder die Gruppe

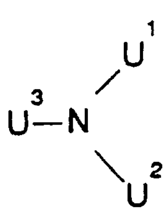

V für die Methingruppe

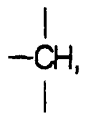

wenn gleichzeitig $U^4$ eine direkte Bindung oder die Gruppe M bedeutet und $U^5$ eine der Bedeutungen von $U^3$ besitzt oder

V für die Gruppe

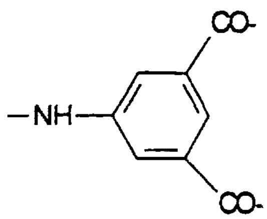

,

wenn gleichzeitig $U^4$ und $U^5$ identisch sind und die direkte Bindung oder die Gruppe M bedeuten,

U5' für die Gruppe M, und

$U^6$ für die Gruppe

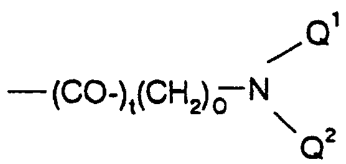

oder eine direkte Bindung stehen

mit der Maßgabe, daß mindestens eine der Kaskadenreproduktionseinheiten für die oben angegebene 1,4,7,10-Tetraazacyclododecan- oder 1,4,8,11-Tetraazacyclo tetradecan-Reproduktionseinheit steht. Bevorzugt ist hierbei die 1,4,7,10-Tetraazacyclododecan-Reproduktionseinheit.

Bevorzugte Kaskadenreproduktionseinheiten X, Y, Z und W sind diejenigen, bei denen in den oben genannten allgemeinen Formeln der Rest $U^3$ für

$-CO-$, $-COCH_2OCH_2CO-$, $-COCH_2-$, $-CH_2CH_2-$, $-CONHC_6H_4-$, $-NHCOCH_2-$, $-COCH_2CH_2CO-$, $-COCH_2-CH_2CH_2CO-$, $-COCH_2CH_2CH_2CH_2CO-$, $-CONHCH_2CH_2NHCOCH_2CH_2CO-$, $-COCH_2CH_2NHCOCH_2CH_2CO-$, der Rest $U^4$ für eine direkte Bindung, für $-CH_2CO-$, der Rest $U^5$ für eine direkte Bindung, für $-(CH_2)_4-$, $-CH_2CO-$, $-CH(COOH)-$, $CH_2OCH_2CH_2-$, $-CH_2C_6H_4-$, $CH_2-C_6H_4OCH_2CH_2-$,

der Rest E für eine Gruppe

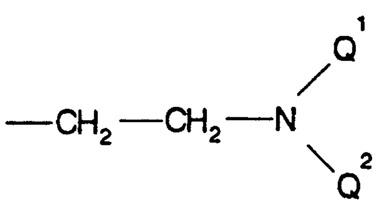

steht.

Als beispielhaft genannte Kaskadenreproduktionseinheiten X, Y, Z und W seien angeführt:

$-CH_2CH_2NH-$; $-CH_2CH_2N<$ ;

$-COCH(NH-)(CH_2)_4NH-$; $-COCH(N<)(CH_2)_4N<$ ;

$-COCH_2OCH_2CON(CH_2CH_2NH-)_2$; $-COCH_2OCH_2CON(CH_2CH_2N< )_2$ ;
$-COCH_2N(CH_2CH_2NH-)_2$; $-COCH_2N(CH_2CH_2N< )_2$;
$-COCH_2NH-$; $-COCH_2N<$ ;
$-COCH_2CH_2CON(CH_2CH_2NH-)_2$; $-COCH_2CH_2CON(CH_2CH_2N< )_2$ ;
$-COCH_2OCH_2CONH-C_6H_4-CH[CH_2CON(CH_2CH_2NH-)_2]_2$;
$-COCH_2OCH_2CONH-C_6H_4-CH[CH_2CON(CH_2CH_2N< )_2]_2$ ;
$-COCH_2CH_2CO-NH-C_6H_4-CH[CH_2CON(CH_2CH_2NH-)_2]_2$;
$-COCH_2CH_2CO-NH-C_6H_4-CH[CH_2CON(CH_2CH_2N<)_2]_2$;
$-CONH-C_6H_4-CH(CH_2CON(CH_2CH_2NH-)_2]_2$;
$-CONH-C_6H_4-CH[CH_2CON(CH_2CH_2N<)_2]_2$;
$-COCH(NH-)CH(COOH)NH-$; $-COCH(N< )CH(COOH)N<$ ;

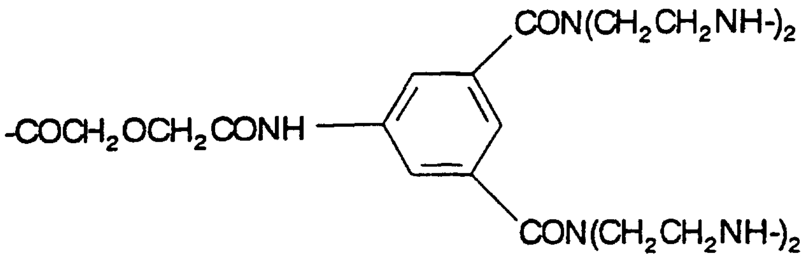
;

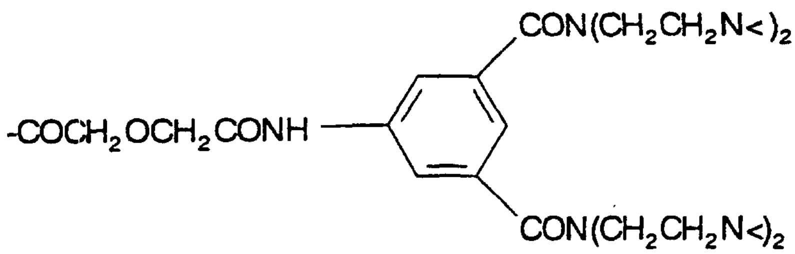
;

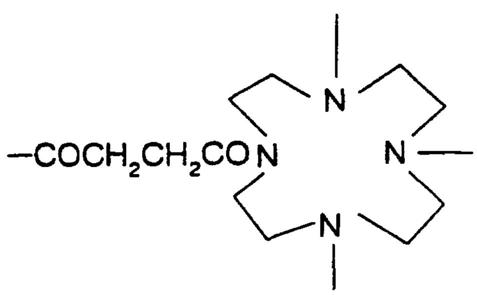
;
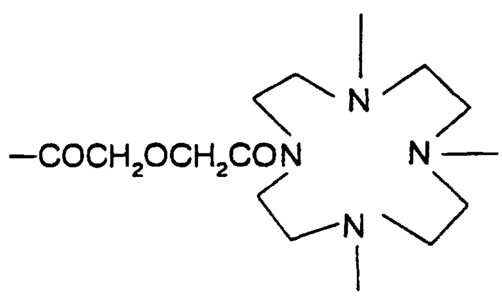
;

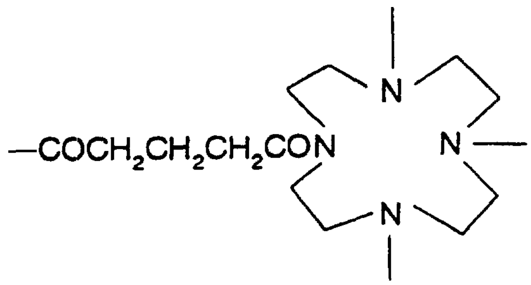

,

$-COCH(CH_2CH_2CH_2-N\langle)_2$; $-COCH(CH_2CH_2-N\langle)_2$;

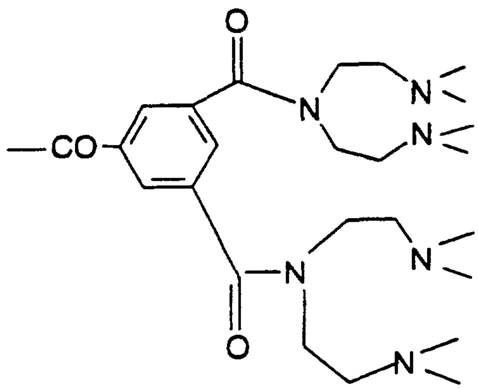

;

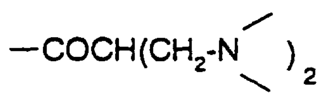

;

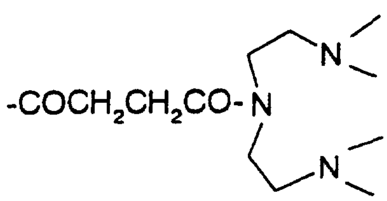

;

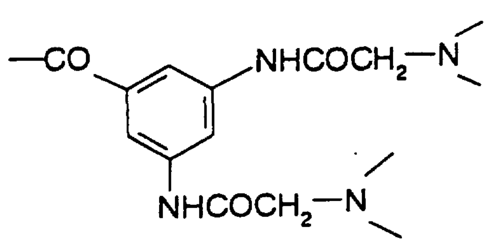

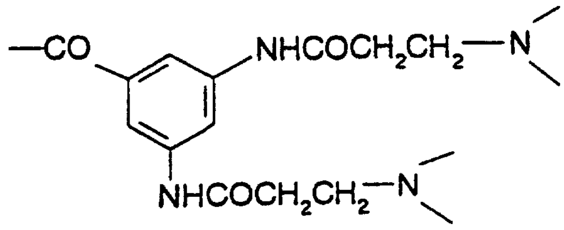

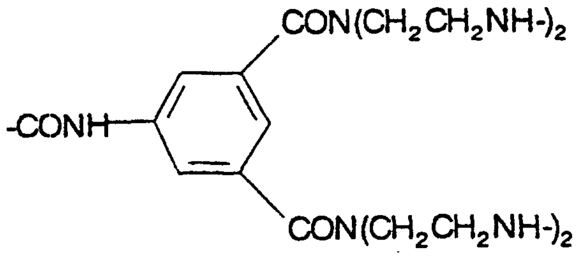

;

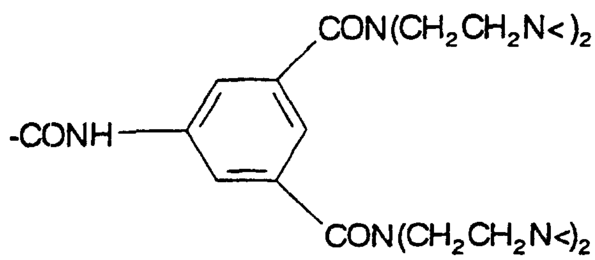

;

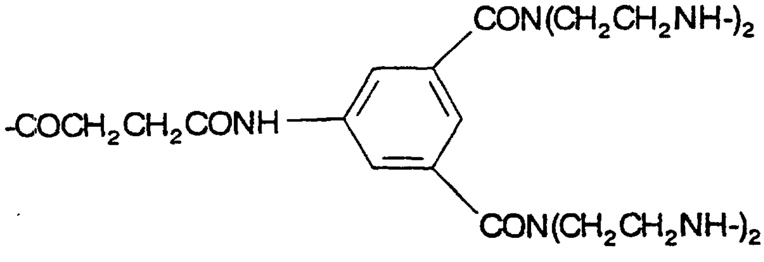

;

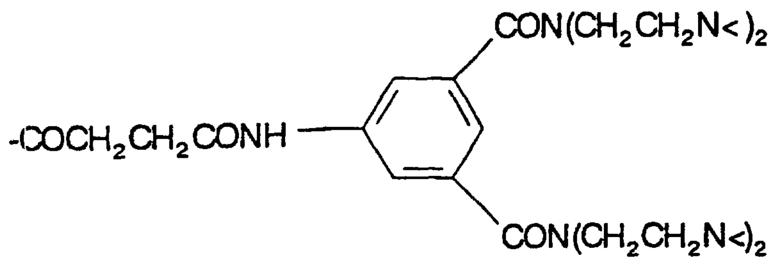

;

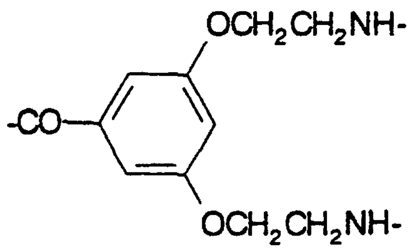

;

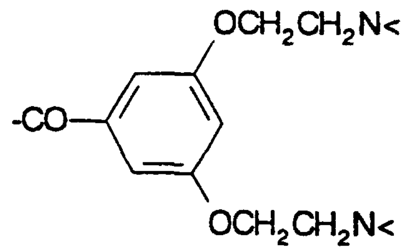

;

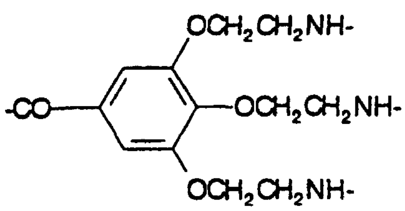

;

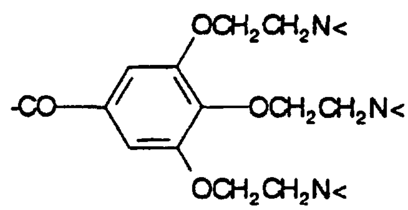

;

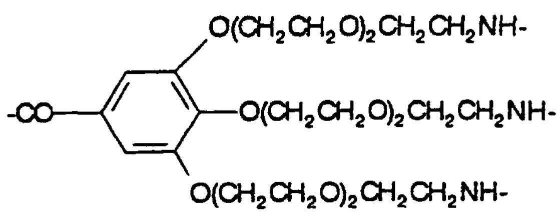

;

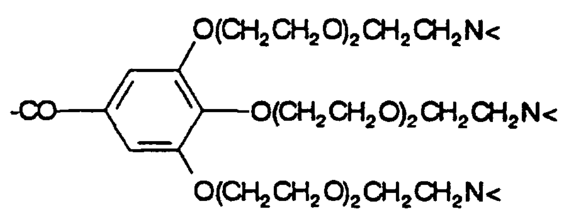

;

Die Komplexbildner-Reste K werden durch die allgemeinen Formeln IA, IB und IC beschrieben:

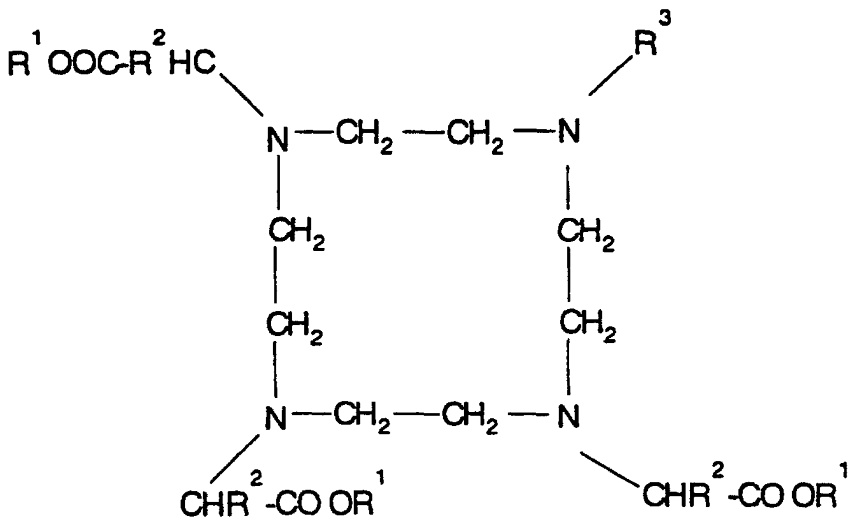

(IA),

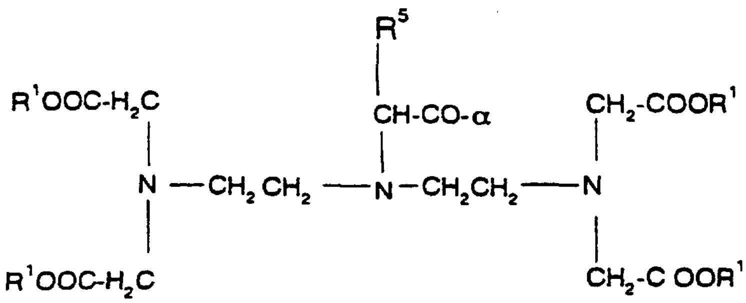

(IB) ,

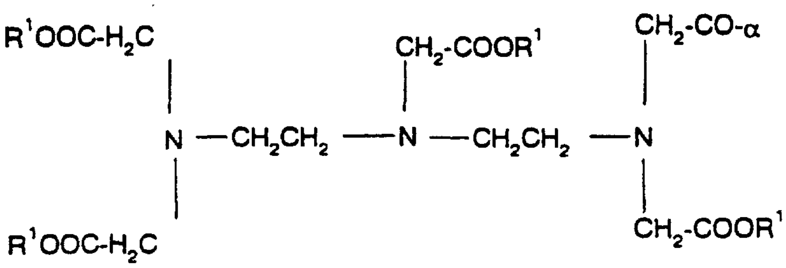

(IC) ,

steht, worin

$R^1$ unabhängig voneinander für ein Wasserstoffatom oder ein Metallionenäquivalent der Ordnungszahlen 20 - 29, 39, 42 - 44 oder 57 - 83,

$R^2$ für ein Wasserstoffatom, einen Methyl- oder einen Ethylrest, der gegebenenfalls mit 1 - 2 Hydroxy- oder 1 Carboxygruppe(n) substituiert ist,

$R^3$ für eine

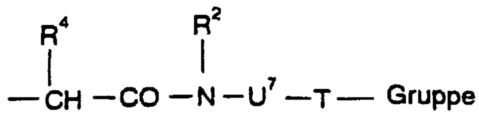

oder eine

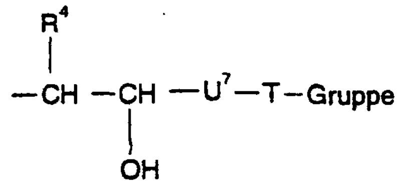

,

$R^4$ für ein Wasserstoffatom oder eine geradkettige, verzweigte, gesättigte oder ungesättigte $C_1$-$C_{30}$-Alkylkette, die gegebenenfalls durch 1 - 10 Sauerstoff atome, 1 Phenylen-, 1 Phenylenoxygruppen unterbrochen und/oder gegebenenfalls durch 1 - 5 Hydroxy-, 1 - 3 Carboxy-, 1 Phenylgruppe(n) substituiert ist,

$R^5$ für ein Wasserstoffatom oder für $R^4$,

$U^7$ für eine gegebenenfalls 1 - 5 Imino-, 1 - 3 Phenylen-, 1 - 3 Phenylenoxy-, 1 - 3 Phenylenimino-, 1 - 5 Amid-, 1 - 2 Hydrazid-, 1 - 5 Carbonyl-, 1 - 5 Ethylenoxy-, 1 Harnstoff-, 1-Thioharnstoff-, 1 - 2 Carboxyalkyl imino-, 1 - 2 Estergruppen, 1 - 10 Sauerstoff-, 1 - 5 Schwefel- und/oder 1 - 5 Stickstoff-Atom(e) enthaltende und/oder gegebenenfalls durch 1 - 5 Hydroxy-, 1 - 2 Mercapto-, 1 - 5 Oxo-, 1 - 5 Thioxo-, 1 - 3 Carboxy-, 1 - 5 Carboxyalkyl-, 1 - 5 Ester- und/oder 1 - 3 Aminogruppe(n) substituierte geradkettige, verzweigte, gesättigte oder ungesättigte $C_1$-$C_{20}$-Alkylengruppe, wobei die gegebenenfalls enthaltenden Phenylengruppen durch 1 - 2 Carboxy-, 1 - 2 Sulfon- oder 1 - 2 Hydroxygruppen substituiert sein können,

T für eine -CO-$\alpha$, -NHCO-$\alpha$- oder -NHCS-$\alpha$-Gruppe und

$\alpha$ für die Bindungsstelle an die terminalen Stickstoffatome der letzten Generation, der Reproduktionseinheit W

stehen.

Bevorzugt sind Komplexbildner-Reste-K der allgemeinen Formel IA.

Als bevorzugte Komplexbildner-Reste K seien weiterhin diejenigen genannt, bei denen in der oben angegebenen Formel IA die für $U^7$ stehende $C_1$-$C_{20}$-, bevorzugt $C_1$-$C_{12}$-, Alkylenkette die Gruppen
$-CH_2-$, $-CH_2NHCO-$, $-NHCOCH_2O-$, $-NHCOCH_2OC_6H_4-$, $-N(CH_2CO_2H)-$, $-NHCOCH_2C_6H_4-$, $-NHCSNHC_6H_4-$, $-CH_2OC_6H_4-$, $-CH_2CH_2O-$, enthält und/oder durch die Gruppen -COOH, $-CH_2COOH$ substituiert ist.

Als Beispiele für $U^7$ seien folgende Gruppen angeführt:

$-CH_2-$, $-CH_2CH_2-$, $-CH_2CH_2CH_2-$, $-C_6H_4-$, $-C_6H_{10}-$, $-CH_2C_6H_5-$, $-CH_2NHCOCH_2CH(CH_2CO_2H)-C_6H_4-$,
$-CH_2NHCOCH_2OCH_2-$,
$-CH_2NHCOCH_2C_6H_4-$,

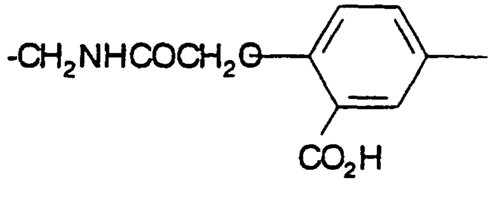

,

$-CH_2NHCSNH-C_6H_4-CH(CH_2COOH)CH_2-$,
$-CH_2OC_6H_4-N(CH_2COOH)CH_2-$,
$-CH_2NHCOCH_2O(CH_2CH_2O)_4-C_6H_4-$,
$-CH_2O-C_6H_4-$,
$-CH_2CH_2-O-CH_2CH_2-$, $-CH_2CH_2-O-CH_2CH_2-O-CH_2CH_2-$,

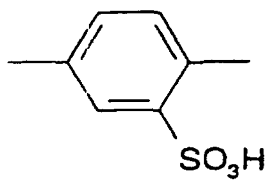

,

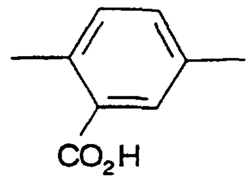

Besonders bevorzugt steht $U^7$ für die $CH_2$-Gruppe.

Als Beispiele für $R^4$ seien folgende Gruppen angegeben:

$-CH_3$, $-C_6H_5$, $-CH_2-COOH$,
$-CH_2-C_6H_5$, $-CH_2-O-(CH_2CH_2-O-)_6CH_3$, $-CH_2-OH$

Bevorzugt sind das Wasserstoffatom und die Methylgruppe,

T steht bevorzugt für die -CO-$\alpha$-Gruppe.

Ist das erfindungsgemäße Mittel zur Anwendung in der NMR-Diagnostik bestimmt, so muß das Zentralion des Komplexsalzes paramagnetisch sein. Dies sind insbesondere die zwei- und dreiwertigen Ionen der Elemente der Ordnungszahlen 21 - 29, 42, 44 und 58 - 70. Geeignete Ionen sind beispielsweise das Chrom(III)-, Eisen(II)-, Cobalt (II)-, Nickel(II)-, Kupfer(II)-, Praseodym(III)-, Neodym(III)-, Samarium(III)- und Ytterbium(III)-ion. Wegen ihres sehr starken magnetischen Moments sind besonders bevorzugt das Gadolinium(III)-, Terbium(III)-, Dysprosium(III)-, Holmium (III)-, Erbium(III)-, Mangan(II)- und Eisen(III)-ion.

Ist das erfindungsgemäße Mittel zur Anwendung in der Röntgen-Diagnostik bestimmt, so muß sich das Zentralion von einem Element höherer Ordnungszahl ableiten, um eine ausreichende Absorption der Röntgenstrahlen zu erzielen. Es wurde gefunden, daß zu diesem Zweck diagnostische Mittel, die ein physiologisch verträgliches Komplexsalz mit Zentralionen von Elementen der Ordnungszahlen zwischen 21 - 29, 39, 42, 44, 57 - 83 enthalten, geeignet sind; dies sind beispielsweise das Lanthan(III)-ion und die oben genannten Ionen der Lanthanidenreihe.

Die erfindungsgemäßen Kaskaden-Polymer-Komplexe enthalten mindestens 16 Ionen eines Elements der oben genannten Ordnungszahl.

Die restlichen aciden Wasserstoffatome, das heißt diejenigen, die nicht durch das Zentralion substituiert worden sind, können gegebenenfalls ganz oder teilweise durch Kationen von anorganischen und/oder organischen Basen, Aminosäuren oder Aminosäureamiden ersetzt sein.

Geeignete anorganische Kationen sind beispielsweise das Lithiumion, das Kaliumion, das Calciumion, das Magnesiumion und insbesondere das Natriumion. Geeignete Kationen organischer Basen sind unter anderem solche von primären, sekundären oder tertiären Aminen, wie zum Beispiel Ethanolamin, Diethanolamin, Morpholin, Glucamin, N,N-Dimethylglucamin und insbesondere N-Methylglucamin. Geeignete Kationen von Aminosäuren sind beispielsweise die des Lysins, des Arginins und des Omithins sowie die Amide ansonsten saurer oder neutraler Aminosäuren.

Die erfindungsgemäßen Verbindungen, die ein Molekulargewicht von 10.000 - 80.000 D, vorzugsweise 15.000 - 40.000 D, besitzen, weisen die eingangs geschilderten gewünschten Eigenschaften auf. Sie enthalten die für ihre Verwendung benötigte große Anzahl von Metallionen im Komplex stabil gebunden.

Sie reichern sich in Gebieten mit erhöhter Gefäßpermeabilität, wie z.B. in Tumoren, an, erlauben Aussagen über die Perfusion von Geweben, geben die Möglichkeit, das Blutvolumen in Geweben zu bestimmen, die Relaxationszeiten bzw. Densitäten des Blutes selektiv zu verkürzen, und die Permeabilität der Blutgefäße bildlich darzustellen. Solche physiologischen Informationen sind nicht durch den Einsatz von extrazellulären Kontrastmitteln, wie z.B. Gd-DTPA [Magnevist®], zu erhalten. Aus diesen Gesichtspunkten ergeben sich auch die Einsatzgebiete bei den modernen bildgebenden Verfahren Kernspintomographie und Computertomographie: spezifischere Diagnose von malignen Tumoren, frühe Therapiekontrolle bei zytostatischer, antiphlogistischer oder vasodilatativer Therapie, frühe Erkennung von minderperfundierten Gebieten (z.B. im Myokard), Angio-graphie bei Gefäßerkrankungen, und Erkennung und Diagnose von (sterilen oder infektiösen) Entzündungen.

Als weitere Vorteile gegenüber extrazellulären Kontrastmitteln, wie z.B. Gd-DTPA [Magnevist®], muß die höhere Effektivität als Kontrastmittel für die Kernspintomographie (höhere Relaxivität) hervorgehoben werden, was zu

einer deutlichen Reduktion der diagnostisch notwendigen Dosis führt. Gleichzeitig können die erfindungsgemäßen Kontrastmittel als Lösungen isoosmolar zum Blut formuliert werden und verringern dadurch die osmotische Belastung des Körpers, was sich in einer verringerten Toxizität der Substanz (höhere toxische Schwelle) niederschlägt. Geringere Dosen und höhere toxische Schwelle führen zu einer signifikanten Erhöhung der Sicherheit von Kontrastmittelanwendungen bei modernen bildgebenden Verfahren.

Im Vergleich zu den makromolekularen Kontrastmitteln auf der Basis von Kohlenhydraten, z.B. Dextran (Europäische Patentanmeldung, Publikations-Nr. 0 326 226), die - wie erwähnt - in der Regel nur ca. 5 % des signalverstärkenden paramagnetischen Kations tragen, weisen die erfindungsgemäßen Polymer-Komplexe einen Gehalt von in der Regel ca. 20 % des paramagnetischen Kations auf. Somit bewirken die erfindungsgemäßen Makromoleküle pro Molekül eine sehr viel höhere Signalverstärkung, was gleichzeitig dazu führt, daß die zur Kernspintomographie notwendige Dosis gegenüber der makromolekularer Kontrastmittel auf Kohlenhydratbasis erheblich kleiner ist.

Mit den erfindungsgemäßen Polymer-Komplexen ist es gelungen, Makromoleküle so zu konstruieren und herzustellen, daß diese ein einheitlich definiertes Molekulargewicht haben. Es ist somit überraschenderweise möglich, die Größe der Makromoleküle so zu steuern, daß diese groß genug sind, um den vasalen Raum nur langsam verlassen zu können, aber gleichzeitig klein genug, die Kapillaren der Niere, welche 300 - 800 Å groß sind, noch passieren zu können.

Im Vergleich zu den anderen erwähnten Polymer-Verbindungen des Stands der Technik zeichnen sich die erfindungsgemäßen Kaskaden-Polymer-Komplexe durch verbessertes Ausscheidungsverhalten, höhere Wirksamkeit, größere Stabilität und/oder bessere Verträglichkeit aus.

Ein weiterer Vorteil der vorliegenden Erfindung liegt darin, daß nunmehr Komplexe mit hydrophilen oder lipophilen, makrocyclischen oder offenkettigen, niedermolekularen oder hochmolekularen Liganden zugänglich geworden sind. Dadurch ist die Möglichkeit gegeben, Verträglichkeit und Pharmakokinetik dieser Polymer-Komplexe durch chemische Substitution zu steuern.

Die Herstellung der erfindungsgemäßen Kaskaden-Polymer-Komplexe erfolgt dadurch, daß man Verbindungen der allgemeinen Formel I'

$$A\text{-}\{X\text{-}[Y\text{-}(Z\text{-}\langle W\text{-}\beta_w\rangle_z)_y]_x\}_a \qquad (I')$$

worin

| | |
|---|---|
| A | für einen stickstoffhaltigen Kaskadenkern der Basismultiplizität a, |
| X und Y | unabhängig voneinander für eine direkte Bindung oder eine Kaskadenreproduktionseinheit der Reproduktionsmultiplizität x bzw. y, |
| Z und W | unabhängig voneinander für eine Kaskadenreproduktionseinheit der Reproduktionsmultiplizität z bzw. w, |
| a | für die Ziffern 2 bis 12, |
| x, y, z | und w unabhängig voneinander für die Ziffern 1 bis 4 und |
| β | für die Bindungsstelle der terminalen NH-Gruppen der letzten Generation, der Reproduktionseinheit W stehen |

mit der Maßgabe, daß mindestens zwei Reproduktionseinheiten unterschiedlich sind, daß für das Produkt der Multiplizitäten gilt

$$16 \leq a \cdot x \cdot y \cdot z \cdot w \leq 64$$

und daß mindestens eine der Kaskadenreproduktionseinheiten X, Y, Z, W für eine 1,4,7,10-Tetraazacyclododecan- oder 1,4,8,11-Tetraazacyclotetradecan-Reproduktionseinheit steht,
mit einem Komplex oder Komplexbildner K' der allgemeinen Formel I'A, I'B oder I'C

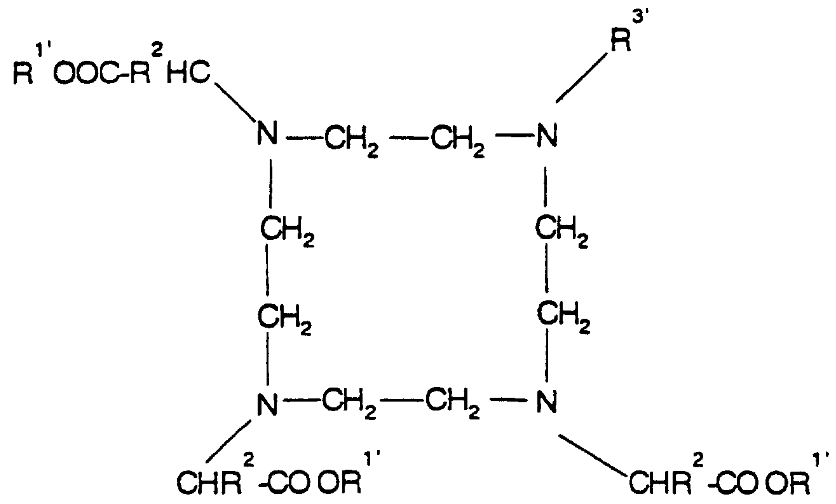

(I'A)

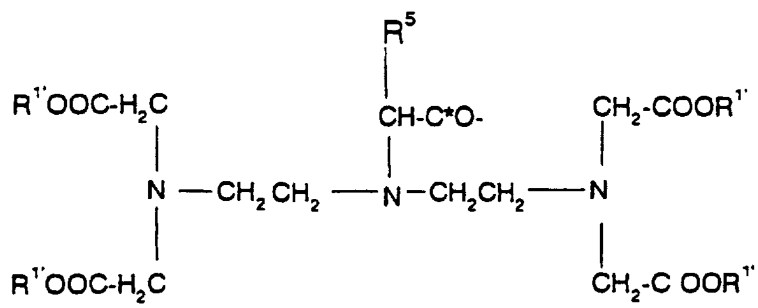

(I'B),

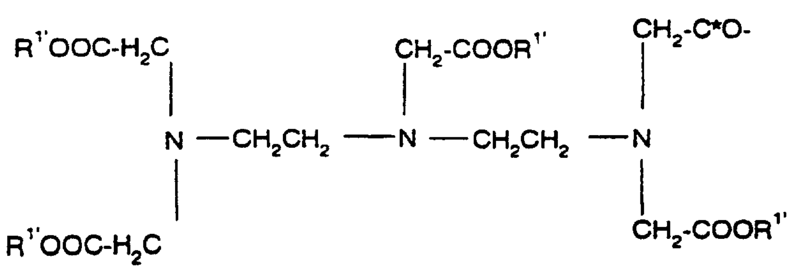

(I'C)

wobei

R1' unabhängig voneinander für ein Wasserstoffatom, ein Metallionenäquivalent der Ordnungszahlen 20 - 29, 39, 42 - 44 oder 57 - 83 oder eine Säureschutzgruppe,

$R^2$ für ein Wasserstoffatom, einen Methyl- oder einen Ethylrest, der gegebenenfalls mit 1 - 2 Hydroxy- oder 1 Carboxygruppe(n) substituiert ist,

$R^{3'}$ für eine

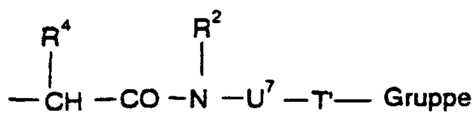

oder eine

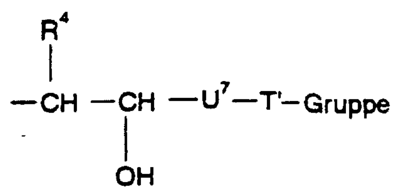

$R^4$ für ein Wasserstoffatom oder eine geradkettige, verzweigte, gesättigte oder ungesättigte $C_1$-$C_{30}$-Alkylkette, die gegebenenfalls durch 1 - 10 Sauerstoff atome, 1 Phenylen-, 1 Phenylenoxygruppen unterbrochen und/oder gegebenenfalls durch 1 - 5 Hydroxy-, 1 - 3 Carboxy-, 1 Phenylgruppe(n) substituiert ist,

$R^5$ für ein Wasserstoffatom oder für $R^4$,

$U^7$ für eine gegebenenfalls 1 - 5 Imino-, 1 - 3 Phenylen-, 1 - 3 Phenylenoxy-, 1 - 3 Phenylenimino-, 1 - 5 Amid-, 1 - 2 Hydrazid-, 1 - 5 Carbonyl-, 1 - 5 Ethylenoxy-, 1 Harnstoff-, 1-Thioharnstoff-, 1 - 2 Carboxyalkylimino-, 1 - 2 Estergruppen, 1 - 10 Sauerstoff-, 1 - 5 Schwefel- und/oder 1 - 5 Stickstoff-Atom(e) enthaltende und/oder gegebenenfalls durch 1 - 5 Hydroxy-, 1 - 2 Mercapto-, 1 - 5 Oxo-, 1 - 5 Thioxo-, 1 - 3 Carboxy-, 1 - 5 Carboxyalkyl-, 1 - 5 Ester- und/oder 1 - 3 Aminogruppe(n) substituierte geradkettige, verzweigte, gesättigte oder ungesättigte $C_1$-$C_{20}$-Alkylengruppe, wobei die gegebenenfalls enthaltenden Phenylengruppen durch 1 - 2 Carboxy-, 1 - 2 Sulfon- oder 1 - 2 Hydroxygruppen substituiert sein können,

T' für eine -C*O-, -COOH-, -N=C=O- oder -N=C=S-Gruppe und

C*O für eine aktivierte Carboxylgruppe

stehen,

mit der Maßgabe, daß - sofern K' für einen Komplex steht - mindestens zwei (bei zweiwertigen Metallen) bzw. drei (bei dreiwertigen Metallen) der Substituenten $R^1$ für ein Metallionenäquivalent der oben genannten Elemente stehen und daß gewünschtenfalls weitere Carboxylgruppen in Form ihrer Salze mit anorganischen und/oder organischen Basen, Aminosäuren oder Aminosäureamiden vorliegen,

umsetzt, gegebenenfalls vorhandene Schutzgruppen abspaltet, die so erhaltenen Kaskaden-Polymere - sofern K' für einen Komplexbildner steht - in an sich bekannter Weise mit mindestens einem Metalloxid oder Metallsalz eines Elements der Ordnungszahlen 20 - 29, 39, 42, 44 oder 57 - 83 umsetzt und gegebenenfalls anschließend in den so erhaltenen Kaskaden-Polymer-Komplexen noch vorhandene acide Wasserstoffatome ganz oder teilweise durch Kationen von anorganischen und/oder organischen Basen, Aminosäuren oder Aminosäureamiden substituiert und gegebenenfalls noch vorhandene freie terminale Aminogruppen gewünschtenfalls - vor oder nach der Metallkomplexierung - acyliert.

Einen weiteren Aspekt der vorliegenden Erfindung stellen die neuen Verbindungen der allgemeinen Formel I'A dar

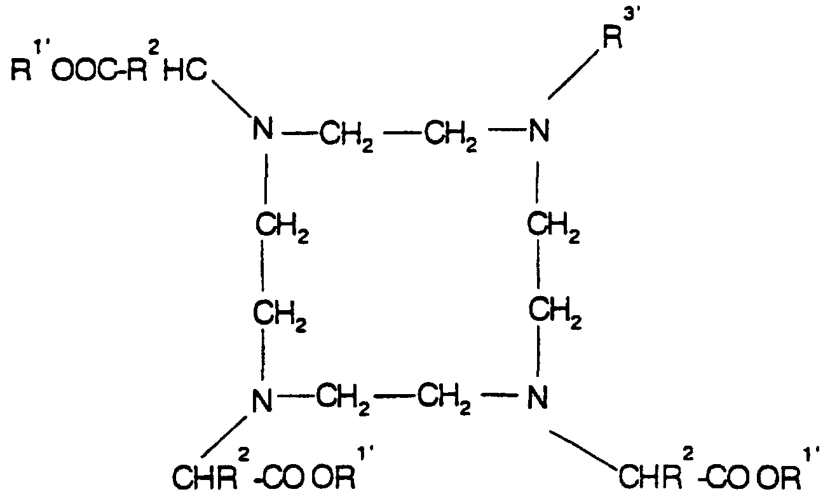

(I'A)

wobei

R1' unabhängig voneinander für ein Wasserstoffatom, ein Metallionenäquivalent der Ordnungszahlen 20 - 29, 39, 42 - 44 oder 57 - 83 oder eine Säureschutzgruppe,

$R^2$ für ein Wasserstoffatom, einen Methyl- oder einen Ethylrest, der gegebenenfalls mit 1 - 2 Hydroxy- oder 1 Carboxygruppe(n) substituiert ist,

$R^{3'}$ für eine

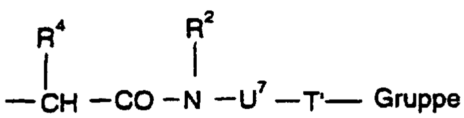

oder eine

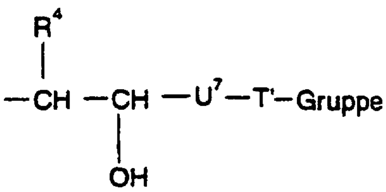

,

$R^4$ für ein Wasserstoffatom oder eine geradkettige, verzweigte, gesättigte oder ungesättigte $C_1$-$C_{30}$-Alkylkette, die gegebenenfalls durch 1 - 10 Sauerstoff atome, 1 Phenylen-, 1 Phenylenoxygruppen unterbrochen und/oder gegebenenfalls durch 1 - 5 Hoydroxy-, 1 - 3 Carboxy-, 1 Phenylgruppe(n) substituiert ist,

$U^7$ für eine gegebenenfalls 1 - 5 Imino-, 1 - 3 Phenylen-, 1 - 3 Phenylenoxy-, 1 - 3 Phenylenimino-, 1 - 5 Amid-, 1 - 2 Hydrazid-, 1 - 5 Carbonyl-, 1 - 5 Ethylenoxy-, 1 Harnstoff-, 1-Thioharnstoff-, 1 - 2 Carboxyalkylimino-, 1 - 2 Estergruppen, 1 - 10 Sauerstoff-, 1 - 5 Schwefel- und/oder 1 - 5 Stickstoff-Atom(e) enthaltende und/oder gegebenenfalls durch 1 - 5 Hydroxy-, 1 - 2 Mercapto-, 1 - 5 Oxo-, 1 - 5 Thioxo-, 1 - 3 Carboxy-, 1 - 5 Carboxyalkyl-, 1 - 5 Ester- und/oder 1 - 3 Aminogruppe(n) substituierte geradkettige, verzweigte, gesättigte oder ungesättigte $C_1$-$C_{20}$-Alkylengruppe, wobei die gegebenenfalls enthaltenden Phenylengruppen durch 1 - 2 Carboxy-, 1 - 2

Sulfon- oder 1 - 2 Hydroxygruppen substituiert sein können,

T' für eine -C*O-, -COOH-, -N=C=O- oder -N=C=S-Gruppe und

C*O für eine aktivierte Carboxylgruppe

stehen.

Sie dienen als wichtige Zwischenprodukte für die Herstellung der Kaskaden-Polymer-Komplexe der allgemeinen Formel I.

Als Beispiel für eine aktivierte Carbonylgruppe C*O in den Komplexen bzw. Komplexbildnern K' seien Anhydrid, p-Nitrophenylester, N-Hydroxysuccinimidester, Pentafluorphenylester und Säurechlorid genannt.

Die zur Einführung der Komplexbildner-Einheiten vorgenommene Addition oder Acylierung wird mit Substraten durchgeführt, die den gewünschten Substituenten K (eventuell gebunden an eine Fluchtgruppe) enthalten, oder aus denen der gewünschte Substituent durch die Reaktion generiert wird.

Als Beispiele für Additionsreaktionen seien die Umsetzung von Isocyanaten und Isothiocyanaten genannt, wobei die Umsetzung von Isocyanaten bevorzugt in aprotischen Solventien wie z.B. THF, Dioxan, DMF, DMSO, Methylenchlorid bei Temperaturen zwischen 0 und 100 °C, bevorzugt zwischen 0 und 50 °C, gegebenenfalls unter Zusatz einer organischen Base wie Triethylamin, Pyridin, Lutidin, N-Ethyldiisopropylamin, N-Methylmorpholin, durchgeführt wird. Die Umsetzung mit Isothiocyanaten wird in der Regel in Lösungsmitteln wie z.B. Wasser oder niederen Alkoholen wie z.B. Methanol, Ethanol, Isopropanol oder deren Mischungen, DMF oder Mischungen aus DMF und Wasser bei Temperaturen zwischen 0 und 100 °C, bevorzugt zwischen 0 und 50 °C, gegebenenfalls unter Zusatz einer organischen oder anorganischen Base wie z.B. Triethylamin, Pyridin, Lutidin, N-Ethyldiisopropylamin, N-Methylmorpholin oder Erdalkali-, Alkalihydroxiden wie z.B. Lithium-, Natrium-, Kalium-, Calciumhydroxid oder deren Carbonate wie z.B. Magnesiumcarbonat, durchgeführt.

Als Beispiele für Acylierungsreaktionen seien die Umsetzung von freien Carbonsäuren nach den dem Fachmann bekannten Methoden [z.B. J.P. Greenstein, M. Winitz, Chemistry of the Amino Acids, John Wiley & Sons, N.Y. (1961), S. 943- 945] genannt. Als vorteilhaft hat sich jedoch erwiesen, die Carbonsäuregruppe vor der Acylierungsreaktion in eine aktivierte Form wie z.B. Anhydrid, Aktivester oder Säurechlorid zu überführen [z.B. E. Gross, J. Meienhofer, The Peptides, Academic Press, N.Y. (1979), Vol. 1, S. 65- 314; N.F. Albertson, Org. React. 12, 157 (1962)].

Im Falle der Umsetzung mit Aktivester sei auf die dem Fachmann bekannte Literatur [z.B. Houben-Weyl, Methoden der organischen Chemie, Georg Thieme Verlag, Stuttgart, Band E 5 (1985), 633] verwiesen. Sie kann unter den oben für die Anhydridreaktion angegebenen Bedingungen durchgeführt werden. Es können aber auch aprotische Lösungsmittel wie z.B. Methylenchlorid, Chloroform verwendet werden.

Im Falle der Säurechlorid-Umsetzungen werden nur aprotische Lösungsmittel wie z.B. Methylenchlorid, Chloroform, Toluol oder THF bei Temperaturen zwischen -20 bis 50 °C, bevorzugt zwischen 0 bis 30 °C, verwendet. Des weiteren sei auf die dem Fachmann bekannte Literatur [z.B. Houben-Weyl, Methoden der Organischen Chemie, Georg-Thieme-Verlag, Stuttgart, (1974), Band 15/2, S. 355- 364] verwiesen.

Falls R1' für eine Säureschutzgruppe steht, kommen niedere Alkyl-, Aryl- und Aralkylgruppen, beispielsweise die Methyl-, Ethyl-, Propyl-, Butyl-, Phenyl-, Benzyl-, Diphenylmethyl-, Triphenylmethyl-, bis-(p-Nitrophenyl)-methylgruppe, sowie Trialkylsilylgruppen in Frage.

Die gegebenenfalls gewünschte Abspaltung der Schutzgruppen erfolgt nach den dem Fachmann bekannten Verfahren, beispielsweise durch Hydrolyse, Hydrogenolyse, alkalische Verseifung der Ester mit Alkali in wäßrig-alkoholischer Lösung bei Temperaturen von 0 °C bis 50 °C oder im Fall von tert.-Butylestern mit Hilfe von Trifluoressigsäure.

Gegebenenfalls unvollständig mit Ligand oder Komplex acylierte terminale Aminogruppen können, wenn gewünscht, in Amide oder Halbamide überführt werden. Beispielhaft genannt sei die Umsetzung mit Acetanhydrid, Bernsteinsäureanhydrid oder Diglykolsäureanhydrid.

Die Einführung der gewünschten Metallionen erfolgt in der Weise, wie sie z.B. in der Deutschen Offenlegungsschrift 34 01 052 offenbart worden ist, indem man das Metalloxid oder ein Metallsalz (beispielsweise das Nitrat, Acetat, Carbonat, Chlorid oder Sulfat) des Elements der Ordnungszahlen 20 - 29, 42, 44, 57 - 83 in Wasser und/oder einem niederen Alkohol (wie Methanol, Ethanol oder Isopropanol) löst oder suspendiert und mit der Lösung oder Suspension der äquivalenten Menge des komplexbildenden Liganden umsetzt und anschließend, falls gewünscht, vorhandene acide Wasserstoffatome der Säuregruppen durch Kationen von anorganischen und/oder organischen Basen, Aminosäuren oder Aminosäureamiden substituiert.

Die Einführung der gewünschten Metallionen kann sowohl auf der Stufe der Komplexbildner I'A oder I'B, d. h. vor der Kopplung an die Kaskaden-Polymere, als auch nach Kopplung der unmetallierten Liganden I'A, I'B oder I'C erfolgen.

Die Neutralisation erfolgt dabei mit Hilfe anorganischer Basen (zum Beispiel Hydroxiden, Carbonaten oder Bicarbonaten) von zum Beispiel Natrium, Kalium, Lithium, Magnesium oder Calcium und/oder organischer Basen wie

unter anderem primärer, sekundärer und tertiärer Amine, wie zum Beispiel Ethanolamin, Morpholin, Glucamin, N-Methyl- und N,N-Dimethylglucamin, sowie basischer Aminosäuren, wie zum Beispiel Lysin, Arginin und Ornithin oder von Amiden ursprünglich neutraler oder saurer Aminosäuren, wie zum Beispiel Hippursäure, Glycinacetamid.

Zur Herstellung der neutralen Komplexverbindungen kann man beispielsweise den sauren Komplexsalzen in wäßriger Lösung oder Suspension so viel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie zum Beispiel niederen Alkoholen (Methanol, Ethanol, Isopropanol und andere), niederen Ketonen (Aceton und andere), polaren Ethern (Tetrahydrofuran, Dioxan, 1,2-Dimethoxyethan und andere) auszufällen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrensschritt einzusparen.

Enthalten die sauren Komplexverbindungen mehrere freie acide Gruppen, so ist es oft zweckmäßig, neutrale Mischsalze herzustellen, die sowohl anorganische als auch organische Kationen als Gegenionen enthalten.

Dies kann beispielsweise geschehen, indem man den komplexbildenden Liganden in wäßriger Suspension oder Lösung mit dem Oxid oder Salz des das Zentralion liefernden Elements und der Hälfte der zur Neutralisation benötigten Menge einer organischen Base umsetzt, das gebildete Komplexsalz isoliert, es gewünschtenfalls reinigt und dann zur vollständigen Neutralisation mit der benötigten Menge anorganischer Base versetzt. Die Reihenfolge der Basenzugabe kann auch umgekehrt werden.

Die Reinigung der so erhaltenen Kaskaden-Polymer-Komplexe erfolgt, gegebenenfalls nach Einstellung des pH-Wertes durch Zusatz einer Säure oder Base auf pH6 bis 8, bevorzugt ca. 7, vorzugsweise durch Ultrafiltration mit Membranen geeigneter Posengröße (z.B. Amicon®XM30, Amicon®YM10, Amicon®YM3) oder Gelfiltration an z.B. geeigneten Sephadex®-Gelen.

Im Falle von neutralen Komplexverbindungen ist es häufig von Vorteil, die polymeren Komplexe über einen Anionenaustauscher, beispielsweise IRA 67 ($OH^-$-Form) und gegebenenfalls zusätzlich über einen Kationenaustauscher, beispielsweise IRC 50 ($H^+$-Form) zur Abtrennung ionischer Komponenten zu geben.

Die Herstellung der für die Kopplung an die Komplexbildner K' (bzw. auch die entsprechenden metallhaltigen Komplexe) benötigten terminale Aminogruppen tragenden Kaskaden-Polymere geht im allgemeinen aus von käuflichen bzw. nach oder analog literaturbekannten Methoden herstellbaren stickstoffhaltigen Kaskadenstartern $A(H)_a$. Die Einführung der Generationen X, Y, Z und W erfolgt nach literaturbekannten Methoden [z.B. J. March, Advanced Organic Chemistry, 3rd ed.; John Wiley & Sons, (1985), 364 - 381] durch Acylierungs- bzw. Alkylierungsreaktionen mit die gewünschten Strukturen aufweisenden geschützten Aminen, die zur Bindung an den Kaskadenkern befähigte funktionelle Gruppen wie z.B. Carbonsäuren, Isocyanate, Isothiocyanate oder aktivierte Carbonsäuren (wie z.B. Anhydride, Aktivester, Säurechloride) bzw. Halogenide (wie z.B. Chloride, Bromide, Iodide), Aziridin, Mesylate, Tosylate oder andere dem Fachmann bekannte Fluchtgruppen enthalten.

Es sei jedoch hier nochmals betont, daß die Unterscheidung zwischen Kaskadenkern A und Reproduktionseinheiten rein formal ist. Es kann synthetisch von Vorteil sein, daß man nicht den formalen Kaskadenstarter $A(H)_a$ verwendet, sondern die per Definition zum Kaskadenkern gehörigen Stickstoffatome erst zusammen mit der ersten Generation einführt. So ist es z.B. zur Synthese der in Beispiel 1b) beschriebenen Verbindung vorteilhafter, nicht den formalen Kaskadenkern Trimesinsäuretriamid mit z.B. Benzyloxycarbonylaziridin (sechsfach) zu alkylieren, sondern Trimesinsäuretrichlorid mit Bis[2-(benzyloxycarbonylamino)-ethyl]-amin (dreifach) umzusetzen.

In gleicher Weise kann es beim Einbau der notwendigerweise vorhandenen 1,4,7,10-Tetraazacyclododecan ("Cyclen")- oder 1,4,8,11-Tetraazacyclotetradecan("Cyclam")-Reproduktionseinheiten von Vorteil sein, das Kaskaden-Polymer nicht formal Schale für Schale aufzubauen.

So ist es beispielsweise möglich, in einem vorgeschalteten Reaktionsschritt die auf eine Cyclen-Schale folgende Reproduktionseinheit an drei Stickstoffatome des Cyclens zu binden. Anschließend können so nach Funktionalisierung des vierten Cyclen-Stickstoffs beide Reproduktionseinheiten gleichzeitig an die wachsende Kaskade geknüpft werden.

Als Aminschutzgruppen seien die dem Fachmann geläufigen Benzyloxycarbonyl-, tertiär-Butoxycarbonyl-, Trifluoracetyl-, Fluorenylmethoxycarbonyl-, Benzyl- und Formylgruppe [Th. W. Greene, P.G.M Wuts, Protective Groups in Organic Syntheses, 2nd ed, John Wiley and Sons (1991), S. 309-385] genannt. Nach Abspaltung dieser Schutzgruppen, die ebenfalls nach literaturbekannten Methoden erfolgt, kann die nächste gewünschte Generation in das Molekül eingeführt werden. Neben diesem aus jeweils zwei Reaktionsstufen (Alkylierung bzw. Acylierung und Schutzgruppenabspaltung) bestehenden Aufbau einer Generation ist auch mit ebenfalls nur zwei Reaktionsstufen die gleichzeitige Einführung von zwei, z.B. X-[Y]x, oder mehrerer Generationen, z.B. $X\text{-}[Y\text{-}(Z)_y]_x$, möglich. Der Aufbau dieser Mehrgenerationen-Einheiten erfolgt durch Alkylierung bzw. Acylierung von die Strukturen der gewünschten Reproduktionseinheiten aufweisenden ungeschützten Aminen ("Reproduktionsamin") mit einem zweiten Reproduktionsamin, dessen Amingruppen in geschützter Form vorliegen.

Die als Kaskadenstarter benötigten Verbindungen der allgemeinen Formel $A(H)_a$ sind käuflich zu erwerben

oder nach bzw. analog literaturbekannten Methoden [z.B. Houben-Weyl, Methoden der Org. Chemie, Georg-Thieme-Verlag, Stutgart (1957), Bd. 11/1; M. Micheloni et al., Inorg. Chem. (1985), 24, 3702; T.J. Atkins et al., Org. Synth., Vol. 58 (1978), 86 - 98; The Chemistry of Heterocyclic Compounds: J.S. Bradshaw et al., Aza-Crown-Macrocycles, John Wiley & Sons, N.Y. (1993)] herstellbar. Beispielhaft angeführt seien:

Tris(aminoethyl)amin [z.B. Fluka Chemie AG, Schweiz; Aldrich-Chemie, Deutschland];
Tris(aminopropyl)amin [z.B. C. Woerner et al., Angew. Chem. Int. Ed. Engl. (1993), 32, 1306];
Diethylentriamin [z.B. Fluka; Aldrich];
Triethylentetramin [z.B. Fluka; Aldrich];
Tetraethylenpentamin [z.B. Fluka; Aldrich];
1,3,5-Tris(aminomethyl)benzol [z.B. T.M. Garrett et al., J. Am. Chem. Soc. (1991), 113, 2965];
Trimesinsäuretriamid [z.B. H. Kurihara; Jpn. Kokai Tokkyo Koho JP 04077481; CA 117, 162453];
1,4,7-Triazacyclononan [z.B. Fluka; Aldrich];
1,4,7,10,13-Pentaazacyclopentadecan [z.B. K.W. Aston, Eur. Pat. Appl. 0 524 161, CA 120, 44580];
1,4,7,10-Tetraazacyclododecan [z.B. Aldrich]
1,4,8,11-Tetraazacyclotetradecan [z.B. Fluka; Aldrich];
1,4,7,10,13,16,19,22,25,28-Decaazacyclotriacontan [z.B. A. Andres et al., J. Chem. Soc. Dalton Trans. (1993), 3507];
1,1,1-Tris(aminomethyl)ethan [z.B. R.J. Geue et al., Aust. J. Chem. (1983), 36, 927];
Tris(aminopropyl)-nitromethan [z.B. G.R. Newkome et al., Angew. Chem. 103, 1205 (1991) analog zu R.C. Larock, Comprehensive Organic Transformations, VCH Publishers, N.Y. (1989), 419 - 420]
1,3,5,7-Adamantantetracarbonsäureamid [z.B. H. Stetter et al., Tetr. Lett. 1967, 1841];
1,2-Bis[Phenoxyethan]-3',3",5',5"-tetracarbonsäureamid [z.B. J.P. Collman et al.; J. Am. Chern. Soc. (1988), 110, 3477 - 86 analog zur Vorschrift für das Beispiel 1b)];
1,4,7,10,13,16,21,24-Octaazabicyclo[8.8.8.]hexacosan [z.B. P.H. Smith et al., J. Org. Chem. (1993), 58, 7939].

Die Herstellung der für den Aufbau der Generationen benötigten die oben genannten funktionellen Gruppen enthaltenden Reproduktionsamine erfolgt nach bzw. analog den im experimentellen Teil beschriebenen Vorschriften bzw. nach literaturbekannten Verfahren.

Beispielhaft genannt seien:

$N^{\alpha}$,$N^{\varepsilon}$-Di-Benzyloxycarabonyl-Lysin-p-nitrophenylester;
$HOOC-CH_2OCH_2CO-N(CH_2CH_2NH-CO-O-CH_2C_6H_5)_2$;
$HOOC-CH_2N(CH_2CH_2NH-CO-O-CH_2C_6H_5)_2$;
$HOOC-CH_2CH_2CO-N(CH_2CH_2NH-COCF_3)_2$ [herzustellen nach Vorschrift für das Beispiel 5a), indem man anstelle von Bis(benzyloxycarbonylaminoethyl)amin von Bis(trifluoracetylaminoethyl)amin ausgeht],
$HOOC-CH_2OCH_2CONH-C_6H_4-CH[CH_2CON(CH_2CH_2NH-CO-O-CH_2C_6H_5)_2]_2$;
$O=C=N-C_6H_4-CH[CH_2CON(CH_2CH_2NH-CO-O-CH_2C_6H_5)_2]_2$

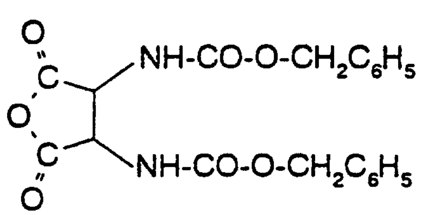

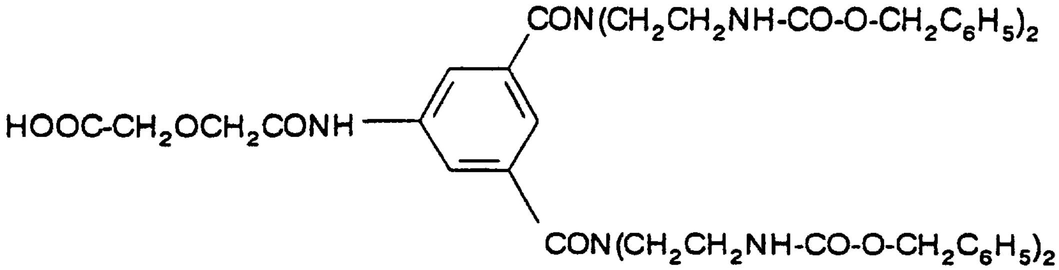

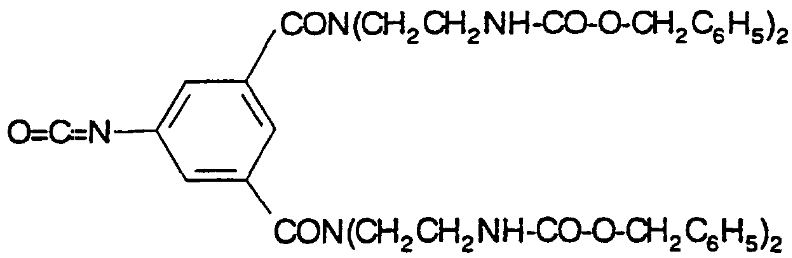

| | |
|---|---|
| N-Benzyloxycarbonyl-aziridin | herzustellen nach M. Zinic et al., J. Chem. Soc, Perkin Trans 1, 21-26 (1993) |
| N-Benzyloxycarbonyl-glycin | käuflich bei z.B. Bachem California |

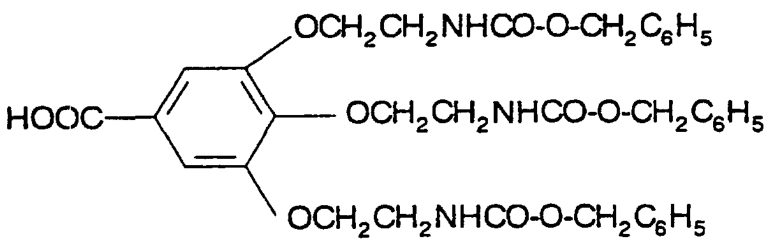

herzustellen nach CJ. Cavallito et al., J. Amer. Chem. Soc. 1943, 65, 2140, indem man anstelle von Benzylchlorid von N-CO-O-$CH_2C_6H_5$-(2-Bromethyl)amin [A.R. Jacobson et al., J. Med. Chem. (1991), 34, 2816] ausgeht.

Die Herstellung der Komplexe und Komplexbildner der allgemeinen Formel I'A und I'B erfolgt nach bzw. analog den im experimentellen Teil beschriebenen Vorschriften bzw. nach literaturbekannten Methoden (s. z.B. Europäische Patentanmeldungen Nr. 0 512 661, 0 430 863, 0 255 471 und 0 565 930.

So kann die Herstellung von Verbindungen der allgemeinen Formel I'A z.B. dadurch erfolgen, daß als Vorstufe der funktionellen Gruppe T' eine Gruppe T" dient, entweder in der Bedeutung einer geschützten Säurefunktion, die unabhängig von den Säureschutzgruppen $R^{1'}$ nach den oben aufgeführten Verfahren in die freie Säurefunktion überführt werden kann, oder in der Bedeutung einer geschützten Aminfunktion, die nach literaturbekannten Verfahren deblockiert [Th.W. Greene, P.G.M. Wuts, Protective Groups in Organic Synthesis, 2nd edition, John Wiley & Sons (1991), S. 309-385] und anschließend in die Isocyanate bzw. Isothiocyanate überrührt werden kann [Methoden der Org. Chemie (Houben-Weyl), E 4, S. 742-749, 837-843, Georg Thieme Verlag, Stuttgart, New York (1983)]. Solche Verbindungen sind nach bzw. analog den im experimentellen Teil beschriebenen Vorschriften durch Monoalkylierung von Cyclen mit

geeigneten α-Halogen-carbonsäureamiden [in aprotischen Lösungsmitteln, wie z.B. Chloroform] herstellbar.

Die Herstellung von Verbindungen der allgemeinen Formel I'B kann beispielsweise dadurch erfolgen, daß als Vorstufe der aktivierten Carboxylgruppe-C*O- eine geschützte Säurefunktion dient, die unabhängig von den Säureschutzgruppen $R^{1'}$ nach den oben aufgeführten Verfahren in die freie Säurefunktion überführt und nach den ebenfalls oben beschriebenen literaturbekannten Verfahren aktiviert werden kann. Solche Verbindungen sind nach bzw. analog den im experimentellen Teil beschriebenen Vorschriften herstellbar oder beispielsweise dadurch, daß ein Aminosäurederivat der allgemeinen Formel II

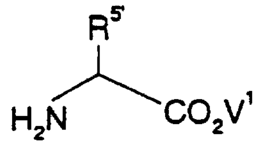

(II)

worin

$R^{5'}$ die für $R^5$ angegebene Bedeutung hat, wobei gegebenenfalls in $R^5$ enthaltene Hydroxy- oder Carboxygruppen gegebenenfalls in geschützter Form vorliegen und

$V^1$ eine geradkettige oder verzweigte C1-C6-Alkylgruppe, eine Benzyl-, Trimethylsilyl-, Triisopropylsilyl-, 2,2,2,-Trifluorethoxy- oder 2,2,2,-Trichlorethoxygruppe, wobei $V^1$ verschieden von $R^{1''}$ ist, mit einem Alkylierungsagenz der allgemeinen Formel III

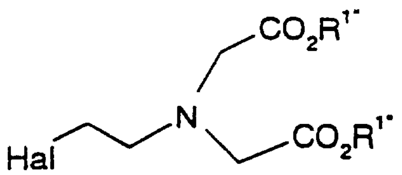

(III)

worin

$R^{1''}$ für eine Schutzgruppe und

Hal für ein Halogenatom wie Cl, Br oder I, bevorzugt jedoch Cl, steht,

umgesetzt wird [siehe auch M.A. Williams, H. Rapoport, J. Org. Chem. 58, 1151 (1993)].

Bevorzugte Aminosäurederivate sind die Ester von natürlich vorkommenden α-Aminosäuren.

Die Reaktion von Verbindung (II) mit Verbindung (III) erfolgt bevorzugt in einer gepufferten Alkylierungsreaktion, wobei als Puffer eine wäßrige Phosphat-Pufferlösung dient. Die Umsetzung erfolgt bei pH-Werten von 7-9, bevorzugt jedoch bei pH8. Die Pufferkonzentration kann zwischen 0,1 - 2,5 M liegen, bevorzugt wird jedoch eine 2 M-Phosphat-Pufferlösung verwendet. Die Temperatur der Alkylierung kann zwischen 0 und 50 °C liegen; die bevorzugte Temperatur ist Raumtemperatur.

Die Reaktion wird in einem polaren Lösungsmittel, wie z.B. Acetonitril, Tetrahydrofuran, 1,4-Dioxan oder 1,2-Dimethoxyethan durchgeführt. Bevorzugt wird Acetonitril verwendet.

Die Herstellung der erfindungsgemäßen pharmazeutischen Mittel erfolgt ebenfalls in an sich bekannter Weise, indem man die erfindungsgemäßen Komplexverbindungen - gegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie zum Beispiel Tromethamin), Zusätze von Komplexbildnern oder schwachen Komplexen (wie zum Beispiel Diethylentriaminpentaessigsäure oder die korrespondierenden Ca-Kaskaden-Polymer-Komplexe) oder - falls erforderlich - Elektrolyte wie zum Beispiel Natriumchlorid oder - falls erforderlich - Antioxidantien wie zum Beispiel Ascorbinsäure.

Sind für die enterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoff(en) [zum Beispiel Methylcellulose, Lactose, Mannit] und/oder Tensid(en) [zum Beispiel Lecithine, Tween®, Myrj® ] und/oder Aromastoff(en) zur Geschmackskorrektur [zum Beispiel ätherischen Ölen] gemischt.

Prinzipiell ist es auch möglich, die erfindungsgemäßen pharmazeutischen Mittel auch ohne Isolierung der Komplexsalze herzustellen. In jedem Fall muß besondere Sorgfalt darauf verwendet werden, die Chelatbildung so vorzunehmen, daß die erfindungsgemäßen Salze und Salzlösungen praktisch frei sind von nicht komplexierten toxisch wirkenden Metallionen.
Dies kann beispielsweise mit Hilfe von Farbindikatoren wie Xylenolorange durch Kontrolltitrationen während des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexsalzes.
Die erfindungsgemäßen pharmazeutischen Mittel enthalten vorzugsweise 1μMol - 1 Mol/l des Komplexsalzes und werden in der Regel in Mengen von 0,0001 - 5 mMol/kg dosiert. Sie sind zur enteralen und parenteralen Applikation bestimmt. Die erfindungsgemäßen Komplexverbindungen kommen zur Anwendung

1. für die NMR- und Röntgen-Diagnostik in Form ihrer Komplexe mit den Ionen der Elemente mit den Ordnungszahlen 21 - 29, 39, 42, 44 und 57 - 83;

2. für die Radiodiagnostik und Radiotherapie in Form ihrer Komplexe mit den Radioisotopen der Elemente mit den Ordnungszahlen 27, 29, 31, 32, 37 - 39, 43, 49, 62, 64, 70, 75 und 77.

Die erfindungsgemäßen Mittel erfüllen die vielfältigen Voraussetzungen für die Eignung als Kontrastmittel für die Kernspintomographie. So sind sie hervorragend dazu geeignet, nach oraler oder parenteraler Applikation durch Erhöhung der Signalintensität das mit Hilfe des Kernspintomographen erhaltene Bild in seiner Aussagekraft zu verbessern. Ferner zeigen sie die hohe Wirksamkeit, die notwendig ist, um den Körper mit möglichst geringen Mengen an Fremdstoffen zu belasten, und die gute Verträglichkeit, die notwendig ist, um den nichtinvasiven Charakter der Untersuchungen aufrechtzuerhalten.
Die gute Wasserlöslichkeit und geringe Osmolalität der erfindungsgemäßen Mittel erlaubt es, hochkonzentrierte Lösungen herzustellen, damit die Volumenbelastung des Kreislaufs in vertretbaren Grenzen zu halten und die Verdünnung durch die Körperflüssigkeit auszugleichen, das heißt NMR-Diagnostika müssen 100- bis 1000fach besser wasserlöslich sein als für die NMR-Spektroskopie. Weiterhin weisen die erfindungsgemäßen Mittel nicht nur eine hohe Stabilität in-vitro auf, sondern auch eine überraschend hohe Stabilität in-vivo, so daß eine Freigabe oder ein Austausch der in den Komplexen nicht kovalent gebundenen - an sich giftigen - Ionen innerhalb der Zeit, in der die neuen Kontrastmittel vollständig wieder ausgeschieden werden, nur äußerst langsam erfolgt.
Im allgemeinen werden die erfindungsgemäßen mittel für die Anwendung als NMR-Diagnostika in Mengen von 0,0001 - 5 mMol/kg, vorzugsweise 0,005 - 0,5 mMol/kg, dosiert. Details der Anwendung werden zum Beispiel in H.-J. Weinmann et al., Am. J. of Roentgenology 142, 619 (1984) diskutiert.
Besonders niedrige Dosierungen (unter 1 mg/kg Körpergewicht) von organspezifischen NMR-Diagnostika sind zum Beispiel zum Nachweis von Tumoren und von Herzinfarkt einsetzbar.
Ferner können die erfindungsgemäßen Komplexverbindungen vorteilhaft als Suszeptibilitäts-Reagenzien und als shift-Reagenzien für die in-vivo-NMR-Spektroskopie verwendet werden.
Die erfindungsgemäßen Mittel sind aufgrund ihrer günstigen radioaktiven Eigenschaften und der guten Stabilität der in ihnen enthaltenen Komplexverbindungen auch als Radiodiagnostika geeignet. Details ihrer Anwendung und Dosierung werden z.B. in "Radiotracers for Medical Applications", CRC-Press, Boca Raton, Florida, beschrieben.
Eine weitere bildgebende Methode mit Radioisotopen ist die Positronen-Emissions-Tomographie, die positronenemittierende Isotope wie z.B. $^{43}Sc$, $^{44}Sc$, $^{52}Fe$, $^{55}Co$ und $^{68}Ga$ verwendet (Heiss, W.D.; Phelps, M.E.; Positron Emission Tomography of Brain, Springer Verlag Berlin, Heidelberg, New York 1983).
Die erfindungsgemäßen Verbindungen sind überraschenderweise auch zur Differenzierung von malignen und benignen Tumoren in Bereichen ohne Blut-Hirn-Schranke geeignet.
Sie zeichnen sich auch dadurch aus, daß sie vollständig aus dem Körper eliminiert werden und somit gut verträglich sind.
Da sich die erfindungsgemäßen Substanzen in malignen Tumoren anreichern (keine Diffusion in gesunde Gewebe, aber hohe Durchlässigkeit von Tumorgefäßen), können sie auch die Strahlentherapie von malignen Tumoren unterstützen. Diese unterscheidet sich von der entsprechenden Diagnostik nur durch die Menge und Art des verwendeten Isotops. Ziel ist dabei die Zerstörung von Tumorzellen durch energiereiche kurzwellige Strahlung mit einer möglichst geringen Reichweite. Hierzu werden Wechselwirkungen der in den Komplexen enthaltenen Metalle (wie z.B. Eisen oder Gadolinium) mit ionisierenden Strahlungen (z.B. Röntgenstrahlen) oder mit Neutronenstrahlen ausgenutzt. Durch diesen Effekt wird die lokale Strahlendosis am Ort, wo sich der Metallkomplex befindet (z.B. in Tumoren) signifikant erhöht. Um die gleiche Strahlendosis im malignen Gewebe zu erzeugen, kann bei Anwendung solcher Metallkomplexe die Strahlenbelastung für gesunde Gewebe erheblich reduziert und damit belastende Nebenwirkungen für die Patienten vermieden werden. Die erfindungsgemäßen Metallkomplex-Konjugate eignen sich deshalb auch als radiosensibilisierende Substanz bei Strahlentherapie von malignen Tumoren (z.B. Ausnutzen von Mössbauer-Effekten

oder bei Neutroneneinfangtherapie). Geeignete β-emittierende Ionen sind zum Beispiel $^{46}Sc$, $^{47}Sc$, $^{48}Sc$, $^{72}Ga$, $^{73}Ga$ und $^{90}Y$. Geeignete geringe Halbwertzeiten aufweisende α-emittierende Ionen sind zum Beispiel $^{211}Bi$, $^{212}Bi$, $^{213}Bi$ und $^{214}Bi$, wobei $^{212}Bi$ bevorzugt ist. Ein geeignetes Photonen- und Elektronenemittierendes Ion ist $^{158}Gd$, das aus $^{157}Gd$ durch Neutroneneinfang erhalten werden kann.

Ist das erfindungsgemäße Mittel zur Anwendung in der von R.L. Mills et al. (Nature Vol. 336, (1988), S. 787] vorgeschlagenen Variante der Strahlentherapie bestimmt, so muß sich das Zentralion von einem Mößbauer-Isotop wie beispielsweise $^{57}Fe$ oder $^{151}Eu$ ableiten.

Bei der in-vivo-Applikation der erfindungsgemäßen therapeutischen Mittel können diese zusammen mit einem geeigneten Träger wie zum Beispiel Serum oder physiologischer Kochsalzlösung und zusammen mit einem anderen Protein wie zum Beispiel Human Serum Albumin verabreicht werden. Die Dosierung ist dabei abhängig von der Art der zellulären Störung, dem benutzten Metallion und der Art der bildgebenden Methode.

Die erfindungsgemäßen therapeutischen Mittel werden parenteral, vorzugsweise i.v., appliziert.

Details der Anwendung von Radiotherapeutika werden z.B. in R.W. Kozak et al. TIBTEC, Oktober 1986, 262, diskutiert.

Die erfindungsgemäßen Mittel sind hervorragend als Röntgenkontrastmittel geeignet, wobei besonders hervorzuheben ist, daß sich mit ihnen keine Anzeichen der von den jodhaltigen Kontrastmitteln bekannten anaphylaxieartigen Reaktionen in biochemischpharmakologischen Untersuchungen erkennen lassen. Besonders wertvoll sind sie wegen der günstigen Absorptionseigenschaften in Bereichen höherer Röhrenspannungen für digitale Substraktionstechniken.

Im allgemeinen werden die erfindungsgemäßen Mittel für die Anwendung als Röntgenkontrastmittel in Analogie zu zum Beispiel Meglumin-Diatrizoat in Mengen von 0,1 - 5 mMol/kg, vorzugsweise 0,25 - 1 mMol/kg, dosiert.

Details der Anwendung von Röntgenkontrastmitteln werden zum Beispiel in Barke, Röntgenkontrastmittel, G. Thieme, Leipzig (1970) und P. Thurn, E. Bücheler "Einführung in die Röntgendiagnostik", G. Thieme, Stuttgart, New York (1977) diskutiert.

Insgesamt ist es gelungen, neue Komplexbildner, Metallkomplexe und Metallkomplexsalze zu synthetisieren, die neue Möglichkeiten in der diagnostischen und therapeutischen Medizin erschließen.

Die nachfolgenden Beispiele dienen zur näheren Erläuterung des Erfindungsgegenstands:

**Beispiel 1**

a) 1,4,7-Tris(N,N'-dibenzyloxycarbonyl-lysyl)-1,4,7,10-tetraazacyclododecan

49,07 g (95,9 mmol) Di-Z-Lysin-N-hydroxysuccinimidester und 5 g (29 mmol) Cyclen (= 1,4,7,10-tetraazacyclododecan) werden in einer Mischung aus 200 ml Toluol/100 ml Dioxan gelöst. Man setzt 9,7 g (95,9 mmol) Triethylamin zu und erhitzt 12 Stunden auf 70°C. Man dampft zur Trockne ein, nimmt den Rückstand in 600 ml Dichlormethan auf und extrahiert 3 mal mit je 200 ml 5 %iger aqu. Kaliumcarbonat-Lösung. Die organische Phase wird über Magnesiumsulfat getrocknet und im Vakuum zur Trockne eingedampft. Der Rückstand wird an Kieselgel chromatographiert (Laufmittel: Ethylacetat/Ethanol = 15:1).
Ausbeute: 29,61 g (75 % d. Th.) eines farblosen Feststoffes

| Elementaranalyse: | | | |
|---|---|---|---|
| ber. | C 65,28 | H 6,81 | N 10,29 |
| gef. | C 65,41 | H 6,97 | N 10,10 |

b) 1-(Carboxymethoxyacetyl)-4,7,10-tris(N,N'-dibenzyloxycarbonyl-lysyl)-1,4,7,10-tetraazacyclododecan

Zu 28 g (20,56 mmol) der Titelverbindung aus Beispiel 1a (gelöst in 200 ml Tetrahydrofuran) gibt man 3,58 g (30,86 mmol) Diglycolsäureanhydrid und 6,24 g (61,72 mmol) Triethylamin. Man erwärmt 6 Stunden auf 50 °C. Die Lösung wird im Vakuum zur Trockne eingedampft, mit 300 ml Dichlormethan aufgenommen und zweimal mit je 150 ml 5 %iger aqu. Salzsäure extrahiert. Die organische Phase wird über Magnesiumsulfat getrocknet, im Vakuum zur Trockne eingedampft und der Rückstand an Kieselgel chromatographiert (Laufmittel: Dichlormethan/Methanol= 20:1).
Ausbeute: 27,65 g (91 % d. Th.) eines farblosen Feststoffes

| Elementaranalyse: | | | |
|---|---|---|---|
| ber. | C 63,40 | H 6,55 | N 9,48 |
| gef. | C 63,21 | H 6,70 | N 9,27 |

c) 1-[5-(4-Nitrophenoxy)-3-oxaglutaryl]-4,7,10-tris(N,N'-dibenzyloxycarbonyl-lysyl)-1,4,7,10-tetraazacyclododecan

14,78 g (10 mmol) der im Beispiel 1b beschriebenen Carbonsäure, gelöst in 150 ml Dichlormethan, werden zunächst mit 1,53 g (11 mmol) 4-Nitrophenol und anschließend bei 0 °C mit 2,27 g (11 mmol) Dicyclohexylcarbodiimid versetzt. Nach Rühren über Nacht bei Raumtemperatur wird vom Dicyclohexylharnstoff abgesaugt, das Filtrat eingeengt und aus Isopropanol umgefällt. Vom ölig anfallenden Produkt wird die Mutterlauge abdekantiert, das Öl in Dichlormethan aufgenommen und im Vakuum eingeengt. Man erhält 15,4 g (96,3 %) schaumig erstarrten Feststoff.

| Elementaranalyse: | | | |
|---|---|---|---|
| ber. | C 63,11 | H 6,24 | N 9,64 |
| gef. | C 62,98 | H 6,31 | N 9,80 |

d) Bis[2-(benzyloxycarbonylamino)-ethyl]-amin

51,5 g (500 mmol) Diethylentriamin und 139 ml (1 mol) Triethylamin werden in Dichlormethan gelöst und bei -20 °C mit 161 g Benzylcyanformiat (Fluka) in Dichlormethan versetzt und anschließend über Nacht bei Raumtemperatur gerührt. Nach Beendigung der Reaktion wird im Abzug eingedampft, der Rückstand in Diethylether aufgenommen, die organische Phase mit Natriumcarbonatlösung gewaschen und mit Natriumsulfat getrocknet. Das Filtrat wird mit Hexan versetzt, der Niederschlag abfiltriert und getrocknet.
Ausbeute: 163,4 g (88 % d. Th.)

| Elementaranalyse: | | | |
|---|---|---|---|
| ber. | C 64,67 | H 6,78 | N 11,31 |
| gef. | C 64,58 | H 6,83 | N 11,28 |

e) N,N,N',N',N",N"-Hexakis[2-(benzyloxycarbonylamino)-ethyl]-trimesinsäuretriamid

13,27 g (50 mmol) Trimesinsäure-trichlorid (Aldrich) und 34,7 ml (250 mmol) Triethylamin werden in Dimethylformamid (DMF) gelöst und bei 0 °C mit 65,0 g (175 mmol) des in Beispiel 1d beschriebenen Amins versetzt und anschließend über Nacht bei Raumtemperatur gerührt. Die Lösung wird im Vakuum eingedampft und der Rückstand mit Ethylacetat an Kieselgel chromatographiert.
Ausbeute: 39,4 g (62 % d. Th.)

| Elementaranalyse: | | | |
|---|---|---|---|
| ber. | C 65,24 | H 5,95 | N 9,92 |
| gef. | C 65,54 | H 5,95 | N 9,87 |

f) Vollgeschütztes Benzyloxycarbonyl-36mer-Polyamin, aufgebaut aus N,N,N',N',N",N"-Hexakis(2-aminoethyl)-trimesinsäuretriamid-Core und sechs im Beispiel 1b beschriebenen amingeschützten Hexaamin-monocarbonsäuren

1,27 g (1 mmol) des im Beispiel 1e beschriebenen Hexa-Benzyloxycarbonylamins werden in Eisessig gelöst und unter Rühren mit 33 %igem Bromwasserstoff in Eisessig versetzt. Nach 60 Minuten wird mit Diethylether die begonnene Fällung vervollständigt, das entstandene Hexa-amin-hydrobromid mit Ether gewaschen, im Vakuum getrocknet und ohne weitere Reinigung weiter umgesetzt.
Ausbeute: 0,95 g (quantitativ)
Anschließend wird das Hexa-amin-hydrobromid in 150 ml DMF gelöst, mit 15,99 g (10 mmol) des in Beispiel 1c beschriebenen 4-Nitrophenylaktivesters und mit 4,05 g (40 mmol) Triethylamin versetzt, über Nacht bei Raumtemperatur gerührt und schließlich im Vakuum zur Trockne eingedampft. Der Rückstand wird in Ethylacetat aufgenommen und nacheinander mit Wasser, verd. Natronlauge und gesättigter NaCl-Lösung gewaschen. Die organische Phase wird über Natriumsulfat getrocknet und das Filtrat zur Trockne eingedampft und der Rückstand an Kieselgel (Laufmittel: Dichlormethan/Methanol 18:2) chromatographiert.
Ausbeute: 6,55 g (71 % d. Th.) eines farblosen Feststoffes

| Elementaranalyse: | | | |
|---|---|---|---|
| ber. | C 63,68 | H 6,59 | N 10,48 |
| gef. | C 63,83 | H 6,70 | N 10,29 |

MALDI-TOF-Massenspektrum: Molpeak bei 9246 (M + $Na^+$)

g) 1-(Benzyloxycarbonylmethyl)-4,7,10-tris(tert.-butoxycarbonylmethyl)-1,4,7,10-tetraazacyclododecan (als Natriumbromid-Komplex)

20 g (38,87 mmol) 1,4,7-tris(tert.-butoxy-carbonylmethyl)-1,4,7,10-tetraazacyclododecan (DO3A-tris-tert.-Butylester, hergestellt nach EP 0 299 795, Beispiel 22a werden in 100 ml Acetonitril gelöst. Dann werden 11,45 g (50 mmol) Bromessigsäurebenzylester und 10,6 g (100 mmol) Natriumcarbonat zugegeben und 12 Stunden bei 60 °C gerührt. Man filtriert von den Salzen ab, dampft das Filtrat im Vakuum zur Trockne ein und chromatographiert den Rückstand an Kieselgel (Laufmittel: Methylenchlorid/Methanol = 20:1).
Ausbeute: 21,72 g (73 % d. Th.) eines farblosen amorphen Pulvers

| Elementaranalyse: | | | | | |
|---|---|---|---|---|---|
| ber. | C 54,90 | H 7,63 | N 7,32 | Na 3,00 | Br 10,44 |
| gef. | C 54,80 | H 7,72 | N 7,21 | Na 2,89 | Br 10,27 |

h) 1-(Carboxymethyl)-4,7,10-tris(tert.-butoxycarbonylmethyl)-1,4,7,10-tetraazacyclododecan (als Natriumbromid-Komplex)

20 g (26,12 mmol) der Titelverbindung aus Beispiel 1g werden in 300 ml Isopropanol gelöst und 3 g Palladiumkatalysator (10 % Pd/C) zugegeben. Man hydriert über Nacht bei Raumtemperatur. Man filtriert vom Katalysator ab und dampft das Filtrat zur Trockne ein. Ausbeute: 17,47 g (99 % d. Th.) eines farblosen amorphen Pulvers

| Elementaranalyse: | | | | | |
|---|---|---|---|---|---|
| ber. | C 49,78 | H 7,76 | N 8,29 | Na 4,44 | Br 11,83 |
| gef. | C 49,59 | H 7,59 | N 8,17 | Na 4,40 | Br 11,70 |

i) 1-(4-Carboxy-2-oxo-3-azabutyl)-4,7,10-tris(tert.-butoxycarbonylmethyl)-1,4,7,10-tetraazacyclododecan

Zu 10 g (14,80 mmol) der Titelverbindung aus Beispiel 1h in 100 ml Dimethylformamid gibt man 1,73 g (15 mmol) N-Hydroxysuccinimid und kühlt auf 0 °C. Dann gibt man 4,13 g (20 mmol) Dicyclohexylcarbodiimid zu und rührt 1 Stunde bei 0 °C, dann 2 Stunden bei Raumtemperatur. Man kühlt auf 0 °C ab und gibt anschließend 5,1 g (50 mmol) Triethylamin und 2,25 g (30 mmol) Glycin zu. Man rührt über Nacht bei Raumtemperatur. Man filtriert vom ausgefallenen Harnstoff ab und dampft das Filtrat im Vakuum zur Trockne ein.

Der Rückstand wird mit Wasser aufgenommen und zwei mal mit Methylenchlorid extrahiert. Die organische Phase wird über Magnesiumsulfat getrocknet und im Vakuum eingedampft. Der Rückstand wird an Kieselgel chromatographiert (Laufmittel: Methylenchlorid/Methanol = 15:1).
Ausbeute: 8,20 g (88 % d. Th.) eines farblosen Feststoffes

| Elementaranalyse: | | | |
|---|---|---|---|
| ber. | C 57,21 | H 8,80 | N 11,12 |
| gef. | C 57,10 | H 8,91 | N 11,03 |

k) 36mer-N-(5-DO3A-yl-4-oxo-3-azapentanoyl)-Kaskadenpolyamid auf der Basis des im Beispiel 1f beschriebenen 36mer-Polyamins [DO3A= 1,4,7-Tris(carboxymethyl)-1,4,7,10-tetraazacyclododecan]

1,84 (0,2 mmol) des im Beispiel 1f beschriebenen 36mer-Benzyloxycarbonylamins werden in Eisessig gelöst und unter Rühren mit 33 %igem Bromwasserstoff in Eisessig versetzt. Nach 5 Stunden wird mit Diethylether die begonnene Fällung vervollständigt, das entstandene 36mer-Amin-hydrobromid mit Ether gewaschen, im Vakuum getrocknet und ohne weitere Reinigung in die weiter unten beschriebene Reaktion eingesetzt.
Ausbeute: 1,5 g (quantitativ)

14,7 g (20 mmol) der in Beispiel 1i beschriebenen Carbonsäure, 3,0 g (20 mmol) 1-Hydroxybenzotriazol und 6,4 g (20 mmol) 2-(1H-Benzotriazol-1-yl)-1,1,3,3-tetramethyluronium tetrafluorborat (TBTU; Peboc Limited, UK) werden in DMF gelöst und 15 Minuten gerührt. Die Lösung wird anschließend mit 10,3 ml (60 mmol) N-Ethyldiisopropylamin und mit 1,5 g (0,2 mmol) des oben beschriebenen 36mer-Amin-hydrobromids versetzt und 4 Tage bei Raumtemperatur gerührt. Nach Beendigung der Reaktion wird im Vakuum eingedampft, der Rückstand bei 0 °C in Trifluoressigsäure gelöst, über Nacht bei Raumtemperatur gerührt, im Vakuum eingedampft und der Rückstand mit Ether verrührt. Die Substanz wird abgesaugt, mit Ether gewaschen, im Vakuum getrocknet, schließlich in Wasser gelöst, mit 2 N Natronlauge auf pH 7 eingestellt und die Lösung über eine Amicon®-Ultrafiltrationsmembran YM3 (cut off: 3000 Da) gereinigt. Das Retentat wird anschließend filtriert und gefriergetrocknet.
Ausbeute: 3,61 g (72 % d. Th) flockiges Pulver
$H_2O$-Gehalt (Karl-Fischer): 8,9 %

| Elementaranalyse (bezogen auf wasserfreie Substanz): | | | | |
|---|---|---|---|---|
| ber. | C 44,86 | H 5,87 | N 15,34 | Na 10,92 |
| gef. | C 45,09 | H 5,80 | N 15,44 | Na 10,51 |

l) 36mer-Gd-Komplex des im vorstehenden Beispiel beschriebenen Liganden

2,5 g (0,1 mmol) des im vorstehenden Beispiel 1k) beschriebenen Natriumsalzes der Komplexbildnersäure werden in Wasser mit 5 ml Eisessig angesäuert, mit 725 mg (2 mmol) $Gd_2O_3$ versetzt und 2 Stunden bei 80 °C komplexiert. Nach dem Abkühlen wird die Lösung filtriert, das Filtrat über YM3 ultrafiltriert (AMICON®) und das Retentat durch Zugabe von abwechselnd Kationenaustauscher IR 120 ($H^+$-Form) und Anionenaustauscher IRA 410 ($OH^-$-Form) auf minimale Leitfähigkeit eingestellt. Es wird vom Austauscher abfiltriert und das Filtrat gefriergetrocknet.
Ausbeute: 1,96 g (70 % d. Th.) farbloses, flockiges Pulver
$H_2O$-Gehalt (Karl-Fischer): 7,4 %
Gd-Bestimmung (AAS): 19,9 %
MALDI-TOF-Massenspektrum: Molpeak bei 25.905 Da (ber.: 25.911 Da)

| Elementaranalyse (bezogen auf wasserfreie Substanz): | | | | |
|---|---|---|---|---|
| ber. | C 39,35 | H 5,15 | Gd 21,85 | N 13,46 |
| gef. | C 39,08 | H 5,29 | Gd 21,03 | N 13,68 |

| | |
|---|---|
| $T_1$-Relaxivität ($H_2O$) | 18,0 ± 0,2 (l/mmol · sec) |
| (Plasma) | 21,5 ± 0,5 (l/mmol · sec) |

Gesamtkörper-Retention nach intravenöser Gabe (0,1 mmol Gadolinium/kg Körpergewicht; nach 14 Tagen; Ratte): 1,09 ± 0,17 % der Dosis.

Der entsprechende Europium-Komplex zeigte folgende Werte:

| | |
|---|---|
| Kaninchen | 0,23 ± 0,12 % der Dosis |
| Maus | 0,46 ± 0,1 % der Dosis |

**Beispiel 2**

a) 2-Brompropionylglycin-benzylester

Zu 100 g (296,4 mmol) Glycinbenzylester-p-Toluolsulfonsäuresalz und 33,0 g (326,1 mmol) Triethylamin in 400 ml Methylenchlorid tropft man bei 0 °C 55,9 g (326,1 mmol) 2-Brompropionsäurechlorid zu. Man läßt die Temperatur nicht über 5°C kommen. Nach beendeter Zugabe wird 1 Stunde bei 0°C gerührt, anschließend 2 Stunden bei Raumtemperatur. Man setzt 500 ml Eiswasser zu und stellt die Wasserphase mit 10 % aqu. Salzsäure auf pH 2. Die organische Phase wird abgetrennt, je einmal mit 300 ml 5 % aqu. Sodalösung und 400 ml Wasser gewaschen. Man trocknet die organische Phase über Magnesiumsulfat und dampft im Vakuum zur Trockne ein. Der Rückstand wird aus Diisopropylether umkristallisiert.
Ausbeute: 68,51 g (75 % d. Th.) eines farblosen kristallinen Pulvers

| Elementaranalyse: | | | | |
|---|---|---|---|---|
| ber. | C 46,76 | H 7,19 | N 4,54 | Br 25,92 |
| gef. | C 46,91 | H 7,28 | N 4,45 | Br 25,81 |

b) 1-[4-(Benzyloxycarbonyl)-1-methyl-2-oxo-3-azabutyl]-1,4,7,10-tetraazacyclododecan

Zu 55,8 g (324,4 mmol) 1,4,7,10-Tetraazacyclododecan, gelöst in 600 ml Chloroform, gibt man 50 g (162,2 mmol) der Titelverbindung aus Beispiel 2a und rührt über Nacht bei Raumtemperatur. Man gibt 500 ml Wasser zu, trennt die organische Phase ab und wäscht sie noch jeweils zweimal mit 400 ml Wasser. Man trocknet die organische Phase über Magnesiumsulfat und dampft im Vakuum zur Trockne ein. Der Rückstand wird an Kieselgel chromatographiert (Laufmittet: Chloroform/Methanol/aqu. 25 % Ammoniak = 10:5:1).
Ausbeute. 40,0 g (63 % d. Th.bezogen auf eingesetztes 2a) eines leicht gelblichen zähen Öls

| Elementaranalyse: | | | |
|---|---|---|---|
| ber. | C 61,36 | H 8,50 | N 17,89 |
| gef. | C 61,54 | H 8,68 | N 17,68 |

c) 1-[4-(Benzyloxycarbonyl)-1-methyl-2-oxo-3-azabutyl]-4,7,10-tris(tert.-butoxycarbonylmethyl)-1,4,7,10-tetraazacyclododecan (Natriumbromid-Komplex)

Zu 20 g (51,08 mmol) der Titelverbindung aus Beispiel 2b und 17,91 (169 mmol) Natriumcarbonat in 300 ml Acetonitril gibt man 33 g (169 mmol) Bromessigsäure-tert.-butylester zu und rührt 24 Stunden bei 60 °C. Man kühlt auf 0 °C ab, filtriert von den Salzen ab und dampft das Filtrat zur Trockne ein. Der Rückstand wird an Kieselgel chromatographiert (Laufmittel: Ethylacetat/Ethanol: 15:1). Die das Produkt enthaltenden Fraktionen werden eingedampft und der Rückstand aus Diisopropylether umkristallisiert.
Ausbeute: 34,62 g (81 % d. Th.) eines farblosen kristallinen Pulvers

| Elementaranalyse: | | | | | |
|---|---|---|---|---|---|
| ber. | C 54,54 | H 7,59 | N 8,37 | Na 2,74 | Br 9,56 |
| gef. | C 54,70 | H 7,65 | N 8,24 | Na 2,60 | Br 9,37 |

d) 1-(4-Carboxy-1-methyl-2-oxo-3-azabutyl)-4,7,10-tris(tert.-butoxycarbonylmethyl)-1,4,7,10-tetraazacyclododecan (Natriumbromid-Komplex)

30 g (35,85 mmol) der Titelverbindung aus Beispiel 2c werden in 500 ml Isopropanol gelöst und 3 g Palladiumkatalysator (10 % Pd/C) hinzugegeben. Man hydriert über Nacht bei Raumtemperatur. Es wird vom Katalysator abfiltriert, das Filtrat im Vakuum zur Trockne eingedampft und aus Aceton umkristallisiert.
Ausbeute: 22,75 g (85 % d. Th.) eines farblosen kristallinen Pulvers

| Elementaranalyse: | | | | | |
|---|---|---|---|---|---|
| ber. | C 49,86 | H 7,69 | N 9,38 | Na 3,07 | Br 10,71 |
| gef. | C 49,75 | H 7,81 | N 9,25 | Na 2,94 | Br 10,58 |

e) 1-[4-(4-Nitrophenoxycarbonyl)-1-methyl-2-oxo-3-azabutyl]-4,7,10-tris(tert.-butoxycarbonylmethyl)-1,4,7,10-tetraazacyclododecan (Natriumbromid-Komplex)

37,3 g (50 mmol) der im vorstehenden Beispiel 2d beschriebenen Carbonsäure werden in 500 ml Dichlormethan mit 7,6 g (55 mmol) 4-Nitrophenol versetzt und auf 0 °C gekühlt. Nach Zugabe von 10,8 g (52,5 mmol) Dicyclohexylcarbodiimid wird über Nacht bei Raumtemperatur gerührt, anschließend unter erneuter Kühlung vom ausgefallenen Dicyclohexylharnstoff abgesaugt und das Filtrat im Vakuum zur Trockne eingedampft. Der Rückstand wird aus Ethylacetat umkristallisiert.

Ausbeute: 40,3 g (92,9 % d. Th.) leicht gelbliches Pulver

| Elementaranalyse: | | | | | |
|---|---|---|---|---|---|
| ber. | C 51,21 | H 6,97 | N 9,68 | Na 2,65 | Br 9,21 |
| gef. | C 51,06 | H 7,07 | N 9,82 | Na 2,40 | Br 8,77 |

f) 36mer-N-(5-DO3A-yl-4-oxo-3-azahexanoyl)-Kaskadenpolyamid auf der Basis des im Beispiel 1f beschriebenen 36mer-Polyamins

1,84 g (0,2 mmol) des im Beispiel 1f beschriebenen 36mer-Benzyloxycarbonylamins werden in Eisessig gelöst und unter Rühren mit 33 %igem Bromwasserstoff in Eisessig versetzt. Nach 5 Stunden wird mit Diethylether die begonnene Fällung vervollständigt, das entstandene 36mer-Amin-hydrobromid mit Ether gewaschen, im Vakuum getrocknet und ohne weitere Reinigung umgesetzt.
Ausbeute: 1,5 g (quantitativ)
1,5 g des oben beschriebenen 36mer Amin-hydrobromids in 100 ml DMF werden mit 17,4 g (20 mmol) des in Beispiel 2e beschriebenen p-Nitrophenyl-aktivesters versetzt. Innerhalb einer Stunde wird anschließend eine Lösung von 5,05 g (50 mmol) Triethylamin in 20 ml DMF so langsam zugetropft, daß der sich zu Anfang bildende Niederschlag wieder in Lösung gehen kann. Man rührt über Nacht bei 45 °C, anschließend wird die Lösung im Vakuum eingeengt, der Rückstand bei 0 °C in Trifluoressigsäure gelöst und über Nacht bei Raumtemperatur gerührt. Es wird im Vakuum eingedampft, der Rückstand mit Diethylether verrührt, der Niederschlag abgesaugt und im Vakuum getrocknet. Das saure Rohprodukt wird schließlich in Wasser gelöst, mit verdünnter Natronlauge auf pH 7 gestellt und über eine AMICON® YM-3 Membran ultrafiltriert. Das Retentat wird gefriergetrocknet. Ausbeute: 4,0 g (78 % d. Th.)
$H_2O$-Gehalt (Karl-Fischer): 9,3 %

| Elementaranalyse (bezogen auf wasserfreie Substanz): | | | | |
|---|---|---|---|---|
| ber. | C 45,74 | H 6,05 | N 15,01 | Na 10,68 |
| gef. | C 45,84 | H 5,93 | N 15,22 | Na 10,20 |

g) 36mer-Gd-Komplex des im vorstehenden Beispiel beschriebenen Liganden

2,5 g (0,1 mmol) des im vorstehenden Beispiel 2f beschriebenen Natriumsalzes der Komplexbildnersäure werden in Wasser mit 5 ml Eisessig angesäuert, mit 725 mg (2 mmol) $Gd_2O_3$ versetzt und 2 Stunden bei 80 °C komplexiert. Nach dem Abkühlen wird die Lösung filtriert, das Filtrat über YM3 ultrafiltriert (AMICON®) und das Retentat durch Zugabe von abwechselnd Kationenaustauscher IR 120 ($H^+$-Form) und Anionenaustauscher IRA 410 ($OH^-$-Form) auf minimale Leitfähigkeit eingestellt. Es wird vom Austauscher abfiltriert und das Filtrat gefriergetrocknet.
Ausbeute: 2,14 g (74 % d. Th.) farbloses, flockiges Pulver
$H_2O$-Gehalt (Karl-Fischer): 8,7 %
Gd-Bestimmuna (AAS): 19,4 %
MALDI-TOF-Massenspektrum: Molpeak bei 26.426 Da (ber.: 26.416 Da)

| Elementaranalyse (bezogen auf wasserfreie Substanz): | | | | |
|---|---|---|---|---|
| ber. | C 40,24 | H 5,32 | Gd 21,43 | N 13,20 |
| gef. | C 39,97 | H 5,50 | Gd 21,19 | N 13,32 |

| | |
|---|---|
| $T_1$-Relaxivität ($H_2O$) | $17,5 \pm 0,1$ (l/mmol · sec) |
| (Plasma) | $18,2 \pm 0,2$ (l/mmol · sec) |

Gesamtkörper-Retention nach intravenöser Gabe (0,1 mmol Gadolinium/kg Körpergewicht; nach 14 Tagen; Ratte): $1,74 \pm 0,22$ % der Dosis.
Der entsprechende Europium-Komplex zeigte folgende Werte:

| | |
|---|---|
| Kaninchen | $0,32 \pm 0,16$ % der Dosis |
| Maus | $1,0 \pm 0,1$ % der Dosis |

**Beispiel 3**

a) 1,4,7-Tris(N-benzyloxycarbonylglycyl)-1,4,7,10-tetraazacyclododecan

29,37 g (95,9 mmol) Z-Glycin-N-hydroxysuccinimidester und 5 g (29 mmol) Cyclen (= 1,4,7,10-tetraazacyclododecan) werden in einer Mischung aus 100 ml Toluol/50 ml Dioxan gelöst. Man setzt 9,7 g (95,9 mmol) Triethylamin zu und erhitzt 12 Stunden auf 70 °C. Man dampft zur Trockne ein, nimmt den Rückstand in 400 ml Dichlormethan auf und extrahiert 3 mal mit je 200 ml 5 %iger aqu. Kaliumcarbonat-Lösung. Die organische Phase wird über Magnesiumsulfat getrocknet und im Vakuum zur Trockne eingedampft. Der Rückstand wird an Kieselgel chromatographiert (Laufmittel: Ethylacetat/Ethanol = 15:1).
Ausbeute: 17,52 g (81 % d. Th.) eines farblosen Feststoffes

| Elementaranalyse: | | | |
|---|---|---|---|
| ber. | C 61,20 | H 6,35 | N 13,15 |
| gef. | C 61,07 | H 6,45 | N 13,01 |

b) 1-(Carboxymethoxyacetyl)-4,7,10-tris(N-benzyloxycarbonylglycyl)-1,4,7,10-tetraazacyclododecan

Zu 17 g (22,79 mmol) der Titelverbindung aus Beispiel 3a (gelöst in 100 ml Tetrahydrofuran) gibt man 3,97 g (34,19 mmol) Diglycolsäureanhydrid und 6,92 g (68,38 mmol) Triethylamin. Man erwärmt 6 Stunden auf 50 °C. Die Lösung wird im Vakuum zur Trockne eingedampft, mit 250 ml Dichlormethan aufgenommen und zweimal mit je 100 ml 5 %iger aqu. Salzsäure extrahiert. Die organische Phase wird über Magnesiumsulfat getrocknet, im Vakuum zur Trockne eingedampft und der Rückstand an Kieselgel chromatographiert (Laufmittet: Dichlormethan/Methanol = 20:1).
Ausbeute: 17,48 g (89 % d. Th.) eines farblosen Feststoffes

| Elementaranalyse: | | | |
|---|---|---|---|
| ber. | C 58,53 | H 5,96 | N 11,38 |
| gef. | C 58,37 | H 5,81 | N 11,45 |

c) Bis[2-($N_\alpha$,$N_\varepsilon$-dibenzyloxycarbonyl-lysylamino)-ethyl]-amin

1,03 g (10 mmol) Diethylentriamin werden in 100 ml THF gelöst, mit 2,02 g (2,77 ml, 20 mmol) Triethylamin und 11,25 g (21 mmol) N,N'-Di-benzyloxycarbonyl-lysin-p-nitrophenylester [O.W. Lever et al., J. Heterocyclic Chem., 23, 901 - 903 (1986)] versetzt und 3 Stunden bei Raumtemperatur gerührt. Die entstandene dicke Suspension wird mit Ether auf 250 ml aufgefüllt, über Nacht bei Raumtemperatur gerührt, der voluminöse Niederschlag abgesaugt und mit 100 ml THF/ Ether (1:1) und anschließend nochmals mit Ether gewaschen. Nach Trocknung im Vakuum bei 40 °C erhält man 8.7 g (97,1 % d. Th.) farbloses Pulver.

| Elementaranalyse: | | | |
|---|---|---|---|
| ber. | C 64,34 | H 6,86 | N 10,94 |
| gef. | C 64,20 | H 6,97 | N 10,81 |

d) N,N,N',N',N",N"-Hexakis[2-($N_\alpha$,$N_\varepsilon$-dibenzyloxycarbonyl-lysylamino)-ethyl]-trimesinsäuretriamid

1,43 g (1.6 mmol) Bis[2-($N_\alpha$,$N_\varepsilon$-dibenzyloxycarbonyl-lysylamino)-ethyl]-amin in 20 ml DMF werden bei 0 °C mit 1,39 ml (1,01 g, 10 mmol) Triethylamin und 0,11 g (0,4 mmol) Trimesinsäuretrichlorid (Aldrich) versetzt, 2 Stunden im Eis und über Nacht bei Raumtemperatur gerührt. Es wird anschließend im Vakuum eingeengt, in Ethylacetat aufgenommen und mit verd. Natronlauge, 1 M Salzsäure und halbgesättigter NaCl-Lösung gewaschen und über Natriumsulfat getrocknet. Nach Zusatz von Aktivkohle wird über Teflon-Membranfilter abfiltriert, das Filtrat eingeengt (1,5 g), erneut in ca. 5 ml Ethylaceta gelöst und mit Ethylacetat/Methanol (18:2) an Kieselgel chromatographiert
Ausbeute: 0,9 g (79,1 %) farbloses Pulver

| Elementaranalyse: | | | |
|---|---|---|---|
| ber. | C 64,61 | H 6,48 | N 10,34 |

(fortgesetzt)

| Elementaranalyse: | | | |
|---|---|---|---|
| gef. | C 64,45 | H 6,60 | N 10,28 |

e) Vollgeschütztes Benzyloxycarbonyl -36mer-Polyamin, aufgebaut aus N,N,N',N',N",N"-Hexakis[2-(lysylamino)-ethyl]-trimesinsäure-triamid-Core und zwölf im Beispiel 3b beschriebenen amingeschützten Triamin-monocarbonsäuren

2,84 g (1 mmol) des im vorstehenden Beispiel 3d beschriebenen 12mer-Benzyloxycarbonylamins werden in Eisessig gelöst und unter Rühren mit 33 %igem Bromwasserstoff in Eisessig versetzt. Nach 3 Stunden wird mit Diethylether die begonnene Fällung vervollständigt, das entstandene 12mer-Amin-hydrobromid mit Ether gewaschen, im Vakuum getrocknet und ohne weitere Reinigung in die weiter unten beschriebene Reaktion eingesetzt.
Ausbeute: 2,2 g (quantitativ)

17,2 g (20 mmol) der in Beispiel 3b beschriebenen Cyclen-carbonsäure, 3,0 g (20 mmol) 1-Hydroxybenzotriazol und 6,4 g (20 mmol) 2-(1H-Benzotriazol-1-yl)-1,1,3,3-tetramethyluronium tetrafluorborat (TBTU; Peboc Limited, UK) werden in DMF gelöst und 15 Minuten gerührt. Die Lösung wird anschließend mit 10,3 ml (60 mmol) N-Ethyldiisopropylamin und mit 2,2 g (1 mmol) des oben beschriebenen 12mer-Amin-hydrobromids versetzt und über Nacht bei Raumtemperatur gerührt. Nach Beendigung der Reaktion wird im Vakuum eingedampft und der Rückstand mit Dichlormethan/Methanol (17:3) an Kieselgel chromatographiert.
Ausbeute: 9,6 g (84,5 % d. Th) farbloses Pulver

| Elementaranalyse: | | | |
|---|---|---|---|
| ber. | C 59,31 | H 6,20 | N 12,94 |
| gef. | C 59,20 | H 6,03 | N 13,19 |

MALDI-TOF-Massenspektrum: Molpeak bei 11.384 ($M+Na^+$)

f) 36mer-N-(5-DO3A-yl-4-oxo-3-azapentanoyl)-Kaskadenpolyamid auf der Basis des im Beispiel 3e beschriebenen 36mer-Polyamins

2,27 (0,2 mmol) des im Beispiel 3e beschriebenen 36mer-Benzyloxycarbonylamins werden in Eisessig gelöst und unter Rühren mit 33 %igem Bromwasserstoff in Eisessig versetzt. Nach 5 Stunden wird mit Diethylether die begonnene Fällung vervollständigt, das entstandene 36mer-Amin-hydrobromid mit Ether gewaschen, im Vakuum getrocknet und ohne weitere Reinigung in die weiter unten beschriebene Reaktion eingesetzt.
Ausbeute: 1,9 g (quantitativ)

14,7 g (20 mmol) der in Beispiel 1i beschriebenen Carbonsäure, 3,0 g (20 mmol) 1-Hydroxybenzotriazol und 6,4 g (20 mmol) 2-(1H-Benzotriazol-1-yl)-1,1,3,3-tetramethyluronium tetrafluorborat (TBTU; Peboc Limited, UK) werden in DMF gelöst und 15 Minuten gerührt. Die Lösung wird anschließend mit 10,3 ml (60 mmol) N-Ethyldiisopropylamin und mit 1,9 g (0,2 mmol) des oben beschriebenen 36mer-Amin-hydrobromids versetzt und 4 Tage bei Raumtemperatur gerührt. Nach Beendigung der Reaktion wird im Vakuum eingedampft, der Rückstand bei 0 °C in Trifluoressigsäure gelöst, über Nacht bei Raumtemperatur gerührt, im Vakuum eingedampft und der Rückstand mit Ether verrührt. Die Substanz wird abgesaugt, mit Ether gewaschen, im Vakuum getrocknet, schließlich in Wasser gelöst, mit 2 N Natronlauge auf pH 7 eingestellt und die Lösung über eine Amicon®-Ultrafiltrationsmembran YM 3 (cut off: 3000 Da) gereinigt. Das Retentat wird anschließend filtriert und gefriergetrocknet.
Ausbeute: 3,93 g (75 % d. Th) flockiges Pulver
$H_2O$-Gehalt (Karl-Fischer): 5,0 %

| Elementaranalyse (bezogen auf wasserfreie Substanz): | | | | |
|---|---|---|---|---|
| ber. | C 44,48 | H 5,75 | N 16,05 | Na 9,98 |
| gef. | C 44,77 | H 5,91 | N 15,96 | Na 9,50 |

g) 36mer-Gd-Komplex des im vorstehenden Beispiel 3f beschriebenen Liganden

2,6 g (0,1 mmol) des im vorstehenden Beispiel 3f beschriebenen Natriumsalzes der Komplexbildnersäure

werden in Wasser mit 5 ml Eisessig angesäuert, mit 725 mg (2 mmol) $Gd_2O_3$ versetzt und 2 Stunden bei 80 °C komplexiert. Nach dem Abkühlen wird die Lösung filtriert, das Filtrat über YM3 ultrafiltriert (AMICON®) und das Retentat durch Zugabe von abwechselnd Kationenaustauscher IR 120 ($H^+$-Form) und Anionenaustauscher IRA 410 ($OH^-$-Form) auf minimale Leitfähigkeit eingestellt. Es wird vom Austauscher abfiltriert und das Filtrat gefriergetrocknet.
Ausbeute: 2,22 g (72 % d. Th.) farbloses, flockiges Pulver
$H_2O$-Gehalt (Karl-Fischer): 8,9 %
Gd-Bestimmung (AAS): 18,5 %
MALDI-TOF-Massenspektrum: Molpeak bei 28.058 Da (ber.: 28.049 Da)

| Elementaranalyse (bezogen auf wasserfreie Substanz): | | | | |
|---|---|---|---|---|
| ber. | C 39,44 | H 5,10 | Gd 20,18 | N 14,23 |
| gef. | C 39,56 | H 5,26 | Gd 19,88 | N 14,09 |

**Beispiel 4**

a) 36mer-N-(5-DO3A-yl-4-oxo-3-azahexanoyl)-Kaskadenpolyamid auf der Basis des im Beispiel 3e beschriebenen 36mer-Polyamins

2,27 g (0,2 mmol) des im Beispiel 3e beschriebenen 36mer-Benzyloxycarbonylamins werden in Eisessig gelöst und unter Rühren mit 33 %igem Bromwasserstoff in Eisessig versetzt. Nach 5 Stunden wird mit Diethylether die begonnene Fällung vervollständigt, das entstandene 36mer-Amin-hydrobromid mit Ether gewaschen, im Vakuum getrocknet und ohne weitere Reinigung weiter umgesetzt.
Ausbeute: 1,9 g (quantitativ)

1,9 g des oben beschriebenen 36mer Amin-hydrobromids in 100 ml DMF werden mit 17,4 g (20 mmol) des in Beispiel 2e beschriebenen p-Nitrophenyl-aktivesters versetzt. Innerhalb einer Stunde wird anschließend eine Lösung von 5,05 g (50 mmol) Triethylamin in 20 ml DMF so langsam zugetropft, daß der sich zu Anfang bildende Niederschlag wieder in Lösung gehen kann. Man rührt über Nacht bei 45 °C, anschließend wird die Lösung im Vakuum eingeengt, der Rückstand bei 0 °C in Trifluoressigsäure gelöst und über Nacht bei Raumtemperatur gerührt. Es wird im Vakuum eingedampft, der Rückstand mit Diethylether verrührt, der Niederschlag abgesaugt und im Vakuum getrocknet. Das saure Rohprodukt wird schließlich in Wasser gelöst, mit verdünnter Natronlauge auf pH 7 gestellt und über eine AMICON® YM-3 Membran ultrafiltriert. Das Retentat wird gefriergetrocknet. Ausbeute: 4,0 g (72,9 % d. Th.)
$H_2O$-Gehalt (Karl-Fischer): 7,5 %

| Elementaranalyse (bezogen auf wasserfreie Substanz): | | | | |
|---|---|---|---|---|
| ber. | C 45,30 | H 5,92 | N 15,73 | Na 9,78 |
| gef. | C 45,56 | H 6,10 | N 15,65 | Na 9,47 |

b) 36mer-Gd-Komplex des im vorstehenden Beispiel 4a beschriebenen Liganden

2,74 g (0,1 mmol) des im vorstehenden Beispiel 4a beschriebenen Natriumsalzes der Komplexbildnersäure werden in Wasser mit 5 ml Eisessig angesäuert, mit 725 mg (2 mmol) $Gd_2O_3$ versetzt und 2 Stunden bei 80 °C komplexiert. Nach dem Abkühlen wird die Lösung filtriert, das Filtrat über YM3 ultrafiltriert (AMICON®) und das Retentat durch Zugabe von abwechselnd Kationenaustauscher IR 120 ($H^+$-Form) und Anionenaustauscher IRA 410 ($OH^-$-Form) auf minimale Leitfähigkeit eingestellt. Es wird vom Austauscher abfiltriert und das Filtrat gefriergetrocknet.
Ausbeute: 2,46 g (77,8 % d. Th.) farbloses, flockiges Pulver
$H_2O$-Gehalt (Karl-Fischer): 9,7 %
Gd-Bestimmung (AAS): 18,1 %
MALDI-TOF-Massenspektrum: Molpeak bei 28.563 Da (ber.: 28.554 Da)

| Elementaranalyse (bezogen auf wasserfreie Substanz): | | | | |
|---|---|---|---|---|
| ber. | C 40,26 | H 5,26 | Gd 19,83 | N 13,98 |
| gef. | C 40,01 | H 5,40 | Gd 19,68 | N 14,11 |

**Beispiel 5**

a) 1,7-Bis(benzyloxycarbonyl)-4-hydroxysuccinyl-1,4,7-triazaheptan

Zu 50 g (134,6 mmol) 1,7-Bis(benzyloxycarbonyl)-1,4,7,-triazaheptan (Beispiel 1d) in 500 ml Tetrahydrofuran gibt man 20,20 g (201,9 mmol) Bernsteinsäureanhydrid und 40,86 g (403,8 mmol) Triethylamin zu und rührt über Nacht bei 40 °C. Man dampft zur Trockne ein, nimmt den Rückstand in 1000 ml Dichlormethan auf und wäscht zweimal mit je 500 ml 5 %iger Salzsäure. Die organische Phase wird über Magnesiumsulfat getrocknet und zur Trockne eingedampft. Der Rückstand wird an Kieselgel chromatographiert (Laufmittel = Dichlormethan/Methanol: 20:1).
Ausbeute: 56,0 g (93 % d. Th.) eines farblosen Feststoffes

| Elementaranalyse: | | | |
|---|---|---|---|
| ber. | C 59,05 | H 6,53 | N 9,39 |
| gef. | C 59,17 | H 6,69 | N 9,27 |

b) N-Hydroxysuccinimidester von 1,7-Bis(benzyloxycarbonyl)-4-hydroxysuccinyl-1,4,7-triazaheptan

Zu 56 g (125,14 mmol) der Titelverbindung aus Beispiel 5a in 300 ml Dichlormethan gibt man 14,4 g (125,14 mmol) N-Hydroxysuccinimid. Man kühlt auf 0 °C und gibt 28,4 g (137,66 mmol) Dicyclohexylcarbodiimid zu. Anschließend rührt man 6 Stunden bei Raumtemperatur. Man filtriert vom ausgefallenen Feststoff ab und dampft das Filtrat im Vakuum zur Trockne ein. Der Rückstand wird aus Ether/2-Propanol umkristallisiert.
Ausbeute: 62,01 g (91 % d. Th.) eines kristallinen farblosen Feststoffes

| Elementaranalyse: | | | |
|---|---|---|---|
| ber. | C 57,35 | H 5,92 | N 10,29 |
| gef. | C 57,24 | H 5,99 | N 10,12 |

c) 1,4,7-Tris{7-benzyloxycarbonylamino-5-[2-(benzyloxycarbonylamino)-ethyl]-4-oxo5-azaheptanoyl}1,4,7,10-tetraazacyclododecan

52,22 g (95,9 mmol) der Titelverbindung aus Beispiel 5b und 5 g (29 mmol) Cyclen (= 1,4,7,10-tetraazacyclododecan) werden in einer Mischung aus 200 ml Toluol/100 ml Dioxan gelöst. Man setzt 9,7 g (95,9 mmol) Triethylamin zu und erhitzt 12 Stunden auf 70 °C. Man dampft zur Trockne ein, nimmt den Rückstand in 600 ml Dichlormethan auf und extrahiert dreimal mit je 300 ml 5 %iger aqu. Kaliumcarbonat-Lösung. Die organische Phase wird über Magnesiumsulfat getrocknet und im Vakuum zur Trockne eingedampft. Der Rückstand wird an Kieselgel chromatographiert (Laufmittel: Ethylacetat/Ethanol = 15:1).
Ausbeute: 28,95 g (69 % d. Th.) eines farblosen Feststoffes

| Elementaranalyse: | | | |
|---|---|---|---|
| ber. | C 61,44 | H 7,04 | N 11,62 |
| gef. | C 61,57 | H 6,91 | N 11,69 |

d) 1,4,7-Tris-{7-benzyloxycarbonylamino-5-[2-(benzyloxycarbonylamino)-ethyl]-4-oxo5-azaheptanoyl}-10-hydroxysuccinyl-1,4,7,10-tetraazacyclododecan

Zu 28 g (19,35 mmol) der Titelverbindung aus Beispiel 5c, gelöst in Tetrahydrofuran, gibt man 2,90 g (29 mmol) Bernsteinsäureanhydrid und 5,87 g (58 mmol) Triethylamin. Man erwärmt 6 Stunden auf 50 °C. Die Lösung wird im Vakuum zur Trockne eingedampft, mit 200 ml Dichlormethan aufgenommen und zweimal mit je 100 ml 5 %iger aqu. Salzsäure extrahiert. Die organische Phase wird über Magnesiumsulfat getrocknet, im Vakuum zur Trockne eingedampft und der Rückstand an Kieselgel chromatographiert (Laufmittel: Dichlormethan/Methanol = 20:1).
Ausbeute: 26,94 g (90 % d. Th.) eines farblosen Feststoffes

| Elementaranalyse: | | | |
|---|---|---|---|
| ber. | C 60,57 | H 6,84 | N 10,87 |

(fortgesetzt)

| Elementaranalyse: | | | |
|---|---|---|---|
| gef. | C 60,41 | H 6,95 | N 10,75 |

e) 1,4,7,10,13,16-Hexakis[N-benzyloxycarbonyl-β-alanyl]-1,4,7,10,13.16-hexaazacyclooctadecan

516 mg (2 mmol) 1,4,7,10,13,16-Hexaazacyclooctadecan (Hexacyclen; Fluka) werden mit Toluol azeotrop entwässert. Zu der abgekühlten Lösung von Hexacyclen in Toluol wird bei Raumtemperatur eine Lösung von 3,35 g (15 mmol) Benzyloxycarbonyl-β-alanin (Sigma) in Tetrahydrofuran (THF) sowie 3,71 g (15 mmol) 2-Ethoxy-1-ethoxycarbonyl-1,2-dihydrochinolin (EEDQ; Fluka) zugegeben und über Nacht gerührt. Nach Beendigung der Reaktion wird das Produkt durch Zugabe von Hexan ausgefällt und der Niederschlag mit Dichlormethan/Hexan/Isopropanol (20:10:1) an Kieselgel chromatographiert.
Ausbeute: 2,06 g (69 % d. Th.)

| Elementaranalyse: | | | |
|---|---|---|---|
| ber. | C 62,89 | H 6,50 | N 11,28 |
| gef. | C 62,74 | H 6,32 | N 11,50 |

f) Vollgeschütztes Benzyloxycarbonyl-36mer-Polyamin, aufgebaut aus 1,4,7,10,13,16-Hexakis(β-alanyl)-1,4,7,10,13,16-hexaazacyclooctadecan-Core und sechs im Beispiel 5d beschriebenen amingeschützten Hexaamin-monocarbonsäuren

1,49 g (1 mmol) des im vorstehenden Beispiel 5e beschriebenen Hexa-Benzyloxycarbonylamins werden in Eisessig gelöst und unter Rühren mit 33 %igem Bromwasserstoff in Eisessig versetzt. Nach 60 Minuten wird mit Diethylether die begonnene Fällung vervollständigt, das entstandene Hexamin-hydrobromid mit Ether gewaschen, im Vakuum getrocknet und ohne weitere Reinigung in die weiter unten beschriebene Reaktion eingesetzt. Ausbeute: 1,2 g (quantitativ)

7,0 g (7,5 mmol) der in Beispiel 5d beschriebenen amingeschützten Hexa-amin-monocarbonsäure, 1,2 g (7,5 mmol) 1-Hydroxybenzotriazol und 2,4 g (7,5 mmol) 2-(1H-Benzotriazol-1-yl)-1,1,3,3-tetramethyluronium tetrafluorborat (TBTU; Peboc Limited, UK) werden in DMF gelöst und 15 Minuten gerührt. Diese Lösung wird anschließend mit 5,16 ml (30 mmol) N-Ethyldiisopropylamin und mit 1,2 g (1 mmol) des oben beschriebenen Hexa-amin-hydrobromids versetzt und über Nacht bei Raumtemperatur gerührt. Nach Beendigung der Reaktion wird im Vakuum eingedampft und der Rückstand mit Dichlormethan/Methanol (17:3) an Kieselgel chromatographiert.
Ausbeute: 8,5 g (82 % d. Th.) eines farblosen Pulvers

| Elementaranalyse: | | | |
|---|---|---|---|
| ber. | C 61,83 | H 6,59 | N 12,15 |
| gef. | C 61,59 | H 6,71 | N 12,02 |

MALDI-TOF-Massenspektrum: Molpeak bei 10.397 ($M+Na^+$)

g) 36mer-N-(5-DO3A-yl-4-oxo-3-azahexanoyl)-Kaskadenpolyamid auf der Basis des im Beispiel 5e beschriebenen Polyamins

2,07 g (0,2 mmol) des in Beispiel 5f beschriebenen 36mer-Benzyloxycarbonylamins werden in Eisessig gelöst und unter Rühren mit 33 %igem Bromwasserstoff in Eisessig versetzt. Nach 5 Stunden wird mit Diethylether die begonnene Fällung vervollständigt, das entstandene 36mer-Amin-hydrobromid mit Ether gewaschen und im Vakuum getrocknet und ohne weitere Reinigung umgesetzt.
Ausbeute: 1,7 g (quantitativ)

1,7 g des oben beschriebenen 36mer Amin-hydrobromids in 100 ml DMF werden mit 17,4 g (20 mmol) des in Beispiel 2e beschriebenen p-Nitrophenyl-aktivesters versetzt. Innerhalb einer Stunde wird anschließend eine Lösung von 5,05 g (50 mmol) Triethylamin in 20 ml DMF so langsam zugetropft, daß der sich zu Anfang bildende Niederschlag wieder in Lösung gehen kann. Man rührt über Nacht bei 45 °C, anschließend wird die Lösung im Vakuum eingeengt, der Rückstand bei 0 °C in Trifluoressigsäure gelöst und über Nacht bei Raumtemperatur gerührt. Es wird im Vakuum

eingedampft, der Rückstand mit Diethylether verrührt, der Niederschlag abgesaugt und im Vakuum getrocknet. Das saure Rohprodukt wird schließlich in Wasser gelöst, mit verdünnter Natronlauge auf pH 7 gestellt und über eine AMICON® YM-3 Membran ultrafiltriert. Das Retentat wird gefriergetrocknet. Ausbeute: 4,4 g (83 % d. Th.)
$H_2O$-Gehalt (Karl-Fischer): 7,8 %

| Elementaranalyse (bezogen auf wasserfreie Substanz): | | | | |
|---|---|---|---|---|
| ber. | C 45,80 | H 6,08 | N 15,51 | Na 10,18 |
| gef. | C 45,88 | H 6,23 | N 15,66 | Na 9,70 |

h) 36mer-Gd-Komplex des im vorstehenden Beispiel 5g beschriebenen Liganden

2,65 g (0,1 mmol) des im vorstehenden Beispiel 5g beschriebenen Natriumsalzes der Komplexbildnersäure werden in Wasser mit 5 ml Eisessig angesäuert, mit 725 mg (2 mmol) $Gd_2O_3$ versetzt und 2 Stunden bei 80 °C komplexiert. Nach dem Abkühlen wird die Lösung filtriert, das Filtrat über YM3 ultrafiltriert (AMICON®) und das Retentat durch Zugabe von abwechselnd Kationenaustauscher IR 120 ($H^+$-Form) und Anionenaustauscher IRA 410 ($OH^-$-Form) auf minimale Leitfähigkeit eingestellt. Es wird vom Austauscher abfiltriert und das Filtrat gefriergetrocknet.
Ausbeute: 2,41 g (81 % d. Th.) farbloses, flockiges Pulver
$H_2O$-Gehalt (Karl-Fischer): 7,5 %
Gd-Bestimmung (AAS): 18,7 %
MALDI-TOF-Massenspektrum: Molpeak bei 27.580 Da (ber.: 27.566 Da)

| Elementaranalyse (bezogen auf wasserfreie Substanz): | | | | |
|---|---|---|---|---|
| ber. | C 40,52 | H 5,37 | Gd 20,54 | N 13,72 |
| gef. | C 40,30 | H 5,50 | Gd 20,11 | N 13,56 |

**Beispiel 6**

36mer-Gd-DTPA-monoamid auf der Basis des im Beispiel 5f beschriebenen 36mer-Polyamins

1,04 g (0,2 mmol) des in Beispiel 5f beschriebenen 36mer-Poly-Benzyloxycarbonylamins werden in Eisessig gelöst und unter Rühren mit 33 %igem Bromwasserstoff in Eisessig versetzt. Nach 3 Stunden wird mit Diethylether die begonnene Fällung vervollständigt, das entstandene 36mer-Amin-hydrobromid mit Ether gewaschen und im Vakuum getrocknet. Der Rückstand wird anschließend in Wasser aufgenommen und durch Zugabe von 1 N Natronlauge auf pH 9,5 eingestellt. In diese Lösung werden 4,35 g (10,8 mmol) $N^3$-(2,6-Dioxomorpholinoethyl)-$N^6$-(ethoxycarbonylmethyl)-3,6-diazaoctandisäure (Beispiel 13a der EP 0331 616) in fester Form zugegeben, wobei der pH durch weitere Zugabe von Natronlauge bei 9,5 konstant gehalten wird. Nach Beendigung der Zugabe wird zur Verseifung des DTPA-ethylesters mit 5 N Natronlauge ein pH > 13 eingestellt und über Nacht bei Raumtemperatur gerührt. Dann wird mit konz. Salzsäure auf pH 5 eingestellt, m 1,96 g (5,4 mmol) $Gd_2O_3$ versetzt, 30 Minuten bei 80 °C gerührt, nach dem Abkühlen auf pH 7 eingestellt und über eine YM3 AMICON-Ultrafiltrationsmembran entsalzt. Das Retentat wird schließlich membranfiltriert und gefriergetrocknet.
Ausbeute: 2,58 g (92,4 % d. Th.)
$H_2O$-Gehalt (Karl-Fischer): 9,0 %
Gd-Bestimmung (AAS): 20,3 %

| Elementaranalyse (bezogen auf wasserfreie Substanz): | | | | | |
|---|---|---|---|---|---|
| ber. | C 35,46 | H 4,26 | Gd 22,29 | N 10,92 | Na 3,26 |
| gef. | C 35,18 | H 4,44 | Gd 21,75 | N 10,83 | Na 3,59 |

**Beispiel 7**

a) 5-Benzyloxycarbonylamino-2-[3-(benzyloxycarbonylamino)-propyl]-valerinsäure

Zu 10 g (57,39 mmol) 4-Carboxy-1,7-Diaminoheptan (hergestellt nach: A. Reissert, Chem. Ber. 26, 2137 (1893); 27, 979 (1894)) in 150 ml Wasser tropft man bei 0 °C gleichzeitig 24,48 g (143, 5 mmol) Chlorameisensäurebenzylester und 5 N aqu. Natronlauge zu und hält den pH-Wert bei 10. Man rührt über Nacht bei Raumtemperatur. Es

wird zweimal mit je 150 ml Essigsäureethylester extrahiert. Die Wasserphase wird vorsichtig mit 4 N aqu. Salzsäure angesäuert (pH 2) und dreimal mit je 200 ml Essigsäureethylester extrahiert. Die organische Phase wird über Magnesiumsulfat getrocknet und im Vakuum zur Trockne eingedampft.
Ausbeute: 24,13 g (95 % d. Th.) eines glasigen Feststoffes

| Elementaranalyse: | | | |
|---|---|---|---|
| ber. | C 59,05 | H 6,53 | N 9,39 |
| gef. | C 59,19 | H 6,71 | N 9,18 |

b) 5-Benzyloxycarbonylamino-2-[3-(benzyloxycarbonylamino)-propyl]-valerinsäure-N-hydroxysuccinimidester

Zu 24 g (54,24 mmol) der Titelverbindung aus Beispiel 7a gelöst in 100 ml Dichlormethan gibt man 6,24 g (54,24 mmol) N-Hydroxysuccinimid. Man kühlt auf 0 °C und gibt 12,31 g (59,66 mmol) Dicyclohexylcarbodiimid zu. Anschließend rührt man 6 Stunden bei Raumtemperatur. Man filtriert vom ausgefallenen Feststoff ab und dampft das Filtrat im Vakuum zur Trockne ein. Der Rückstand wird aus Ether/2-Propanol umkristallisiert.
Ausbeute: 27,51 g (94 % d. Th.) eines kristallinen, farblosen Feststoffes

| Elementaranalyse: | | | |
|---|---|---|---|
| ber. | C 62,33 | H 6,16 | N 7,79 |
| gef. | C 62,17 | H 6,03 | N 7,85 |

c) 1,4,7-Tris{5-benzyloxycarbonylamino-2-[3-(benzyloxycarbonylamino)-propyl]-valeryl}-1,4,7,10-tetraazacyclododecan

27 g (50,04 mmol) der Titelverbindung aus Beispiel 7b und 2,61 g (15,16 mmol) Cyclen (= 1,4,7,10-tetraazacyclododecan) werden in einer Mischung aus 100 ml Toluol/50 ml Dioxan gelöst. Man setzt 3,07 g (30,32 mmol) Triethylamin zu und erhitzt 12 Stunden auf 70°°C. Man dampft zur Trockne ein, nimmt den Rückstand in 300 ml Dichlormethan auf und extrahiert dreimal mit je 150 ml 5 %iger aqu. Kaliumcarbonat-Lösung. Die organische Phase wird über Magnesiumsulfat getrocknet und im Vakuum zur Trockne eingedampft. Der Rückstand wird an Kieselgel chromatographiert (Laufmittel: Essigsäureethylester/Ethanol = 15:1).
Ausbeute: 13,81 g (63 % d. Th.) eines farblosen Feststoffes

| Elementaranalyse: | | | |
|---|---|---|---|
| ber. | C 66,46 | H 7,26 | N 9,69 |
| gef. | C 66,28 | H 7,39 | N 9,51 |

d) 1-[Carboxy-methoxyacetyl)-4,7,10-tris{5-benzyloxycarbonylamino-2-[3-benzyloxycarbonylamino)-propyl]-valeryl}-1,4,7,10-tetraazacyclododecan

Zu 13 g (9 mmol) der Titelverbindung aus Beispiel 7c in 80 ml Tetrahydrofuran gibt man 1,57 g (13,5 mmol) Diglycolsäureanhydrid und 2,73 g (27 mmol) Triethylamin. Man erwärmt 6 Stunden auf 50 °C. Die Lösung wird im Vakuum zur Trockne eingedampft, mit 150 ml Dichlormethan aufgenommen und zweimal mit je 100 ml 5 %iger aqu. Salzsäure extrahiert. Die organische Phase wird über Magnesiumsulfat getrocknet, im Vakuum zur Trockne eingedampft und der Rückstand an Kieselgel chromatographiert (Laufmittel: Dichlormethan/Methanol = 20:1).
Ausbeute: 12,5 g (89 % d. Th.) eines farblosen Feststoffes

| Elementaranalyse: | | | |
|---|---|---|---|
| ber. | C 64,60 | H 6,97 | N 8,97 |
| gef. | C 64,41 | H 6,85 | N 8,90 |

e) Vollgeschütztes Benzyloxycarbonyl-36mer-Polyamin, aufgebaut aus N,N,N',N',N",N"-Hexakis(2-aminoethyl)-trimesinsäuretriamid-Core und sechs im Beispiel 7d beschriebenen amingeschützten Hexaamin-monocarbonsäuren

1,27 g (1 mmol) des im vorstehenden Beispiel 1e beschriebenen Hexa-Benzyloxycarbonylamins werden in

Eisessig gelöst und unter Rühren mit 33 %igem Bromwasserstoff in Eisessig versetzt. Nach 60 Minuten wird mit Diethylether die begonnene Fällung vervollständigt, das entstandene Hexamin-hydrobromid mit Ether gewaschen, im Vakuum getrocknet und ohne weitere Reinigung in die weiter unten beschriebene Reaktion eingesetzt.
Ausbeute: 0,95 g (quantitativ)

11,7 g (7,5 mmol) der in Beispiel 7d beschriebenen Cyclen-carbonsäure, 1,2 g (7,5 mmol 1-Hydroxybenzotriazol und 2,4 g (7,5 mmol) 2-(1H-Benzotriazol-1-yl)-1,1,3,3-tetramethy uronium tetrafluorborat (TBTU; Peboc Limited, UK) werden in DMF gelöst und 15 Minuten gerührt. Diese Lösung wird anschließend mit 5,16 ml (30 mmol) N-Ethyldiisopropylamin und mit 0,95 g (1 mmol) des oben beschriebenen Hexa-amin-hydrobromids ver setzt und über Nacht bei Raumtemperatur gerührt. Nach Beendigung der Reaktion wird im Vakuum eingedampft und der Rückstand mit Dichlormethan/Methanol (17:3) an Kieselgel chromatographiert.
Ausbeute: 7,40 g (76 % d. Th.) eines farblosen Pulvers

| Elementaranalyse: | | | |
|---|---|---|---|
| ber. | C 64,82 | H 6,99 | N 9,93 |
| gef. | C 64,58 | H 7,11 | N 10,04 |

MALDI-TOF-Massenspektrum: Molpeak bei 9751 ($M+Na^+$)

f) 36mer-N-(5-DO3A-yl-4-oxo-3-azahexanoyl)-Kaskadenpolyamid auf der Basis des im Beispiel 7e beschriebenen Polyamins

1,95 g (0,2 mmol) des in Beispiel 7e beschriebenen 36mer-Benzyloxycarbonylamins werden in Eisessig gelöst und unter Rühren mit 33 prozentigem Bromwasserstoff in Eisessig versetzt. Nach 5 Stunden wird mit Diethylether die begonnene Fällung vervollständigt, das entstandene 36mer-Amin-hydrobromid mit Ether gewaschen und im Vakuum getrocknet und ohne weitere Reinigung umgesetzt.
Ausbeute: 1,6 g (quantitativ)

1,6 g des oben beschriebenen 36mer Amin-hydrobromids in 100 ml DMF werden mit 17,4 g (20 mmol) des in Beispiel 2e beschriebenen p-Nitrophenyl-aktivesters versetzt. Innerhalb einer Stunde wird anschließend eine Lösung von 5,05 g (50 mmol) Triethylamin in 20 ml DMF so langsam zugetropft, daß der sich zu Anfang bildende Niederschlag wieder in Lösung gehen kann. Man rührt über Nacht bei 45 °C, anschließend wird die Lösung im Vakuum eingeengt, der Rückstand bei 0 °C in Trifluoressigsäure gelöst und über Nacht bei Raumtemperatur gerührt. Es wird im Vakuum eingedampft, der Rückstand mit Diethylether verrührt, der Niederschlag abgesaugt und im Vakuum getrocknet. Das saure Rohprodukt wird schließlich in Wasser gelöst, mit verdünnter Natronlauge auf pH 7 gestellt und über eine AMICON® YM-3 Membran ultrafiltriert. Das Retentat wird gefriergetrocknet.
Ausbeute: 3,9 g (76 % d. Th.)
$H_2O$-Gehalt (Karl-Fischer): 8,0 %

| Elementaranalyse (bezogen auf wasserfreie Substanz): | | | | |
|---|---|---|---|---|
| ber. | C 46,59 | H 6,23 | N 14,69 | Na 10,46 |
| gef. | C 46,82 | H 6,47 | N 14,55 | Na 10,19 |

g) 36mer-Gd-Komplex des im vorstehenden Beispiel beschriebenen Liganden

2,58 g (0,1 mmol) des im vorstehenden Beispiel 7f beschriebenen Natriumsalzes der Komplexbildnersäure werden in Wasser mit 5 ml Eisessig angesäuert, mit 725 mg (2 mmol) $Gd_2O_3$ versetzt und 2 Stunden bei 80 °C komplexiert. Nach dem Abkühlen wird die Lösung filtriert, das Filtrat über YM3 ultrafiltriert (AMICON®) und das Retentat durch Zugabe von abwechselnd Kationenaustauscher IR 120 ($H^+$-Form) und Anionenaustauscher IRA 410 ($OH^-$-Form) auf minimale Leitfähigkeit eingestellt. Es wird vom Austauscher abfiltriert und das Filtrat gefriergetrocknet.
Ausbeute: 2,08 g (72 % d. Th.) farbloses, flockiges Pulver
$H_2O$-Gehalt (Karl-Fischer): 7,0 %
Gd-Bestimmung (AAS): 19,3 %
MALDI-TOF-Massenspektrum: Molpeak bei 26.915 Da (ber.: 26.921 Da)

| Elementaranalyse (bezogen auf wasserfreie Substanz): | | | | |
|---|---|---|---|---|
| ber. | C 41,09 | H 5,49 | Gd 21,03 | N 12,96 |

(fortgesetzt)

| Elementaranalyse (bezogen auf wasserfreie Substanz): | | | | |
|---|---|---|---|---|
| gef. | C 41,20 | H 5,60 | Gd 20,66 | N 13,19 |

**Beispiel 8**

a) 3,5-Bis[4-(benzyloxycarbonyl)-2-oxo-1,4-diazabutyl]-benzoesäure

Zu 30 g (197,17 mmol) 3,5-Diaminobenzoesäure in 600 ml Dichlormethan gibt man 123,8 g (404,2 mmol) N-Z-Glycin-N-hydroxysuccinimidester. Bei 0 °C tropft man innerhalb von 5 Minuten 60,7 g (800 mmol) Triethylamin, gelöst in 100 ml Dichlormethan, zu und rührt über Nacht bei Raumtemperatur. Man extrahiert dreimal mit je 500 ml 10 %iger Salzsäure, trocknet die organische Phase über Magnesiumsulfat und dampft im Vakuum zur Trockne ein. Der Rückstand wird aus Aceton umkristallisiert.
Ausbeute: 97,87 g (95 % d. Th.) eines farblosen, kristallinen Feststoffes

| Elementaranalyse: | | | |
|---|---|---|---|
| ber. | C 59,77 | H 5,02 | N 10,72 |
| gef. | C 59,65 | H 5,17 | N 10,59 |

b) 3,5-Bis[4-(benzyloxycarbonyl-2-oxo-1,4-diazabutyl]-benzoesäure-N-hydroxysuccinimidester

Zu 60 g (114,8 mmol) der Titelverbindung aus Beispiel 8a gelöst in 300 ml Dichlormethan gibt man 13,21 g (114,8 mmol) N-Hydroxysuccinimid. Man kühlt auf 0 °C und gibt 26,06 g (126,3 mmol) Dicyclohexylcarbodiimid zu. Anschließend rührt man 6 Stunden bei Raumtemperatur. Man filtriert vom ausgefallenen Feststoff ab und dampft das Filtrat im Vakuum zur Trockne ein. Der Rückstand wird aus Ether/2-Propanol umkristallisiert.
Ausbeute: 65,44 g (92 % d. Th.) eines kristallinen, farblosen Feststoffes

| Elementaranalyse: | | | |
|---|---|---|---|
| ber. | C 58,16 | H 4,72 | N 11,30 |
| gef. | C 58,31 | H 4,90 | N 11,15 |

c) 1,4,7-Tris{3,5-bis-[4-benzyloxycarbonyl-2-oxo-1,4-diazabutyl]-benzoyl}-1,4,7,10-tetraazacyclododecan

60 g (96,84 mmol) der Titelverbindung aus Beispiel 8b und 5,05 g (29,34 mmol) Cyclen (= 1,4,7,10-tetraazacyclododecan) werden in einer Mischung aus 200 ml Toluol/100 ml Dioxan gelöst. Man setzt 5,94 g (58,68 mmol) Triethylamin zu und erhitzt 12 Stunden auf 70°°C. Man dampft zur Trockne ein, nimmt den Rückstand in 600 ml Dichlormethan auf und extrahiert dreimal mit je 300 ml 5 %iger aqu. Kaliumcarbonat-Lösung. Die organische Phase wird über Magnesiumsulfat getrocknet und im Vakuum zur Trockne eingedampft. Der Rückstand wird an Kieselgel chromatographiert (Laufmittel: Essigsäureethylester/Ethanol = 15:1).
Ausbeute: 31,65 g (64 % d. Th.) eines farblosen Feststoffes

| Elementaranalyse: | | | |
|---|---|---|---|
| ber. | C 61,27 | H 5,50 | N 13,29 |
| gef. | C 61,15 | H 5,61 | N 13,10 |

d) 1-(Carboxymethoxyacetyl)-4,7,10-tris{3,5-bis-[4-benzyloxycarbonyl-2-oxo-1,4-diazabutyl]-benzoyl}-1,4,7,10-tetraazacyclododecan

Zu 30 g (17,8 mmol) der Titelverbindung aus Beispiel 8c, gelöst in 150 ml Tetrahydrofuran, gibt man 3,1 g (26,7 mmol) Diglycolsäureanhydrid und 5,4 g (53,4 mmol) Triethylamin. Man erwärmt 6 Stunden auf 50 °C. Die Lösung wird im Vakuum zur Trockne eingedampft, mit 250 ml Dichlormethan aufgenommen und zweimal mit je 150 ml 5 %iger aqu. Salzsäure extrahiert. Die organische Phase wird über Magnesiumsulfat getrocknet, im Vakuum zur Trockne eingedampft und der Rückstand an Kieselgel chromatographiert (Laufmittel: Dichlormethan/Methanol = 20:1).
Ausbeute: 29,83 g (93 % d. Th.) eines farblosen Feststoffes

| Elementaranalyse: | | | |
|---|---|---|---|
| ber. | C 59,99 | H 5,37 | N 12,44 |
| gef. | C 59,81 | H 5,45 | N 12,29 |

e) Vollgeschütztes Benzyloxycarbonyl-36mer-Polyamin, aufgebaut aus N,N,N',N',N",N"-Hexakis(2-aminoethyl)-trimesinsäuretriamid-Core und sechs im Beispiel 8d beschriebenen amingeschützten Hexaamin-monocarbonsäuren

1,27 g (1 mmol) des im vorstehenden Beispiel 1e beschriebenen Hexa-Benzyloxycarbonylamins werden in Eisessig gelöst und unter Rühren mit 33 %igem Bromwasserstoff in Eisessig versetzt. Nach 60 Minuten wird mit Diethylether die begonnene Fällung vervollständigt, das entstandene Hexamin-hydrobromid mit Ether gewaschen, im Vakuum getrocknet und ohne weitere Reinigung in die weiter unten beschriebene Reaktion eingesetzt. Ausbeute: 0,95 g (quantitativ)

13,5 g (7,5 mmol) der im vorstehenden Beispiel 8d beschriebenen Cyclen-carbonsäure, 1,2 g (7,5 mmol) 1-Hydroxybenzotriazol und 2,4 g (7,5 mmol) 2-(1H-Benzotriazol-1-yl)-1,1,3,3-tetramethyluronium tetrafluorborat (TBTU; Peboc Limited, UK) werden in DMF gelöst und 15 Minuten gerührt. Diese Lösung wird anschließend mit 5,16 ml (30 mmol) N-Ethyldiisopropylamin und mit 0,95 g (1 mmol) des oben beschriebenen Hexa-amin-hydrobromids versetzt und über Nacht bei Raumtemperatur gerührt. Nach Beendigung der Reaktion wird im Vakuum eingedampft und der Rückstand mit Dichlormethan/Methanol (8:1) an Kieselgel chromatographiert.
Ausbeute: 8,75 g (81 % d. Th.) eines farblosen Pulvers

| Elementaranalyse: | | | |
|---|---|---|---|
| ber. | C 64,34 | H 5,62 | N 13,61 |
| gef. | C 64,22 | H 5,86 | N 13,51 |

MALDI-TOF-Massenspektrum: Molpeak bei 10.832 ($M+Na^+$)

f) 36mer-N-(5-DO3A-yl-4-oxo-3-azahexanoyl)-Kaskadenpolyamid auf der Basis des im Beispiel 8e beschriebenen Polyamins

2,16 g (0,2 mmol) des in Beispiel 8e beschriebenen 36mer-Benzyloxycarbonylamins werden in Eisessig gelöst und unter Rühren mit 33 %igem Bromwasserstoff in Eisessig versetzt. Nach 5 Stunden wird mit Diethylether die begonnene Fällung vervollständigt, das entstandene 36mer-Amin-hydrobromid mit Ether gewaschen und im Vakuum getrocknet und ohne weitere Reinigung umgesetzt.
Ausbeute: 1,8 g (quantitativ)

1,8 g des oben beschriebenen 36mer Amin-hydrobromids in 100 ml DMF werden mit 17,4 g (20 mmol) des in Beispiel 2e beschriebenen p-Nitrophenyl-aktivesters versetzt. Innerhalb einer Stunde wird anschließend eine Lösung von 5,05 g (50 mmol) Triethylamin in 20 ml DMF so langsam zugetropft, daß der sich zu Anfang bildende Niederschlag wieder in Lösung gehen kann. Man rührt über Nacht bei 45 °C, anschließend wird die Lösung im Vakuum eingeengt, der Rückstand bei 0°C in Trifluoressigsäure gelöst und über Nacht bei Raumtemperatur gerührt. Es wird im Vakuum eingedampft, der Rückstand mit Diethylether verrührt, der Niederschlag abgesaugt und im Vakuum getrocknet. Das saure Rohprodukt wird schließlich in Wasser gelöst, mit verdünnter Natronlauge auf pH 7 gestellt und über eine AMICON® YM-3 Membran ultrafiltriert. Das Retentat wird gefriergetrocknet.
Ausbeute: 4,6 g (84 % d. Th.)
$H_2O$-Gehalt (Karl-Fischer): 9,5 %

| Elementaranalyse (bezogen auf wasserfreie Substanz): | | | | |
|---|---|---|---|---|
| ber. | C 47,18 | H 5,66 | N 16,08 | Na 10,00 |
| gef. | C 47,31 | H 5,52 | N 16,30 | Na 9,57 |

g) 36mer-Gd-Komplex des im vorstehenden Beispiel beschriebenen Liganden

2,74 g (0,1 mmol) des im vorstehenden Beispiel 8f beschriebenen Natriumsalzes der Komplexbildnersäure werden in Wasser mit 5 ml Eisessig angesäuert, mit 725 mg (2 mmol) $Gd_2O_3$ versetzt und 2 Stunden bei 80 °C komplexiert. Nach dem Abkühlen wird die Lösung filtriert, das Filtrat über YM3 ultrafiltriert (AMICON®) und das Retentat

durch Zugabe von abwechselnd Kationenaustauscher IR 120 ($H^+$-Form) und Anionenaustauscher IRA 410 ($OH^-$-Form) auf minimale Leitfähigkeit eingestellt. Es wird vom Austauscher abfiltriert und das Filtrat gefriergetrocknet.
Ausbeute: 2,27 g (74 % d. Th.) farbloses, flockiges Pulver
$H_2O$-Gehalt (Karl-Fischer): 8,6 %
Gd-Bestimmung (AAS): 18,2 %
MALDI-TOF-Massenspektrum: Molpeak bei 27.992 Da (ber.: 28.001 Da)

| Elementaranalyse (bezogen auf wasserfreie Substanz): | | | | |
|---|---|---|---|---|
| ber. | C 41,82 | H 5,02 | Gd 20,22 | N 14,26 |
| gef. | C 41,99 | H 4,96 | Gd 19,87 | N 14,40 |

**Beispiel für einen in-vivo-Vergleich mit einem extrazellulären Kontrastmittel**

Die Eignung der im Beispiel 1l beschriebenen Verbindung als blood-pool-agent wird im folgenden Versuch gezeigt.

Als Versuchstiere dienen drei 200-250 g schwere, männliche (Schering-SPF-) Ratten. Je Tier werden 0.2 ml (jeweils 25 mmol/L) der folgenden Kontrastmittel-Lösung intravenös appliziert: Gemisch aus je 1 Teil der Verbindung aus 1l, im folgenden Verbindung 1 genannt, und dem Dysprosium-Komplex der 3,6,9-Triaza-3,6,9-tris(carboxymethyl)-undecandisäure (Dy-DTPA), im folgenden Verbindung 2 genannt. Über einen Katheter in der Arteria carotis communis werden Blutproben zu folgenden Zeitpunkten entnommen: 1, 3, 5, 10, 15, 20, 30, 45, 60, 90, 120 min p.i. In den gewonnenen Blutproben werden jeweils parallel die Konzentrationen an Gadolinium (Gd) und Dysprosium (Dy) mittels Atomemissionsspektrometrie (ICP-AES) gemessen. Der im Blutraum verbliebene Anteil der injizierten Kontrastmittel Verbindung 1 (Gd) und Verbindung 2 (Dy, Vergleichssubstanz) kann durch die unterschiedliche Markierung im gleichen Tier verglichen werden. Aus den Blutkonzentrationen kann mittels spezieller Software (Topfit-Programm) die a- und b-Halbwertzeit, das Verteilungsvolumen sowie die total Clearance errechnet werden. Damit liefern diese Daten Angaben über den Verbleib der Verbindungen im Intravasalraum, die Verteilungsverhältnisse im Organismus und die Eliminierung.

Ergebnisse: Vor allem zu den frühen Zeitpunkten werden deutlich höhere Blutkonzentrationen der Verbindung 1 verglichen mit dem extrazellulären Kontrastmittel (Verbindung 2) erhalten (siehe Figur 1).

Die deutlich höheren Blutkonzentrationen der Verbindung 1 zu den frühen Zeitpunkten (verglichen mit der Verbindung 2) weisen auf ein deutlich kleineres Verteilungsvolumen hin (siehe auch Vd ss), d.h. Verbindung 1 verteilt sich nicht wie Verbindung 2 im Intravasalraum (Gefäße) *und* im Extrazellularraum, sondern größtenteils *nur* im Intravasalraum. Im weiteren Verlauf fällt der Blutspiegel jedoch schnell ab und die Eliminations- oder β-Halbwertzeit von Verbindung 1 ist deutlich kürzer als bei anderen blood-pool-agents. Die totale Blut-Clearance von Verbindung 1 ist nur etwas geringer verglichen zu Verbindung 2, was auf eine ähnlich gute Nierenelimination schließen läßt.

Damit verfügt die im Beispiel 1l beschriebene Verbindung über die Erfordernisse an ein blood-pool-agent: effiziente Elimination aus dem Blut (über die Nieren), aber ein deutlich kleineres Verteilungsvolumen als ein extrazelluläres Kontrastmittel.

Fig. 1

Gemessene Blutkonzentrationen von Gd (Verbindung 1) und Dy (Verbindung 2) in Ratten (n=3)

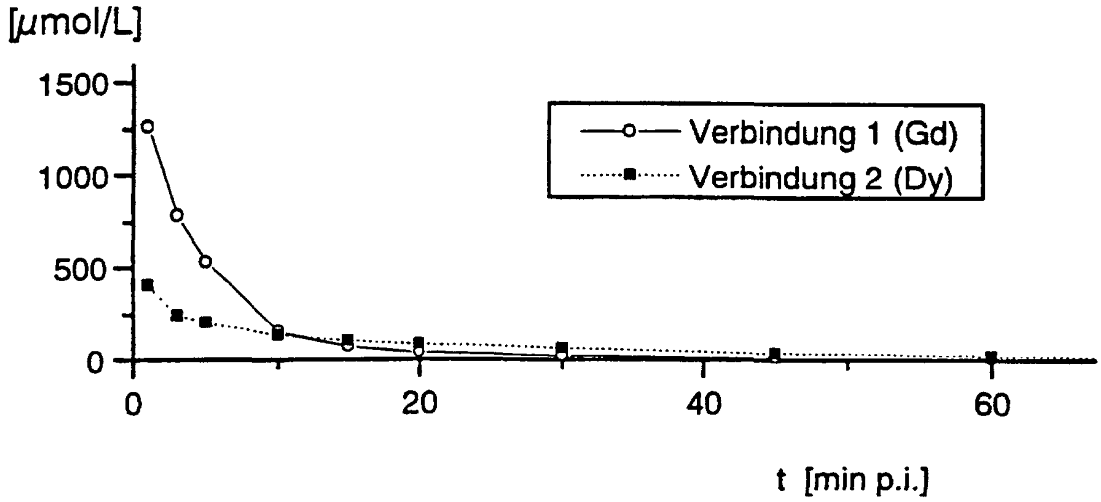


| | Verbindung 1 (Gd) | Verbindung 2 (Dy) | Einheit |
|---|---|---|---|
| α- t½ | 3.0 ± 0.6 | 1.5 ± 0.5 | min |
| β- t½ | 36.5 ± 18.2 | 19.2 ± 2.5 | min |
| Vd ss | 0.18 ± 0.07 | 0.41 ± 0.04 | L/kg |
| Total Clearance | 13.6 ± 1.5 | 16.8 ± 0.9 | ml/min*kg |

**Patentansprüche**

**1.** Kaskaden-Polymer-Komplexe enthaltend

a) komplexbildende Liganden der allgemeinen Formel I

$$A\text{-}\{X\text{-}[Y\text{-}(Z\text{-}\langle W\text{-}K_w\rangle_z)_y]_x\}_a \quad (I),$$

worin

| | |
|---|---|
| A | für einen stickstoffhaltigen Kaskadenkern der Basismultiplizität a, |
| X und Y | unabhängig voneinander für eine direkte Bindung oder eine Kaskadenreproduktionseinheit der Reproduktionsmultiplizität x bzw. y, |
| Z und W | unabhängig voneinander für eine Kaskadenreproduktionseinheit der Reproduktionsmultiplizität z bzw. w, |
| K | für den Rest eines Komplexbildners, |
| a | für die Ziffern 2 bis 12, |
| x, y, | z und w unabhängig voneinander für die Ziffern 1 bis 4 stehen, |

mit der Maßgabe, daß mindestens zwei Reproduktionseinheiten unterschiedlich sind, daß für das Produkt der Multiplizitäten gilt $16 \leq a \cdot x \cdot y \cdot z \cdot w \leq 64$ und daß mindestens eine der Kaskadenreproduktionseinheiten X,

Y, Z, W für eine 1,4,7,10-Tetraazacyclododecan- oder 1,4,8,11-Tetraazacyclotetradecan-Reproduktionseinheit steht.

b) mindestens 16 Ionen eines Elements der Ordnungszahlen 20 bis 29, 39, 42, 44 oder 57 - 83,

c) gegebenenfalls Kationen anorganischer und/oder organischer Basen, Aminosäuren oder Aminosäureamide sowie

d) gegebenenfalls acylierte terminale Aminogruppen.

**2.** Kaskaden-Polymer-Komplexe gemäß Anspruch 1, **dadurch gekennzeichnet, daß** A ein Stickstoffatom,

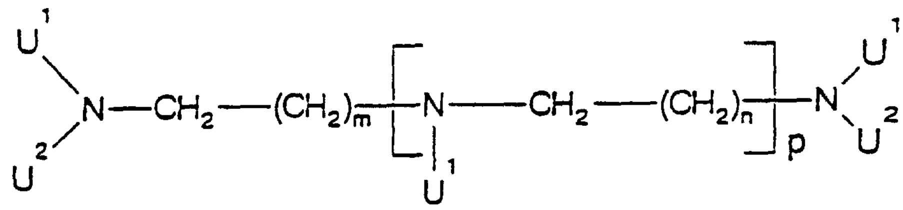

,

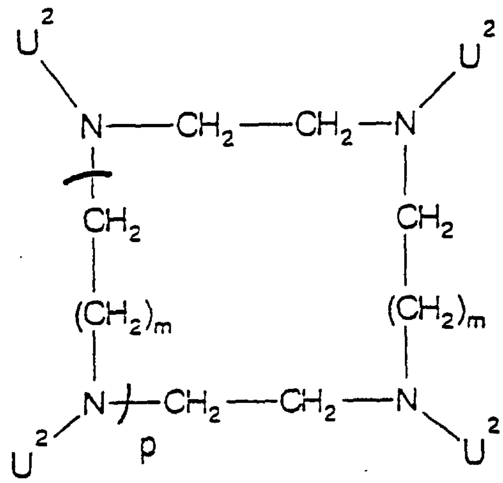

,

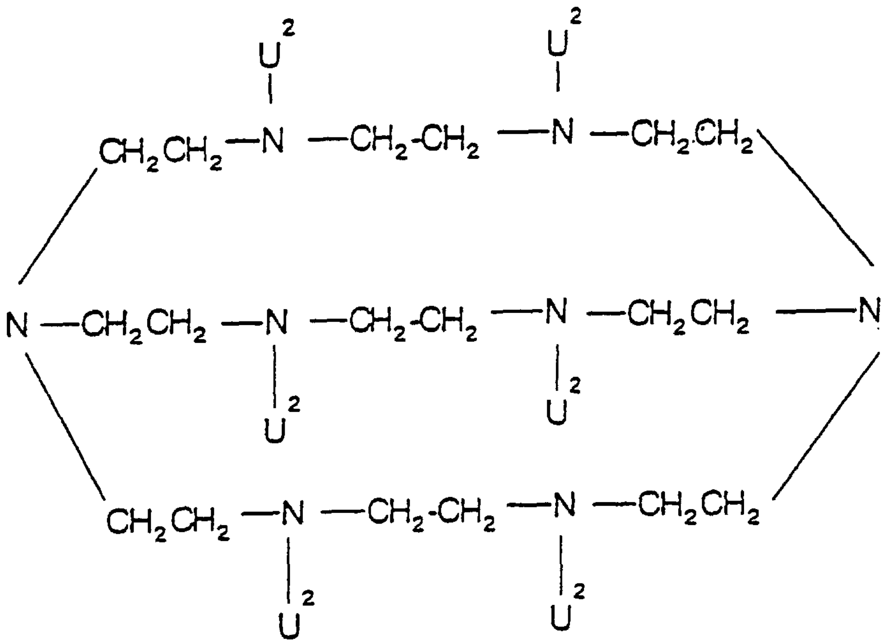

,

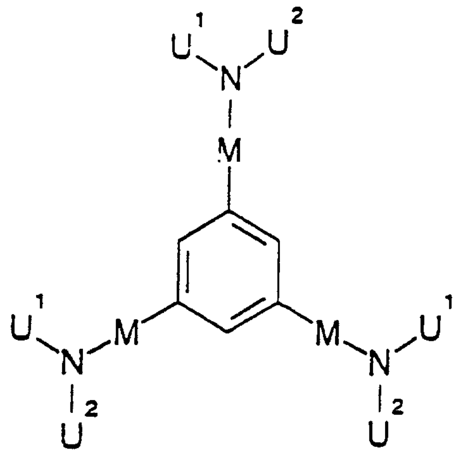

,

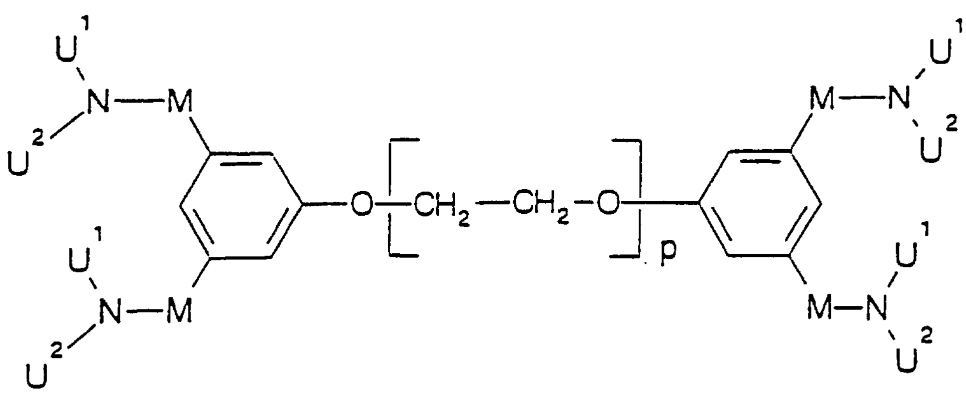

,

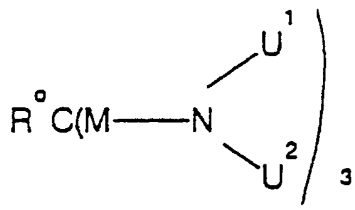

,

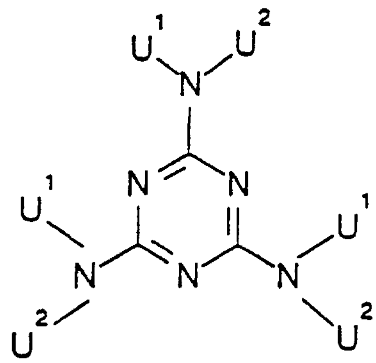

,

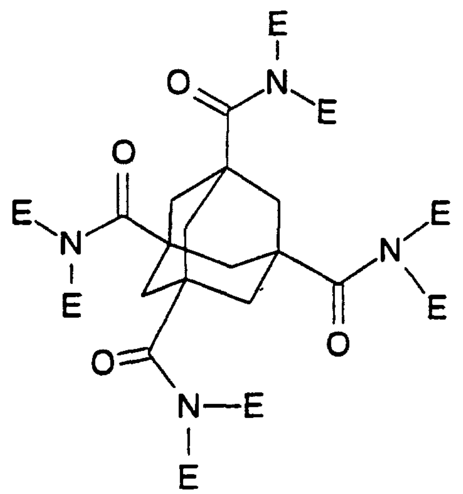

bedeutet, worin

- m und n für die Ziffern 1 bis 10,
- p für die Ziffern 0 bis 10,
- $U^1$ für $Q^1$ oder E,
- $U^2$ für $Q^2$ oder E mit

E in der Bedeutung der Gruppe

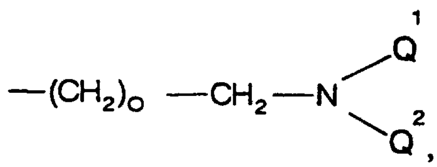

wobei

- o für die Ziffern 1 bis 6,
- $Q^1$ für ein Wasserstoffatom oder $Q^2$ und
- $Q^2$ für eine direkte Bindung

M für eine $C_1$-$C_{10}$-Alkylenkette, die gegebenenfalls durch 1 bis 3 Sauerstoffatome unterbrochen ist und/oder gegebenenfalls mit 1 bis 2 Oxogruppen substituiert ist,

$R^o$ für einen verzweigten oder unverzweigten $C_1$-$C_{10}$-Alkylrest, eine Nitro-, Amino-, Carbonsäuregruppe oder für

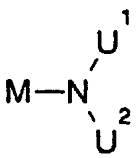

stehen,
wobei die Anzahl $Q^2$ der Basismultiplizität a entspricht.

**3.** Kaskaden-Polymer-Komplexe gemäß Anspruch 1, **dadurch gekennzeichnet, daß** die Kaskadenreproduktionseinheiten X, Y, Z und W unabhängig voneinander für

E ,

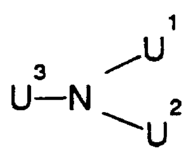

,

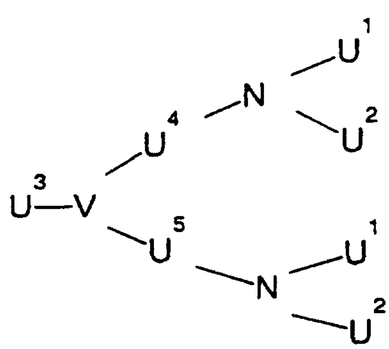

,

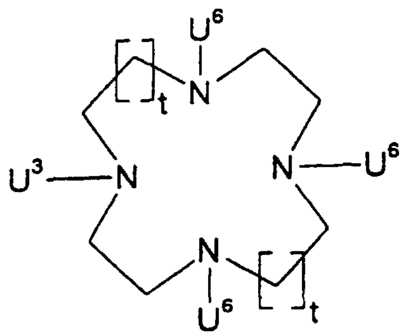

mit t in der Bedeutung der Ziffern 1 oder 2,

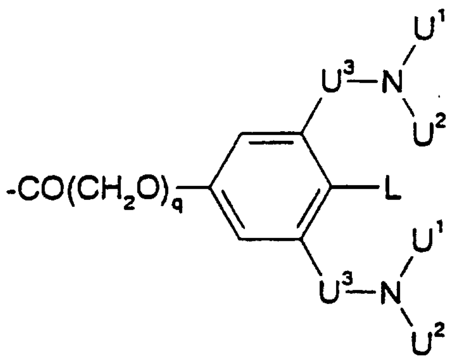

stehen,
worin

$U^1$ für $Q^1$ oder E,
$U^2$ für $Q^2$ oder E mit
E in der Bedeutung der Gruppe

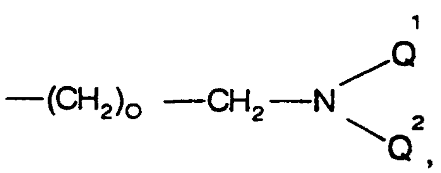

wobei

o für die Ziffern 1 bis 6,
$Q^1$ für ein Wasserstoffatom oder $Q^2$,
$Q^2$ für eine direkte Bindung,

$U^3$ für eine -NHCO-$(CH_2)_o$-Kette oder eine $C_1$-$C_{20}$-Alkylenkette, die gegebenenfalls durch 1 bis 10 Sauerstoffatome und/oder 1 bis 2 -$N(CO)_q$-$R^2$-, 1 bis 2 Phenylen- und/oder 1 bis 2 Phenylenoxyreste unterbrochen ist und/oder gegebenenfalls durch 1 bis 2 Oxo-, Thioxo-, Carboxy-, $C_1$-$C_5$-Alkylcarboxy-, $C_1$-$C_5$-Alkoxy-, Hydroxy-, $C_1$-$C_5$-alkylgruppen substituiert ist, wobei

q für die Ziffern 0 oder 1 und
$R^2$ für ein Wasserstoffatom, einen Methyl- oder einen Ethylrest, der gegebenenfalls mit 1 - 2 Hydroxy- oder 1 Carboxygruppe(n) substituiert ist,

L für ein Wasserstoffatom oder die Gruppe

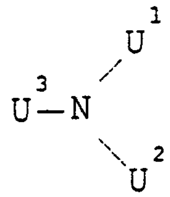

V für die Methingruppe

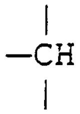

wenn gleichzeitig $U^4$ eine direkte Bindung oder die in Anspruch 2 definierte Gruppe M bedeutet und $U^5$ eine der Bedeutungen von $U^3$ besitzt oder

V für die Gruppe

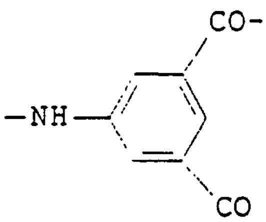

,

wenn gleichzeitig $U^4$ und $U^5$ identisch sind und die direkte Bindung oder die Gruppe M bedeuten, und

$U^6$ für die Gruppe

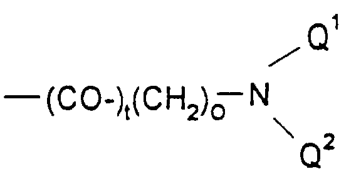

oder eine direkte Bindung

stehen

mit der Maßgabe, daß mindestens eine der Kaskadenreproduktionseinheiten für die oben angegebene 1,4,7,10-Tetraazacyclododecan- oder 1,4,8,11-Tetraazacyclotetradecan-Reproduktionseinheit steht.

**4.** Kaskaden-Polymer-Komplexe gemäß Anspruch 1, **dadurch gekennzeichnet, daß** der an die terminalen Stickstoffatome der letzten Generation der Reproduktionseinheit W gebundene Komplexbildner-Rest K für einen Rest der allgemeinen Formeln IA, IB oder IC

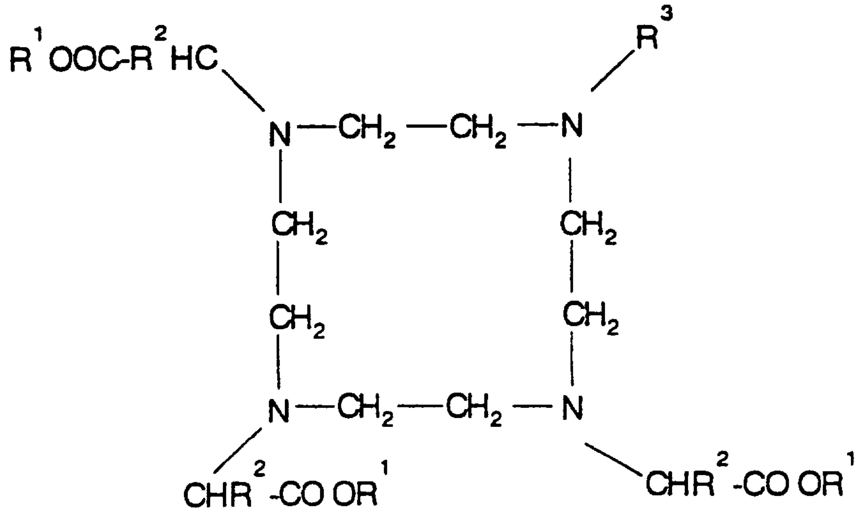

(IA),

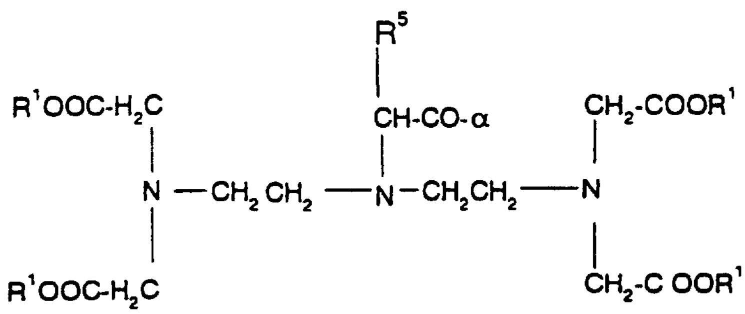

(IB),

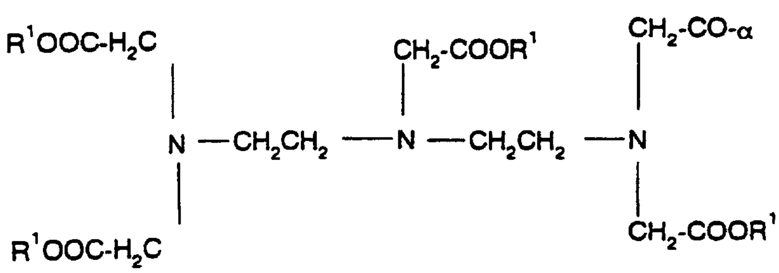

(IC),

steht, worin

$R^1$ unabhängig voneinander für ein Wasserstoffatom oder ein Metallionenäquivalent der Ordnungszahlen 20 - 29, 39, 42 - 44 oder 57 - 83,

$R^2$ für ein Wasserstoffatom, einen Methyl- oder einen Ethylrest, der gegebenenfalls mit 1 - 2 Hydroxy- oder 1 Carboxygruppe(n) substituiert ist,

$R^3$ für eine

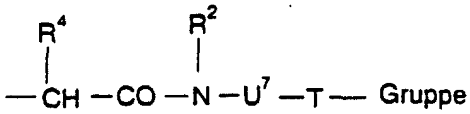

oder eine

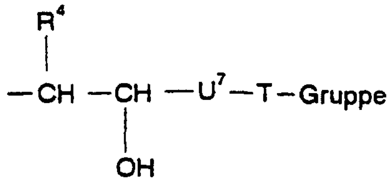

,

$R^4$ für ein Wasserstoffatom oder eine geradkettige, verzweigte, gesättigte oder ungesättigte $C_1$-$C_{30}$-Alkylkette, die gegebenenfalls durch 1 - 10 Sauerstoff atome, 1 Phenylen-, 1 Phenylenoxygruppen unterbrochen und/ oder gegebenenfalls durch 1 - 5 Hydroxy-, 1 - 3 Carboxy-, 1 Phenylgruppe(n) substituiert ist,

$R^5$ für ein Wasserstoffatom oder für $R^4$,

$U^7$ für eine gegebenenfalls 1 - 5 Imino-, 1 - 3 Phenylen-, 1 - 3 Phenylenoxy-, 1 - 3 Phenylenimino-, 1 - 5 Amid-, 1 - 2 Hydrazid-, 1 - 5 Carbonyl-, 1 - 5 Ethylenoxy-, 1 Harnstoff-, 1-Thioharnstoff-, 1 - 2 Carboxyalkylimino-, 1 - 2 Estergruppen, 1 - 10 Sauerstoff-, 1 - 5 Schwefel- und/oder 1 - 5 Stickstoff-Atom(e) enthaltende und/ oder gegebenenfalls durch 1 - 5 Hydroxy-, 1 - 2 Mercapto-, 1 - 5 Oxo-, 1 - 5 Thioxo-, 1 - 3 Carboxy-, 1 - 5 Carboxyalkyl-, 1 - 5 Ester- und/oder 1 - 3 Aminogruppe(n) substituierte geradkettige, verzweigte, gesättigte oder ungesättigte $C_1$-$C_{20}$-Alkylengruppe, wobei die gegebenenfalls enthaltenden Phenylengruppen durch 1 - 2 Carboxy-, 1 - 2 Sulfon- oder 1 - 2 Hydroxygruppen substituiert sein können,

T für eine -CO-α, -NHCO-α- oder -NHCS-α-Gruppe und

α für die Bindungsstelle an die terminalen Stickstoffatome der letzten Generation, der Reproduktionseinheit W

stehen.

**5.** Kaskaden-Polymer-Komplexe gemäß Anspruch 4, **dadurch gekennzeichnet, daß** die für $U^7$ stehende $C_1$-$C_{20}$-Alkylenkette die Gruppen $-CH_2-$, $-CH_2NHCO-$, $-NHCOCH_2O-$, $-NHCOCH_2OC_6H_4-$, $-N(CH_2CO_2H)-$, $-NHCOCH_2C_6H_4-$, $-NHCSNHC_6H_4-$, $-CH_2OC_6H_4-$, $-CH_2CH_2O-$, enthält und/oder durch die Gruppen -COOH, $-CH_2COOH$
substituiert ist.

**6.** Kaskaden-Polymer-Komplexe gemäß Anspruch 4, **dadurch gekennzeichnet, daß** $U^7$ für eine
$-CH_2-$, $-CH_2CH_2-$, $-CH_2CH_2CH_2-$, $-C_6H_4-$, $-C_6H_{10}-$, $-CH_2C_6H_5-$, $-CH_2NHCOCH_2CH(CH_2CO_2H)-C_6H_4-$, $-CH_2NHCOCH_2OCH_2-$, $-CH_2NHCOCH_2C_6H_4-$,
gruppe steht.

**7.** Kaskaden-Polymer-Komplexe gemäß Anspruch 3, **dadurch gekennzeichnet, daß** der in den Kaskadenreproduktionseinheiten X, Y, Z und W enthaltene Rest $U^3$ für -CO-, $-COCH_2OCH_2CO-$, $-COCH_2-$, $-CH_2CH_2-$, $-CONHC_6H_4-$, $-NHCOCH_2-$, $-COCH_2CH_2CO-$, $-COCH_2-CH_2CH_2CO-$, $-COCH_2CH_2CH_2CH_2CO-$, $-CONHCH_2CH_2NHCOCH_2-CH_2CO-$, $-COCH_2CH_2NHCOCH_2CH_2CO-$,
der Rest $U^4$ für eine direkte Bindung, für $-CH_2CO-$,
der Rest $U^5$ für eine direkte Bindung, für $-(CH_2)_4-$, $-CH_2CO-$, -CH(COOH)-, $CH_2OCH_2CH_2-$, $-CH_2C_6H_4-$, $CH_2-C_6H_4OCH_2CH_2-$,

der Rest E für eine Gruppe

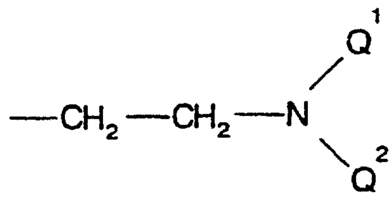

steht.

**8.** Kaskaden-Polymer-Komplexe gemäß Anspruch 3, **dadurch gekennzeichnet, daß** die Kaskadenreproduktionseinheiten X, Y, Z und W unabhängig voneinander für
$-CH_2CH_2NH-$; $-CH_2CH_2N<$;
$-COCH(NH-)(CH_2)_4NH-$; $-COCH(N<)(CH_2)_4N<$ ;
$-COCH_2OCH_2CON(CH_2CH_2NH-)_2$; $-COCH_2OCH_2CON(CH_2CH_2N<)_2$ ;
$-COCH_2N(CH_2CH_2NH-)_2$; $-COCH_2N(CH_2CH_2N<)_2$;
$-COCH_2NH-$; $-COCH_2N<$ ;
$-COCH_2CH_2CON(CH_2CH_2NH-)_2$; $-COCH_2CH_2CON(CH_2CH_2N<)_2$;
$-COCH_2OCH_2CONH-C_6H_4-CH[CH_2CON(CH_2CH_2NH-)_2]_2$;
$-COCH_2OCH_2CONH-C_6H_4-CH[CH_2CON(CH_2CH_2N<)_2]_2$;
$-COCH_2CH_2CO-NH-C_6H_4-CH[CH_2CON(CH_2CH_2NH-)_2]_2$;
$-COCH_2CH_2CO-NH-C_6H_4-CH[CH_2CON(CH_2CH_2N<)_2]_2$;
$-CONH-C_6H_4-CH[CH_2CON(CH_2CH_2NM-)_2]_2$;
$-CONH-C_6H_4-CH[CH_2CON(CH_2CH_2N<)_2]_2$;
$-COCH(NH-)CH(COOH)NH-$; $-COCH(N<)CH(COOH)N<$ ;

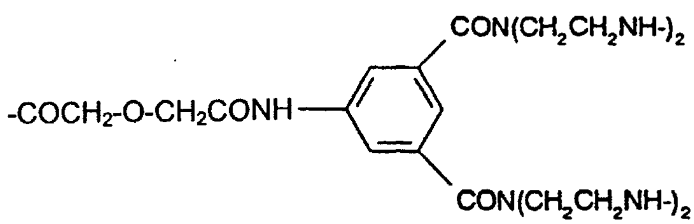

;

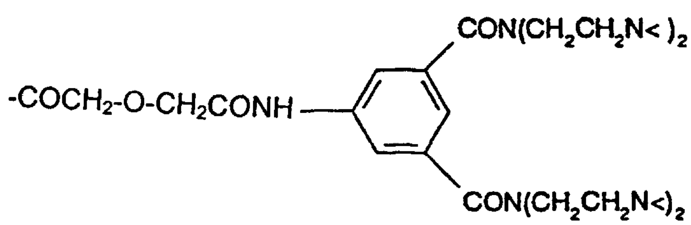

;

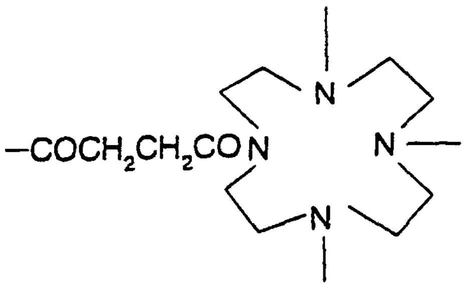 ; 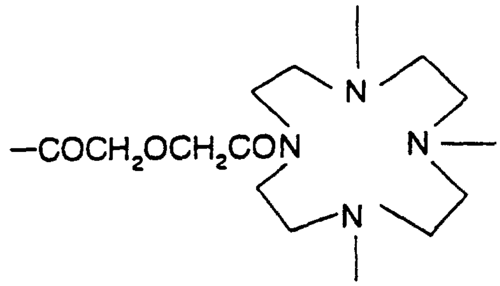 ;

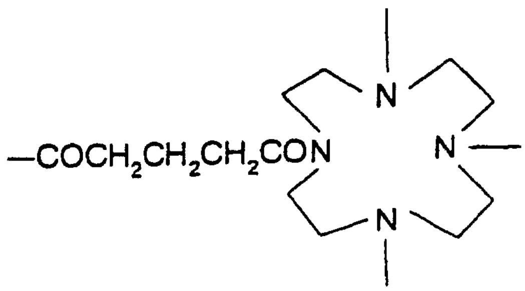 ,

-COCH($CH_2CH_2CH_2$-N<)$_2$; -COCH($CH_2CH_2$-N<)$_2$;

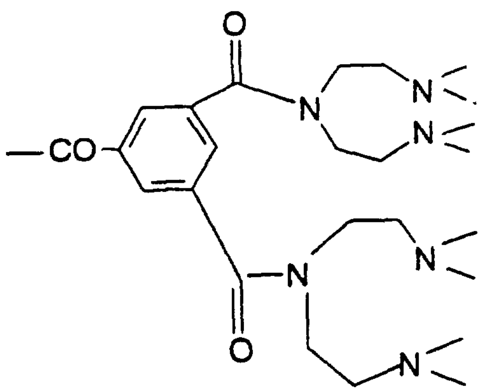 ; 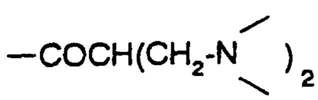 ;

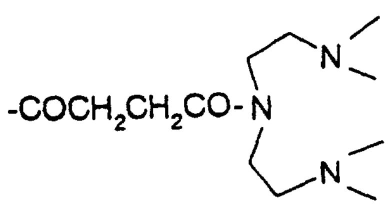 ;

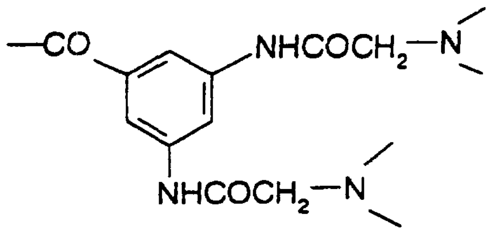

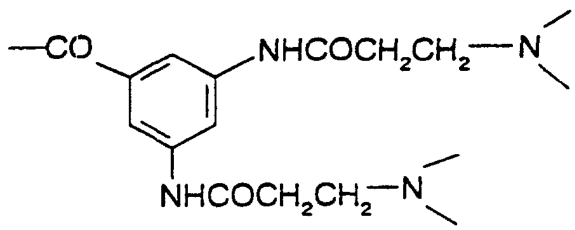

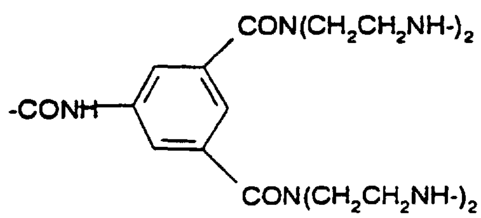

;

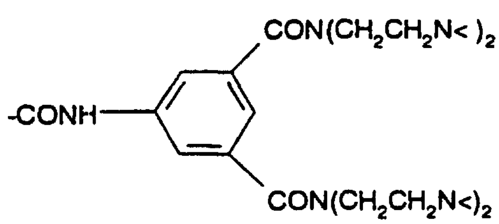

;

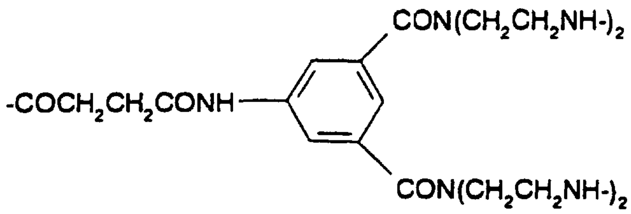

;

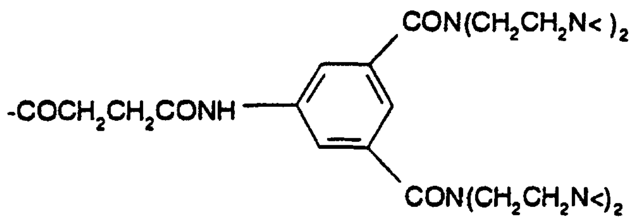

;

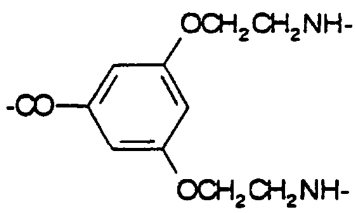

;

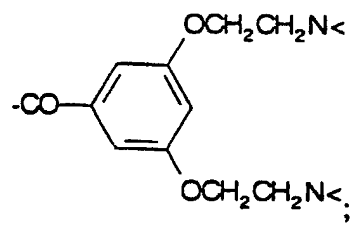

;

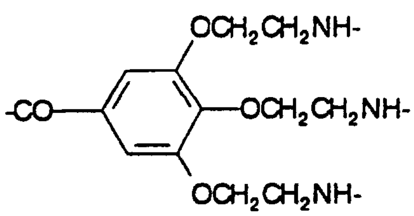

;

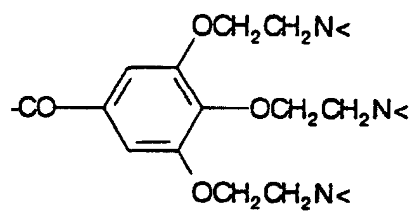

;

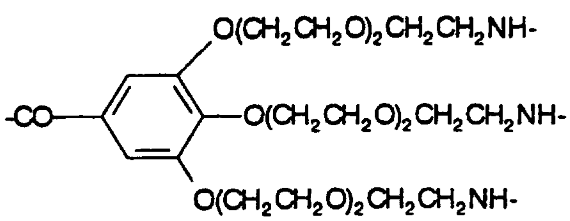

;

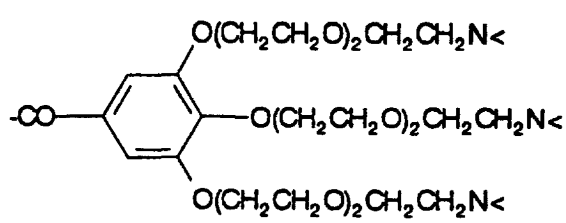

;

stehen.

**9.** Kaskaden-Polymer-Komplexe gemäß Anspruch 2, **dadurch gekennzeichnet, daß**

m für die Ziffern 1 - 3,
n für die Ziffern 1 - 3,
o für die Ziffer 1,
p für die Ziffern 0 - 3,
M für eine $-CH_2-$, -CO- oder $-CH_2CO$-Gruppe und
$R^o$ für eine $-CH_2NU^1U^2$, $CH_3$- oder $NO_2$-Gruppe

stehen.

**10.** Pharmazeutische Mittel enthaltend mindestens einen Kaskaden-Polymer-Komplex nach Anspruch 1, gegebenenfalls mit den in der Galenik üblichen Zusätzen.

**11.** Verwendung von mindestens einem Polymer-Komplex nach Anspruch 1 für die Herstellung von Mitteln für die NMR- oder Röntgendiagnostik.

**12.** Verwendung der Kaskaden-Polymer-Komplexe nach Anspruch 1 fiir die Herstellung von Mitteln zur Differenzierung von benignen und malignen Tumoren in Körperregionen ohne Blut-Hirn-Schranke.

**13.** Verfahren zur Herstellung von Kaskaden-Polymer-Komplexen gemäß den Ansprüchen 1 bis 9, **dadurch gekennzeichnet, daß** man Verbindungen der allgemeinen Formel I'

$$A\text{-}\{X\text{-}[Y\text{-}(Z\text{-}\langle W\text{-}\beta_w\rangle_z)_y]_x\}_a \qquad (I'),$$

worin

A für einen stickstoffhaltigen Kaskadenkern der Basismultiplizität a,
X und Y unabhängig voneinander für eine direkte Bindung oder eine Kaskadenreproduktionseinheit der Reproduktionsmultiplizität x bzw. y,
Z und W unabhängig voneinander für eine Kaskadenreproduktionseinheit der Reproduktionsmultiplizität z bzw. w,
a für die Ziffern 2 bis 12,
x, y, z und w unabhängig voneinander für die Ziffern 1 bis 4 und
β für die Bindungsstelle der terminalen NH-Gruppen der letzten Generation, der Reproduktionseinheit W stehen

mit der Maßgabe, daß mindestens zwei Reproduktionseinheiten unterschiedlich sind, daß für das Produkt der Multiplizitäten gilt $16 \leq a \cdot x \cdot y \cdot z \cdot w \leq 64$ und daß mindestens eine der Kaskadenreproduktionseinheiten X, Y, Z, W für eine 1,4,7,10-Tetraazacyclododecan- oder 1,4,8,11-Tetraazacyclotetradecan-Reproduktionseinheit steht.
mit einem Komplex oder Komplexbildner K' der allgemeinen Formel I'A, I'B oder I'C

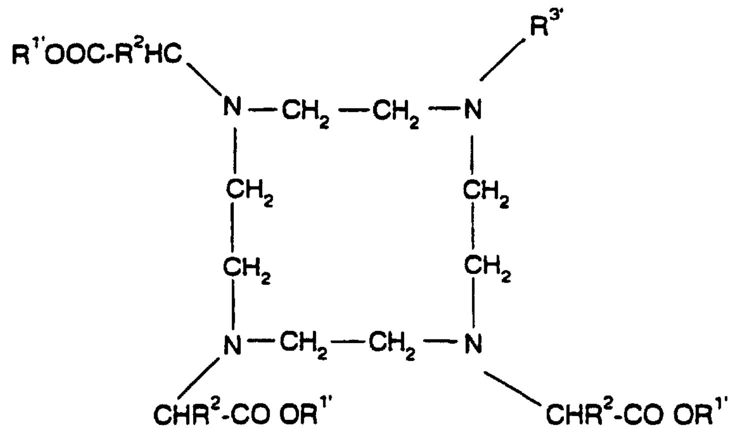

(I'A)

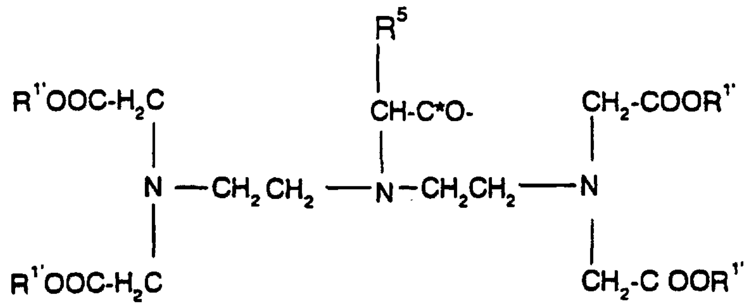

(I'B),

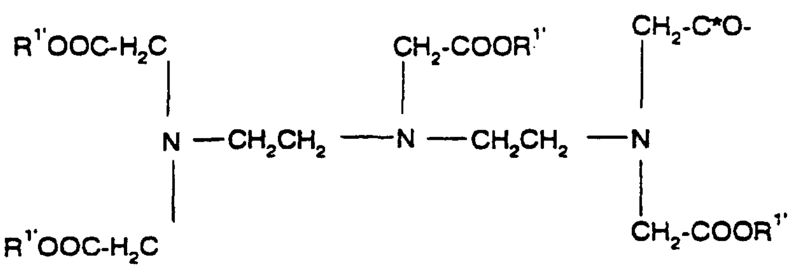

(I'C)

wobei

R1' unabhängig voneinander für ein Wasserstoffatom, ein Metallionenäquivalent der Ordnungszahlen 20 - 29, 39, 42 - 44 oder 57 - 83 oder eine Säureschutzgruppe,

$R^2$ für ein Wasserstoffatom, einen Methyl- oder einen Ethylrest, der gegebenenfalls mit 1 - 2 Hydroxy- oder 1 Carboxygruppe(n) substituiert ist,

$R^{3'}$ für eine

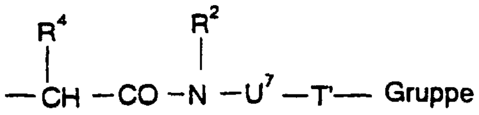

oder eine

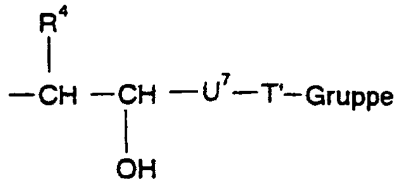

,

$R^4$ für ein Wasserstoffatom oder eine geradkettige, verzweigte, gesättigte oder ungesättigte $C_1$-$C_{30}$-Alkylkette, die gegebenenfalls durch 1 - 10 Sauerstoff atome, 1 Phenylen-, 1 Phenylenoxygruppen unterbrochen und/oder gegebenenfalls durch 1 - 5 Hydroxy-, 1 - 3 Carboxy-, 1 Phenylgruppe(n) substituiert ist,

$R^5$ für ein Wasserstoffatom oder fiir $R^4$,

$U^7$ für eine gegebenenfalls 1 - 5 Imino-, 1 - 3 Phenylen-, 1 - 3 Phenylenoxy-, 1 - 3 Phenylenimino-, 1 - 5 Amid-, 1 - 2 Hydrazid-, 1 - 5 Carbonyl-, 1 - 5 Ethylenoxy-, 1 Harnstoff-, 1-Thioharnstoff-, 1 - 2 Carboxyalkylimino-, 1 - 2 Estergruppen, 1 - 10 Sauerstoff-, 1 - 5 Schwefel- und/oder 1 - 5 Stickstoff-Atom(e) enthaltende und/oder gegebenenfalls durch 1 - 5 Hydroxy-, 1 - 2 Mercapto-, 1 - 5 Oxo-, 1 - 5 Thioxo-, 1 - 3 Carboxy-, 1 - 5 Carboxyalkyl-, 1 - 5 Ester- und/oder 1 - 3 Aminogruppe(n) substituierte geradkettige, verzweigte, gesättigte oder ungesättigte $C_1$-$C_{20}$-Alkylengruppe, wobei die gegebenenfalls enthaltenden Phenylengruppen durch 1 - 2 Carboxy-, 1 - 2 Sulfon- oder 1 - 2 Hydroxygruppen substituiert sein können,

T' für eine -C*O-, -COOH-, -N=C=O- oder -N=C=S-Gruppe und

C*O für eine aktivierte Carboxylgruppe

stehen

mit der Maßgabe, daß - sofern K' für einen Komplex steht - mindestens zwei (bei zweiwertigen Metallen) bzw. drei (bei dreiwertigen Metallen) der Substituenten $R^1$ für ein Metallionenäquivalent der oben genannten Elemente stehen und daß gewünschtenfalls weitere Carboxylgruppen in Form ihrer Salze mit anorganischen und/oder organischen Basen, Aminosäuren oder Aminosäureamiden vorliegen,

umsetzt, gegebenenfalls vorhandene Schutzgruppen abspaltet, die so erhaltenen Kaskaden-Polymere - sofern K' für einen Komplexbildner steht - in an sich bekannter Weise mit mindestens einem Metalloxid oder Metallsalz eines Elements der Ordnungszahlen 20 - 29, 39, 42, 44 oder 57 - 83 umsetzt und gegebenenfalls anschließend in den so erhaltenen Kaskaden-Polymer-Komplexen noch vorhandene acide Wasserstoffatome ganz oder teilweise durch Kationen von anorganischen und/oder organischen Basen, Aminosäuren oder Aminosäureamiden substituiert und gegebenenfalls noch vorhandene freie terminale Aminogruppen gewünschtenfalls - vor oder nach der Metallkomplexierung - acyliert.

**14.** Verbindungen der allgemeinen Formel I'A

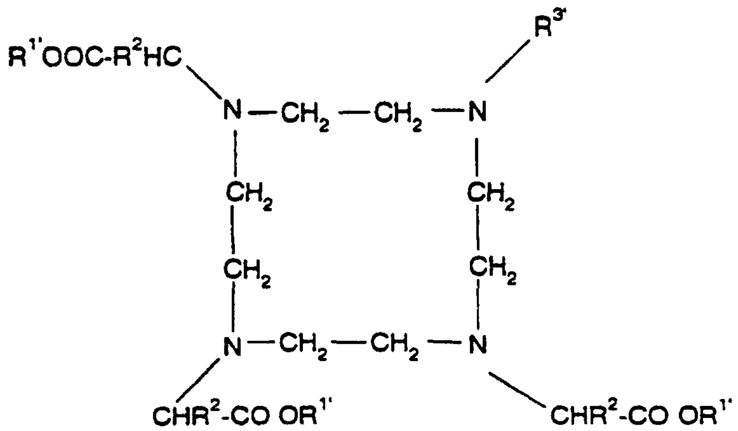

(I'A)

wobei

R1' unabhängig voneinander für ein Wasserstoffatom, ein Metallionenäquivalent der Ordnungszahlen 20 - 29, 39, 42 - 44 oder 57 - 83 oder eine Säureschutzgruppe,

$R^2$ für ein Wasserstoffatom, einen Methyl- oder einen Ethylrest, der gegebenenfalls mit 1 - 2 Hydroxy- oder 1 Carboxygruppe(n) substituiert ist,

$R^{3'}$ für eine

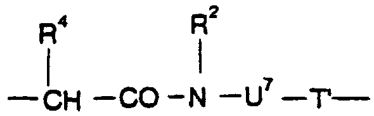 Gruppe

oder eine

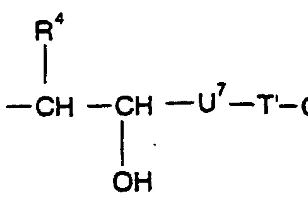 Gruppe

,

$R^4$ für ein Wasserstoffatom oder eine geradkettige, verzweigte, gesättigte oder ungesättigte $C_1$-$C_{30}$-Alkylkette, die gegebenenfalls durch 1 - 10 Sauerstoff atome, 1 Phenylen-, 1 Phenylenoxygruppen unterbrochen und/oder gegebenenfalls durch 1 - 5 Hydroxy-, 1 - 3 Carboxy-, 1 Phenylgruppe(n) substituiert ist,

$U^7$ für eine gegebenenfalls 1 - 5 Imino-, 1 - 3 Phenylen-, 1 - 3 Phenylenoxy-, 1 - 3 Phenylenimino-, 1 - 5 Amid-, 1 - 2 Hydrazid-, 1 - 5 Carbonyl-, 1 - 5 Ethylenoxy-, 1 Harnstoff-, 1-Thioharnstoff-, 1 - 2 Carboxyalkylimino-, 1 - 2 Estergruppen, 1 - 10 Sauerstoff-, 1 - 5 Schwefel- und/oder 1 - 5 Stickstoff-Atom(e) enthaltende und/ oder gegebenenfalls durch 1 - 5 Hydroxy-, 1 - 2 Mercapto-, 1 - 5 Oxo-, 1 - 5 Thioxo-, 1 - 3 Carboxy-, 1 - 5 Carboxyalkyl-, 1 - 5 Ester- und/oder 1 - 3 Aminogruppe(n) substituierte geradkettige, verzweigte, gesättigte oder ungesättigte $C_1$-$C_{20}$-Alkylengruppe, wobei die gegebenenfalls enthaltenden Phenylengruppen durch 1 - 2 Carboxy-, 1 - 2 Sulfon- oder 1 - 2 Hydroxygruppen substituiert sein können,

T' für eine -C*O-, -COOH-, -N=C=O- oder -N=C=S-Gruppe und

C*O für eine aktivierte Carboxylgruppe

stehen.

**15.** Verfahren zur Herstellung der pharmazeutischen Mittel gemäß Anspruch 10, **dadurch gekennzeichnet, daß** man den in Wasser oder physiologischer Salzlösung gelösten oder suspendierten Kaskaden-Polymer-Komplex, gegebenenfalls mit den in der Galenik üblichen Zusätzen, in eine für die enterale oder parenterale Applikation geeignete Form bringt.

## Claims

**1.** Cascade polymer complexes comprising

a) complex-forming ligands of the general formula I

$$A\text{-}\{X\text{-}[Y\text{-}(Z\text{-}<W\text{-}K_w>_z)_y]_x\}_a \quad (I),$$

in which

| | |
|---|---|
| A | is a nitrogen-containing cascade nucleus of base multiplicity a, |
| X and Y | are, independently of one another, a direct linkage or a cascade reproduction unit of reproduction multiplicity x and y respectively, |
| Z and W | are, independently of one another, a cascade reproduction unit of reproduction multiplicity z and w respectively, |
| K | is the radical of a complexing agent, |
| a | is the numbers 2 to 12, |
| x, y, z and w | are, independently of one another, the numbers 1 to 4, |

with the proviso that at least two reproduction units are different, that $16 \leq a \cdot x \cdot y \cdot z \cdot w \leq 64$ applies to the product of the multiplicities, and that at least one of the cascade reproduction units X, Y, Z, W is a 1,4,7,10-tetraazacyclododecane or 1,4,8,11-tetraazacyclotetradecane reproduction unit,
b) at least 16 ions of an element of atomic numbers 20 to 29, 39, 42, 44 or 57-83,
c) where appropriate cations of inorganic and/or organic bases, amino acids or amino amides, and
d) where appropriate acylated terminal amino groups.

**2.** Cascade polymer complexes according to Claim 1, **characterized in that** A is nitrogen atom,

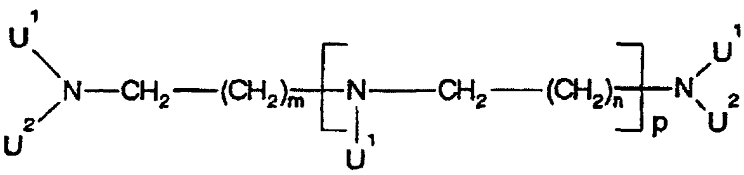

,

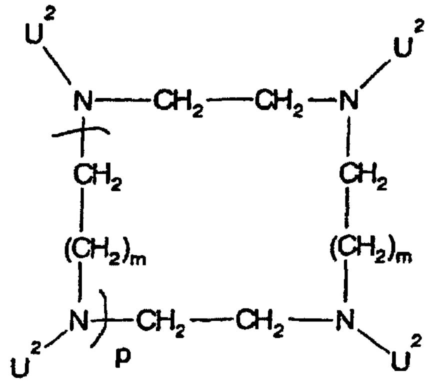

,

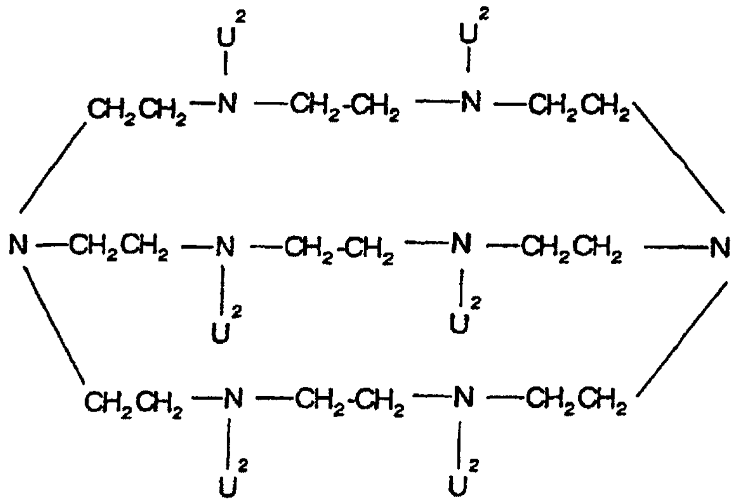

,

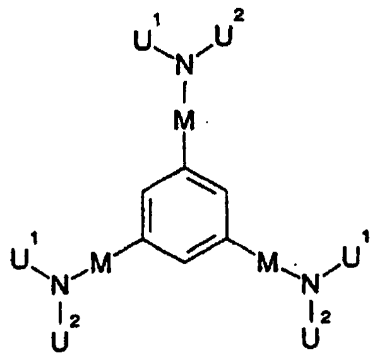

,

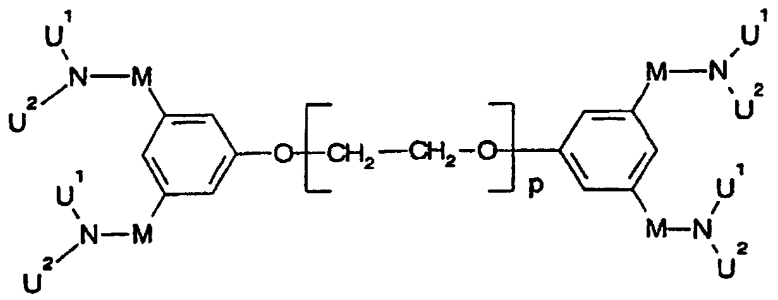

,

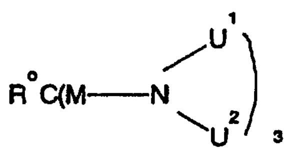

,

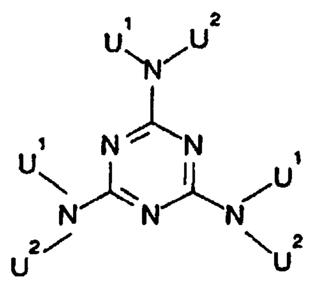

,

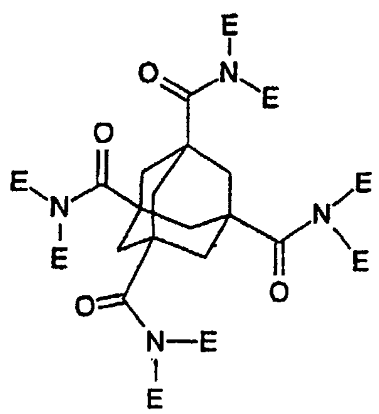

in which

- m and n are the numbers 1 to 10,
- p is the numbers 0 to 10,
- $U^1$ is $Q^1$ or E,
- $U^2$ is $Q^2$ or E with

E meaning the group

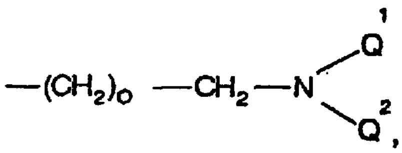

where

- o is the numbers 1 to 6,
- $Q^1$ is a hydrogen atom or $Q^2$ and
- $Q^2$ is a direct linkage

M is a $C_1$-$C_{10}$-alkylene chain which is optionally interrupted by 1 to 3 oxygen atoms and/or is optionally substituted by 1 to 2 oxo groups,

$R^o$ is a branched or unbranched $C_1$-$C_{10}$-alkyl radical, a nitro, amino, carboxylic acid group or

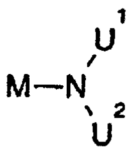

where the number of $Q^2$ corresponds to the base multiplicity a.

3. Cascade polymer complexes according to Claim 1, **characterized in that** the cascade reproduction units X, Y, Z and W are, independently of one another,
E,

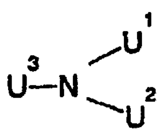

,

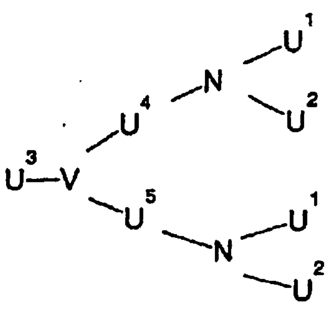

,

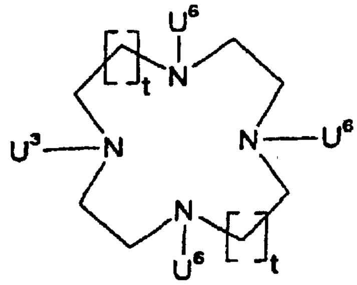

with t meaning the numbers 1 or 2,

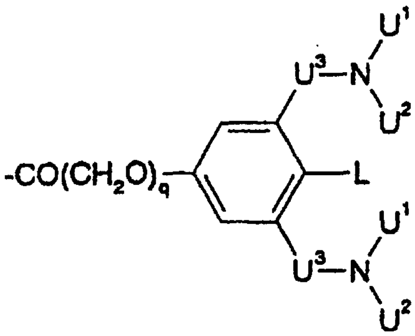

in which

$U^1$ is $Q^1$ or E,
$U^2$ is $Q^2$ or E with
E meaning the group

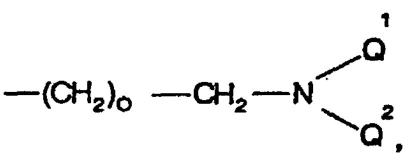

where
o is the numbers 1 to 6,
$Q^1$ is a hydrogen atom or $Q^2$,
$Q^2$ is a direct linkage,
$U^3$ is an $-NHCO-(CH_2)_o-$ chain or a $C_1$-$C_{20}$-alkylene chain which is optionally interrupted by 1 to 10 oxygen atoms and/or 1 to 2 $-N(CO)_q-R^2-$, 1 to 2 phenylene and/or 1 to 2 phenyleneoxy radicals and/or is optionally substituted by 1 to 2 oxo, thioxo, carboxy, $C_1$-$C_5$-alkylcarboxy, $C_1$-$C_5$-alkoxy, hydroxyl, $C_1$-$C_5$-alkyl groups, where

q is the numbers 0 or 1 and
$R^2$ is a hydrogen atom, a methyl or ethyl radical which is optionally substituted by 1-2 hydroxyl or 1 carboxy group(s),
L is a hydrogen atom or the group

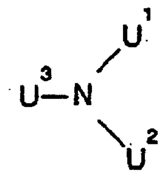

V is the methine group

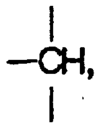

if, at the same time, $U^4$ is a direct linkage or the group M defined in Claim 2 and $U^5$ has one of the meanings of $U^3$, or

V is the group

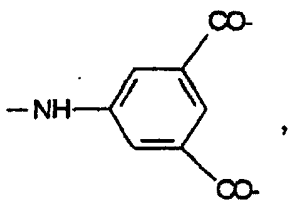

if, at the same time, $U^4$ and $U^5$ are identical and are the direct linkage or the group M, and

$U^6$ is the group

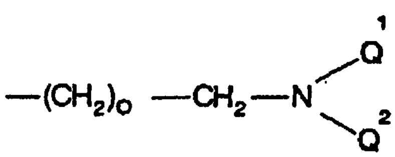

or a direct linkage,

with the proviso that at least one of the cascade reproduction units is the abovementioned 1,4,7,10-tetraazacyclododecane or 1,4,8,11-tetraazacyclotetradecane reproduction unit.

4. Cascade polymer complexes according to Claim 1, **characterized in that** the complexing agent radical K which is bonded to the terminal nitrogen atoms of the last generation of the reproduction unit W is a radical of the general formulae IA, IB or IC

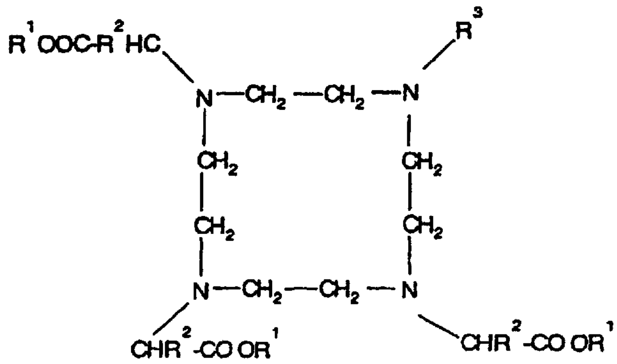

(IA),

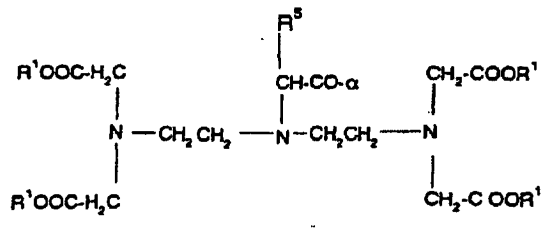

(IB),

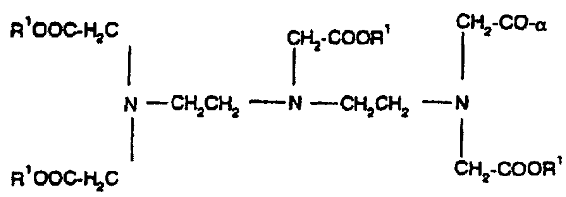

(IC),

in which

$R^1$ is, independently of one another, a hydrogen atom or a metal ion equivalent of atomic numbers 20-29, 39, 42-44 or 57-83,

$R^2$ is a hydrogen atom, a methyl or ethyl radical which is optionally substituted by 1-2 hydroxyl or 1 carboxy group(s),

$R^3$ is a

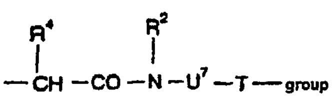

or a

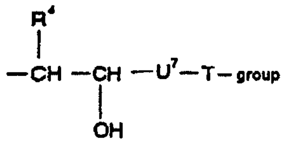

,

$R^4$ is a hydrogen atom or a straight-chain, branched, saturated or unsaturated $C_1$-$C_{30}$-alkyl chain which is optionally interrupted by 1-10 oxygen atoms, 1 phenylene, 1 phenyleneoxy groups and/or is optionally substituted by 1-5 hydroxyl, 1-3 carboxy, 1 phenylene group(s),

$R^5$ is a hydrogen atom or $R^4$,

$U^7$ is a straight-chain, branched, saturated or unsaturated $C_1$-$C_{20}$-alkylene group which optionally contains 1-5 imino, 1-3 phenylene, 1-3 phenyleneoxy, 1-3 phenyleneimino, 1-5 amide, 1-2 hydrazide, 1-5 carbonyl, 1-5 ethyleneoxy, 1 urea, 1 thiourea, 1-2 carboxyalkylimino, 1-2 ester groups, 1-10 oxygen, 1-5 sulphur and/or 1-5 nitrogen atom(s) and/or is optionally substituted by 1-5 hydroxyl, 1-2 mercapto, 1-5 oxo, 1-5 thioxo, 1-3 carboxy, 1-5 carboxyalkyl, 1-5 ester and/or 1-3 amino group(s), where the optionally present phenylene groups may be substituted by 1-2 carboxy, 1-2 sulphone or 1-2 hydroxyl groups,

T is a -CO-$\alpha$, -NHCO-$\alpha$ or -NHCS-$\alpha$ group and

$\alpha$ is the binding site to the terminal nitrogen atoms of the last generation of the reproduction unit W.

**5.** Cascade polymer complexes according to Claim 4, **characterized in that** the $C_1$-$C_{20}$ alkylene chain representing $U^7$ contains the groups $-CH_2-$, $-CH_2NHCO-$, $-NHCOCH_2O-$, $-NHCOCH_2OC_6H_4-$, $-N(CH_2CO_2H)-$, $-NHCOCH_2C_6H_4-$, $-NHCSNHC_6H_4-$, $-CH_2OC_6H_4-$, $-CH_2CH_2O-$, and/or is substituted by the groups -COOH, $-CH_2COOH$.

**6.** Cascade polymer complexes according to Claim 4, **characterized in that** $U^7$ is a $-CH_2-$, $-CH_2CH_2-$, $-CH_2CH_2CH_2-$, $-C_6H_4-$, $-C_6H_{10}-$, $-CH_2C_6H_5-$, $-CH_2NHCOCH_2CH(CH_2CO_2H)-C_6H_4-$, $-CH_2NHCOCH_2OCH_2-$, $-CH_2NHCOCH_2-C_6H_4-$, group.

**7.** Cascade polymer complexes according to Claim 3, **characterized in that** the radical $U^3$ present in the cascade reproduction units X, Y, Z and W is -CO-, $-COCH_2OCH_2CO-$, $-COCH_2-$, $-CH_2CH_2-$, $-CONHC_6H_4-$, $-NHCOCH_2-$, $-COCH_2CH_2CO-$, $-COCH_2-CH_2CH_2CO-$, $-COCH_2CH_2CH_2CH_2CO-$, $-CONHCH_2CH_2NHCOCH_2CH_2CO-$, $-COCH_2CH_2NHCOCH_2CH_2CO-$, the radical $U^4$ is a direct linkage, is $-CH_2CO-$, the radical $U^5$ is a direct linkage, is $-(CH_2)_4-$, $-CH_2CO-$, -CH(COOH)-, $CH_2OCH_2CH_2-$, $-CH_2C_6H_4-$, $CH_2-C_6H_4OCH_2CH_2-$, the radical E is a

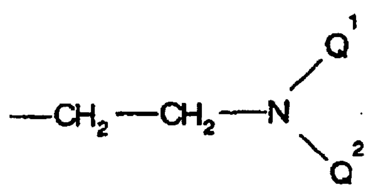

group.

**8.** Cascade polymer complexes according to Claim 3, **characterized in that** the cascade reproduction units X, Y, Z and W are, independently of one another,
$-CH_2CH_2NH-$ ; $-CH_2CH_2N<$ ;
$-COCH(NH-)(CH_2)_4NH-$; $-COCH(N<)(CH_2)_4N<$;
$-COCH_2OCH_2CON(CH_2CH_2NH-)_2$; $-COCH_2OCH_2CON(CH_2CH_2N<)_2$;
$-COCH_2N(CH_2CH_2NH-)_2$; $-COCH_2N(CH_2CH_2N<)_2$;
$-COCH_2NH-$; $-COCH_2N<$;

$-COCH_2CH_2CON(CH_2CH_2NH-)_2$; $-COCH_2CH_2CON(CH_2CH_2N<)_2$;
$-COCH_2OCH_2CONH-C_6H_4-CH[CH_2CON(CH_2CH_2NH-)_2]_2$;
$-COCH_2OCH_2CONH-C_6H_4-CH[CH_2CON(CH_2CH_2N<)_2]_2$;
$-COCH_2CH_2CO-NH-C_6H_4-CH[CH_2CON(CH_2CH_2NH-)_2]_2$;
$-COCH_2CH_2CO-NH-C_6H_4-CH[CH_2CON(CH_2CH_2N<)_2]_2$;
$-CONH-C_6H_4-CH[CH_2CON(CH_2CH_2NH-)_2]_2$;
$-CONH-C_6H_4-CH[CH_2CON(CH_2CH_2N<)_2]_2$;
$-COCH(NH-)CH(COOH)NH-$; $-COCH(N<)CH(COOH)N<$;

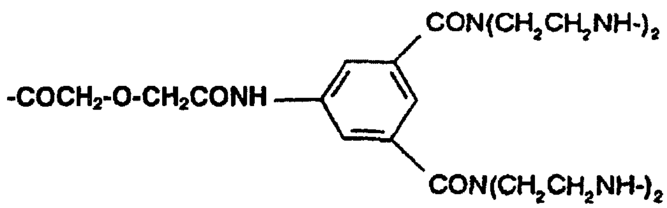 ;

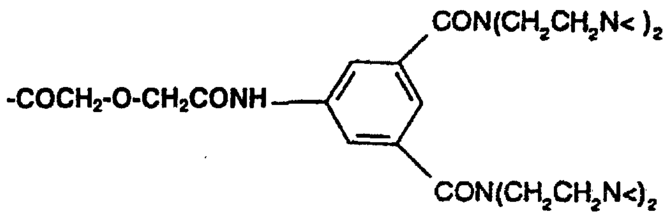 ;

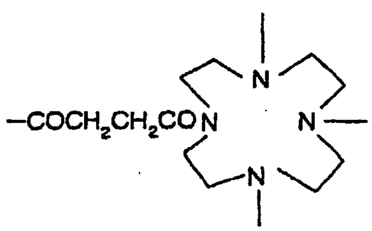 ; 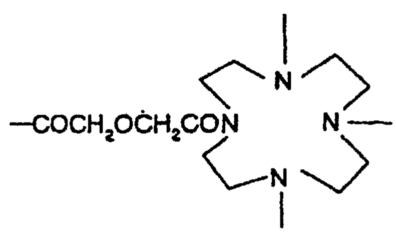 ;

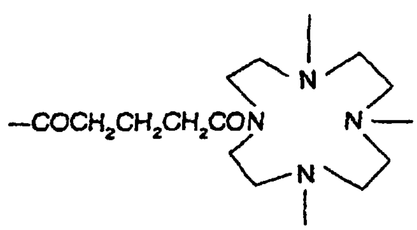 .

$-COCH(CH_2CH_2CH_2-N\langle\rangle)_2$; $-COCH(CH_2CH_2-N\langle\rangle)_2$;

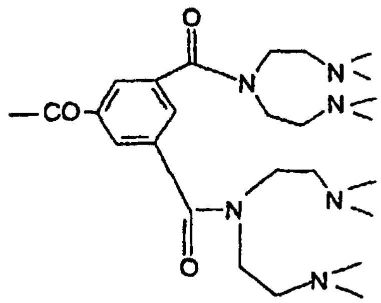

;

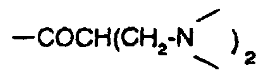

;

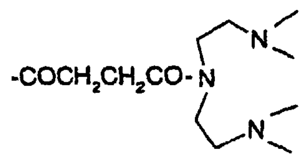

;

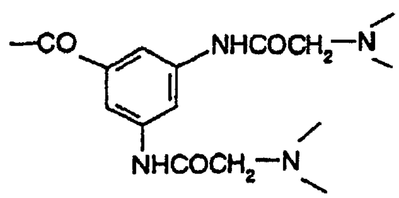

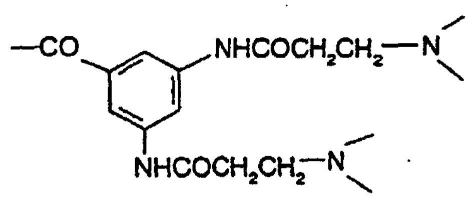

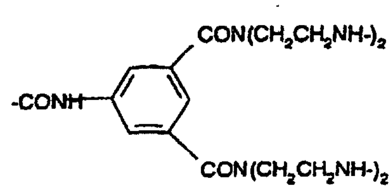

;

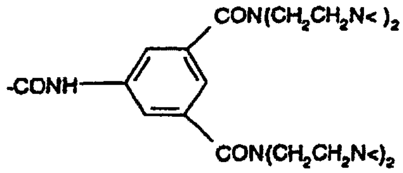

;

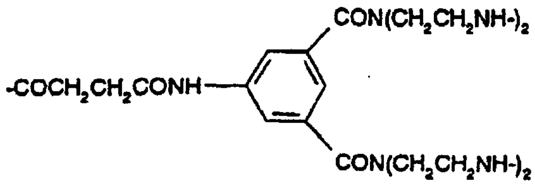

;

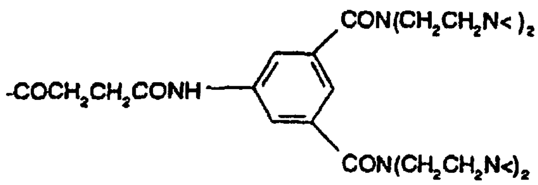

;

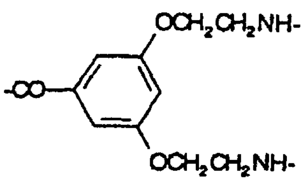

;

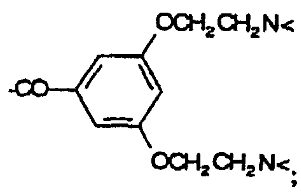

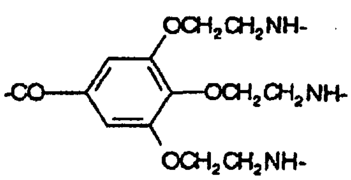

;

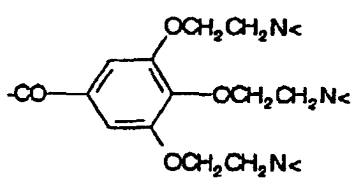

;

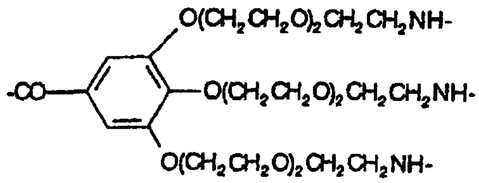
;
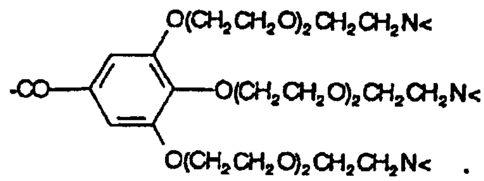
.

**9.** Cascade polymer complexes according to Claim 2, **characterized in that**

| | |
|---|---|
| m | is the numbers 1-3, |
| n | is the numbers 1-3, |
| o | is the number 1, |
| p | is the numbers 0-3, |
| M | is a $-CH_2-$, -CO- or $-CH_2CO-$ group and |
| $R^\circ$ | is a $-CH_2NU^1U^2$, $CH_3$ or $NO_2$ group. |

**10.** Pharmaceutical compositions comprising at least one cascade polymer complex according to Claim 1, where appropriate with the additions customary in pharmaceutical technology.

**11.** Use of at least one polymer complex according to Claim 1 for producing compositions for NMR or X-ray diagnosis.

**12.** Use of the cascade polymer complexes according to Claim 1 for producing compositions for differentiating benign and malignant tumours in body regions without a blood-brain barrier.

**13.** Process for the preparation of cascade polymer complexes according to Claims 1 to 9, **characterized in that** compounds of the general formula I'

$$A\text{-}\{X\text{-}[Y\text{-}(Z\text{-}<W\text{-}\beta_w>_z)_y]_x\}_a \qquad (I'),$$

in which

| | |
|---|---|
| A | is a nitrogen-containing cascade nucleus of base multiplicity a, |
| X and Y | are, independently of one another, a direct linkage or a cascade reproduction unit of reproduction multiplicity x and y respectively, |
| Z and W | are, independently of one another, a cascade reproduction unit of reproduction multiplicity z and w respectively, |
| a | is the numbers 2 to 12, |
| x, y, z and w | are, independently of one another, the numbers 1 to 4, and |
| β | is the binding site of the terminal NH groups of the last generation of the reproduction unit W, |

with the proviso that at least two reproduction units are different, that $16 \leq a \cdot x \cdot y \cdot z \cdot w \leq 64$ applies to the product of the multiplicities, and at least one of the cascade reproduction units X, Y, Z, W is a 1,4,7,10-tetraazacyclododecane or 1,4,8,11-tetraazacyclotetradecane reproduction unit,
are reacted with a complex or complexing agent K' of the general formula I'A, I'B or I'C

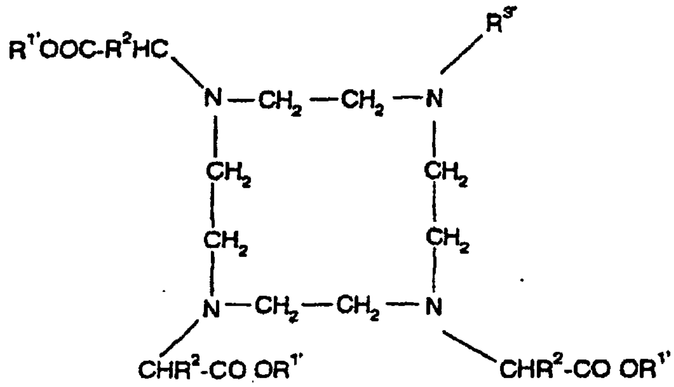

(I'A)

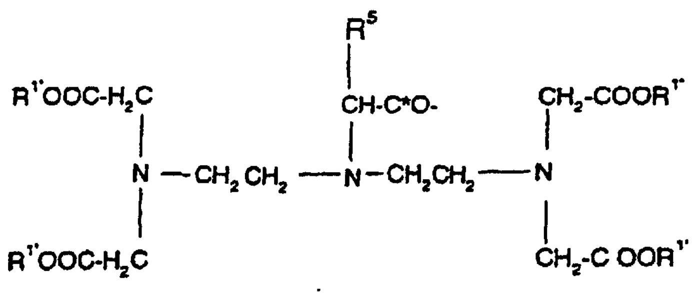

(I'B),

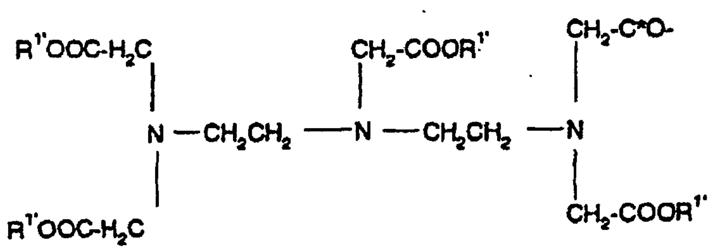

(I'C)

where

R1' is, independently of one another, a hydrogen atom, a metal ion equivalent to the atomic numbers 20-29, 39, 42-44 or 57-83 or an acid protective group,

$R^2$ is a hydrogen atom, a methyl or ethyl radical which is optionally substituted by 1-2 hydroxyl or 1 carboxy group(s),

$R^3$ is a

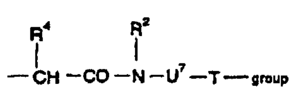

or a

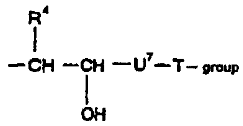

$R^4$ is a hydrogen atom or a straight-chain, branched, saturated or unsaturated $C_1$-$C_{30}$-alkyl chain which is optionally interrupted by 1-10 oxygen atoms, 1 phenylene, 1 phenyleneoxy groups and/or is optionally substituted by 1-5 hydroxyl, 1-3 carboxy, 1 phenyl group(s),

$R^5$ is a hydrogen atom or $R^4$,

$U^7$ is a straight-chain, branched, saturated or unsaturated $C_1$-$C_{20}$-alkylene group which optionally contains 1-5 imino, 1-3 phenylene, 1-3 phenyleneoxy, 1-3 phenyleneimino, 1-5 amide, 1-2 hydrazide, 1-5 carbonyl, 1-5 ethyleneoxy, 1 urea, 1 thiourea, 1-2 carboxyalkylimino, 1-2 ester groups, 1-10 oxygen, 1-5 sulphur and/or 1-5 nitrogen atom(s) and/or is optionally substituted by 1-5 hydroxyl, 1-2 mercapto, 1-5 oxo, 1-5 thioxo, 1-3 carboxy, 1-5 carboxyalkyl, 1-5 ester and/or 1-3 amino group(s), where the optionally present phenylene groups may be substituted by 1-2 carboxy, 1-2 sulphone or 1-2 hydroxyl groups,

T' is a -C*O, -COOH, -N=C=O or -N=C=S group and

C*O is an activated carboxyl group,

with the proviso that - where K' is a complex - at least two (in the case of divalent metals) or three (in the case of trivalent metals) of the substituents $R^1$ are a metal ion equivalent to the abovementioned elements, and that, if desired, further carboxyl groups are present in the form of their salts with inorganic and/or organic bases, amino acids or amino amides,
protective groups which are present where appropriate are eliminated, the cascade polymers obtained in this way are - where K' is a complexing agent - reacted in a manner known per se with at least one metal oxide or metal salt of an element of atomic numbers 20-29, 39, 42, 44 or 57-83 and, where appropriate, subsequently the acidic hydrogen atoms still present in the cascade polymer complexes obtained in this way are replaced wholly or partly by cations of inorganic and/or organic bases, amino acids or amino amides, and, where appropriate, free terminal amino groups which are still present are, if desired, acylated - before or after the metal complexation.

**14.** Compounds of the general formula I'A

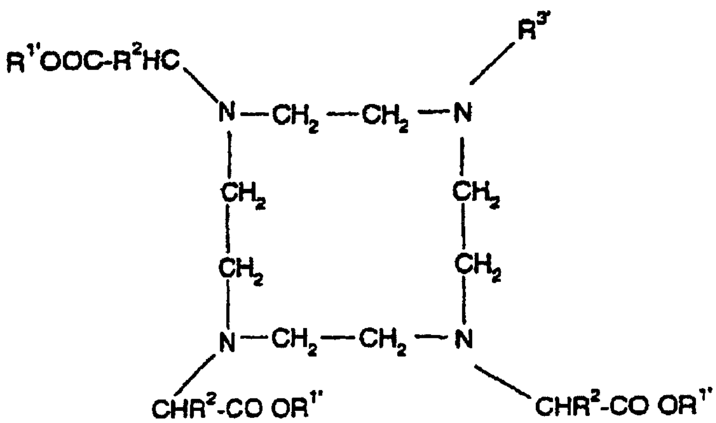

(I'A)

where

R1' is, independently of one another, a hydrogen atom, a metal ion equivalent to atomic numbers 20-29, 39, 42-44 or 57-83 or an acid protective group,

$R^2$ is a hydrogen atom, a methyl or ethyl radical which is optionally substituted by 1-2 hydroxyl or 1 carboxy group(s),

$R^{3'}$ is a

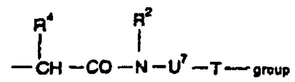

or a

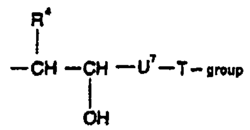

,

$R^4$ is a hydrogen atom or a straight-chain, branched, saturated or unsaturated $C_1$-$C_{30}$-alkyl chain which is optionally interrupted by 1-10 oxygen atoms, 1 phenylene, 1 phenyleneoxy group and/or is optionally substituted by 1-5 hydroxyl, 1-3 carboxy, 1 phenyl group(s),

$U^7$ is a straight-chain, branched, saturated or unsaturated $C_1$-$C_{20}$-alkylene group which optionally contains 1-5 imino, 1-3 phenylene, 1-3 phenyleneoxy, 1-3 phenyleneimino, 1-5 amide, 1-2 hydrazide 1-5 carbonyl, 1-5 ethyleneoxy, 1 urea, 1 thiourea, 1-2 carboxyalkylimino, 1-2 ester groups, 1-10 oxygen, 1-5 sulphur and/or 1-5 nitrogen atom(s) and/or is optionally substituted by 1-5 hydroxyl, 1-2 mercapto, 1-5 oxo, 1-5 thioxo, 1-3 carboxy, 1-5 carboxyalkyl, 1-5 ester and/or 1-3 amino group(s), where the optionally present phenylene groups may be substituted by 1-2 carboxy, 1-2 sulphone or 1-2 hydroxyl groups,

T' is a -C*O, -COOH, -N=C=O or -N=C=S group and

C*O is an activated carboxyl group.

**15.** Process for the production of the pharmaceutical compositions according to Claim 10, **characterized in that** the cascade polymer complex which is dissolved or suspended in water or physiological saline is converted into a form suitable for enteral or parenteral administration, where appropriate with the additions customary in pharmaceutical technology.

## Revendications

**1.** Complexes polymères en cascade comprenant

a) des ligands complexants de formule générale I

$$A\text{-}\{X\text{-}[Y\text{-}(Z\text{-}<W\text{-}K_w>_z)_y]_x\}_a \quad (I),$$

dans laquelle

A représente un noyau en cascade azoté de multiplicité de base a,

X et Y représentent, indépendamment l'un de l'autre, une liaison directe ou un motif de reproduction en cascade de multiplicité de reproduction x ou y respectivement,

Z et W représentent, indépendamment l'un de l'autre, un motif de reproduction en cascade de multiplicité de reproduction z ou w respectivement,

K représente le radical d'un agent complexant,

a représente les chiffres 2 à 12,

x, y, z et w représentent, indépendamment les uns des autres, les chiffres 1 à 4,

à condition qu'au moins deux motifs de reproduction soient différents, que le produit des multiplicités satisfasse à

$16 \leq a \cdot x \cdot y \cdot z \cdot w \leq 64$,

et qu'au moins l'un des motifs de reproduction en cascade X, Y, Z, W représente un motif de reproduction 1,4,7,10-tétraazacyclododécane ou 1,4,8,11-tétraazacyclotétradécane,

b) au moins 16 ions d'un élément des numéros atomiques 20 à 29, 39, 42, 44 ou 57 - 83,

c) éventuellement des cations de bases inorganiques et/ou organiques, d'aminoacides ou d'aminoamides ainsi que

d) éventuellement des groupes amino terminaux acylés.

2. Complexes polymères en cascade selon la revendication 1, **caractérisés en ce que** A représente un atome d'azote,

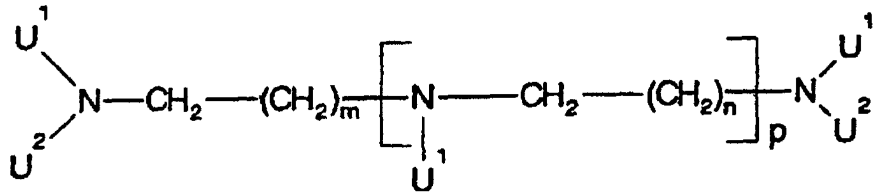

,

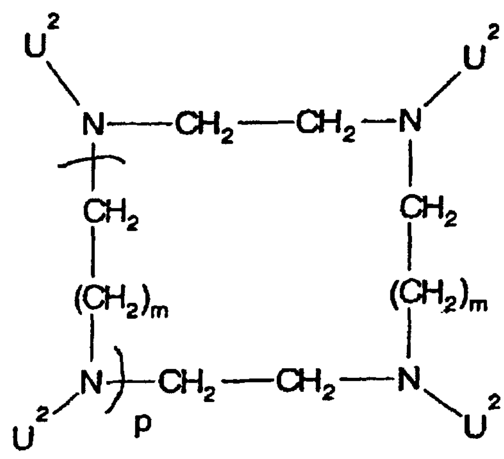

,

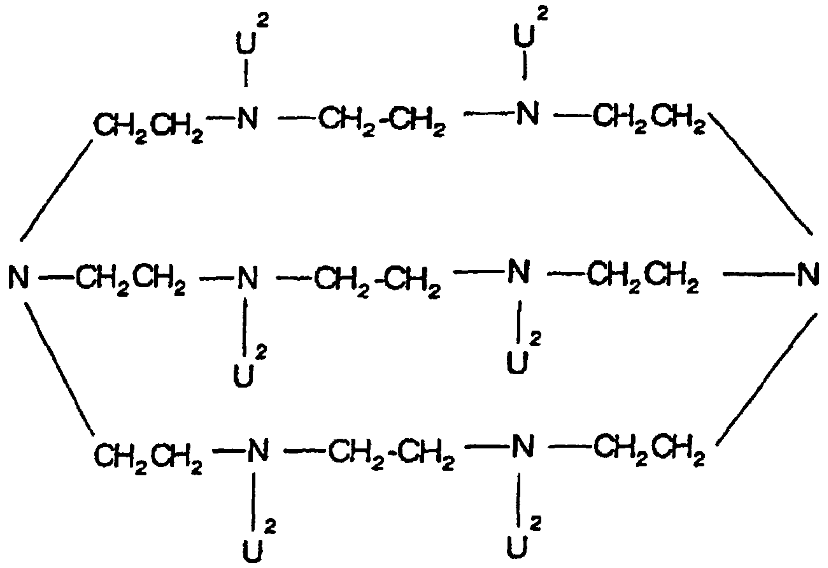

,

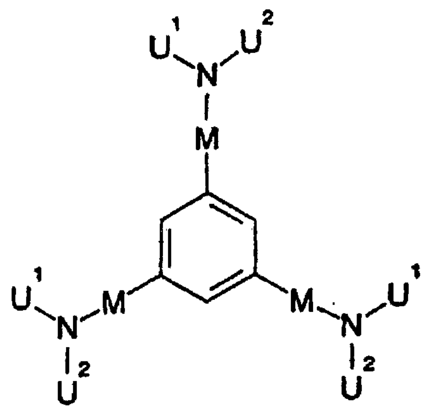

,

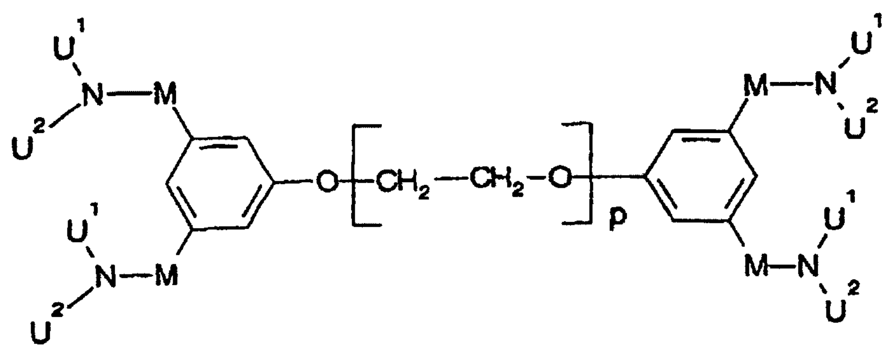

,

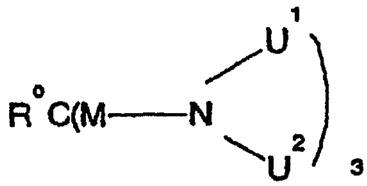

,

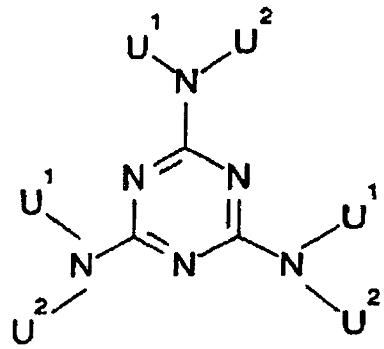

,

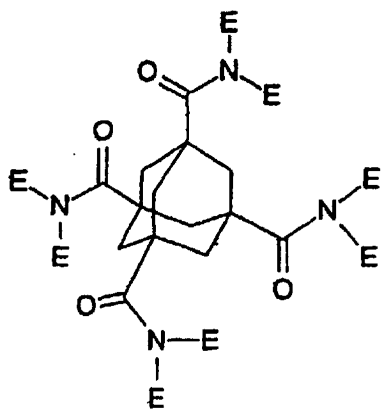

où

- m et n représentent les chiffres 1 à 10,
- p représente les chiffres 0 à 10,
- $U^1$ représente $Q^1$ ou E,
- $U^2$ représente $Q^2$ ou E où
- E représente le groupe

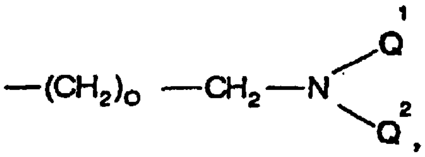,

où

o représente les chiffres 1 à 6,

$Q^1$ représente un atome d'hydrogène ou $Q^2$ et

$Q^2$ représente une liaison directe,

M représente une chaîne alkylène en $C_1$-$C_{10}$ qui est éventuellement interrompue par 1 à 3 atomes d'oxygène et/ou est éventuellement substituée par 1 à 2 groupes oxo,

$R^0$ représente un radical alkyle en $C_1$-$C_{10}$ ramifié ou non ramifié, un groupe nitro, amino, acide carboxylique ou

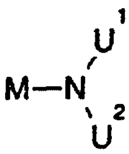

où
le nombre de $Q^2$ correspond à la multiplicité de base a.

**3.** Complexes polymères en cascade selon la revendication 1, **caractérisés en ce que** les motifs de reproduction en cascade X, Y, Z et W représentent, indépendamment les uns des autres,

E,

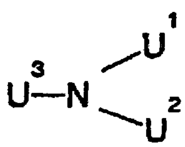,

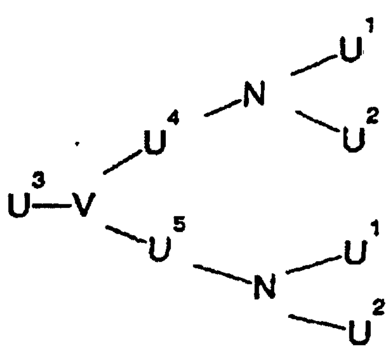,

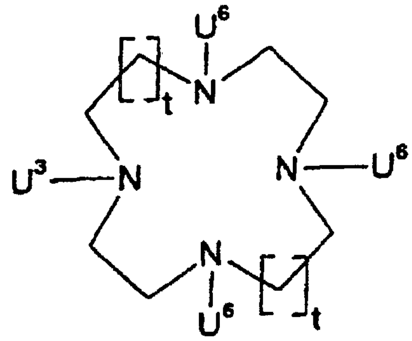

où t représente les chiffres 1 ou 2,

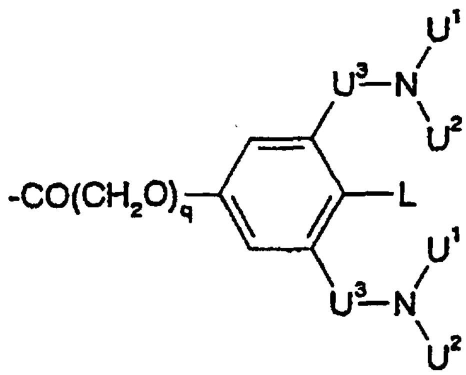

où

$U^1$ représente $Q^1$ ou E,

$U^2$ représente $Q^2$ ou E où

E représente le groupe

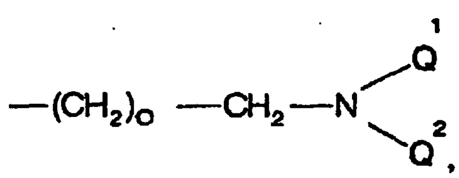

où

o représente les chiffres 1 à 6,

$Q^1$ représente un atome d'hydrogène ou $Q^2$,

$Q^2$ représente une liaison directe,

$U^3$ représente une chaîne $-NHCO-(CH_2)_o-$ ou une chaîne alkylène en $C_1$-$C_{20}$ qui est éventuellement interrompue par 1 à 10 atomes d'oxygène et/ou 1 à 2 radicaux $-N(CO)_q-R^2$, 1 à 2 radicaux phénylène et/ou 1 à 2 radicaux phénylène-oxy et/ou est éventuellement substituée par 1 à 2 groupes oxo, thioxo, carboxy, $C_1$-$C_5$-

alkylcarboxy, alcoxy en $C_1$-$C_5$, hydroxy, alkyle en $C_1$-$C_5$, où

q représente les chiffres 0 ou 1 et

$R^2$ représente un atome d'hydrogène, un radical méthyle ou éthyle qui est éventuellement substitué par 1 - 2 groupe(s) hydroxy ou 1 groupe carboxy,

L représente un atome d'hydrogène ou le groupe

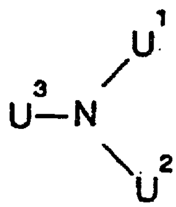

V représente le groupe méthine

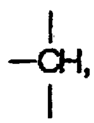

si $U^4$ représente simultanément une liaison directe ou le groupe M défini à la revendication 2 et $U^5$ a l'une des significations de $U^3$, ou

V représente le groupe

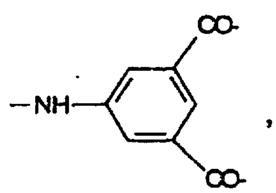

si $U^4$ et $U^5$ sont simultanément identiques et représentent la liaison directe ou le groupe M, et

$U^6$ représente le groupe

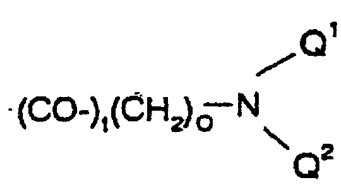

ou une liaison directe,

à condition qu'au moins l'un des motifs de reproduction en cascade représente un motif de reproduction 1,4,7,10-tétraazacyclododécane ou 1,4,8,11-tétraazacyclotétradécane indiqué ci-dessus.

**4.** Complexes polymères en cascade selon la revendication 1, **caractérisés en ce que** le radical d'agent complexant K lié aux atomes d'azote terminaux de la dernière génération du motif de reproduction W représente un radical

de formule générale IA , IB ou IC

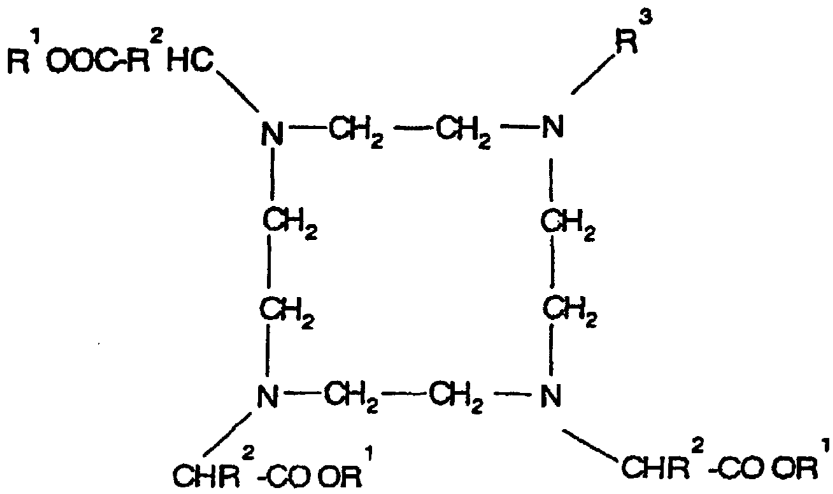

(IA),

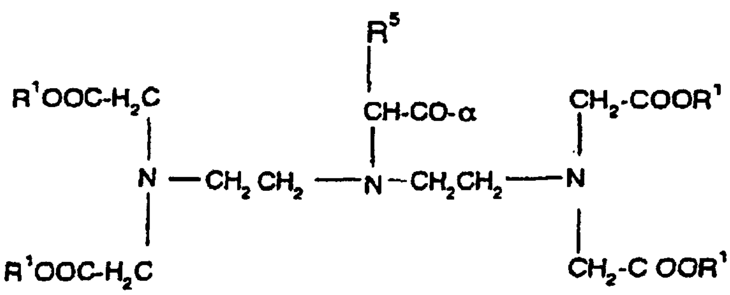

(IB),

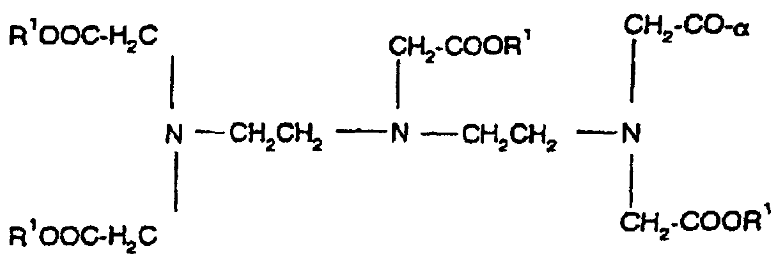

(IC),

dans lesquelles

$R^1$ représente indépendamment un atome d'hydrogène ou un équivalent d'ion métallique des numéros atomiques 20 - 29, 39, 42 - 44 ou 57 - 83,

$R^2$ représente un atome d'hydrogène, un radical méthyle ou éthyle qui est éventuellement substitué par 1 - 2 groupe(s) hydroxy ou 1 groupe carboxy,

$R^3$ représente un groupe

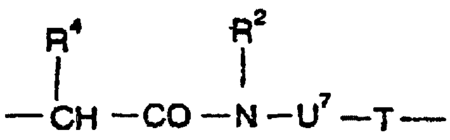

ou un groupe

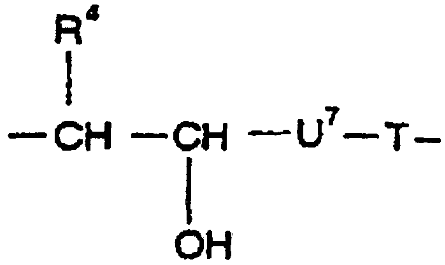

,

$R^4$ représente un atome d'hydrogène ou une chaîne alkyle en $C_1$-$C_{30}$ linéaire, ramifiée, saturée ou insaturée qui est éventuellement interrompue par 1 - 10 atomes d'oxygène, 1 groupe phénylène, 1 groupe phénylène-oxy et/ou éventuellement substituée par 1 - 5 groupe(s) hydroxy, 1 - 3 groupe(s) carboxy, 1 groupe phényle,

$R^5$ représente un atome d'hydrogène ou $R^4$,

$U^7$ représente un groupe alkylène en $C_1$-$C_{20}$ linéaire, ramifié, saturé ou insaturé, renfermant éventuellement 1 - 5 groupe(s) imino, 1 - 3 groupe(s) phénylène, 1 - 3 groupe(s) phénylène-oxy, 1 - 3 groupe(s) phénylène-imino, 1 - 5 groupe(s) amide, 1 - 2 groupe(s) hydrazide, 1 - 5 groupe(s) carbonyle, 1 - 5 groupe(s) éthylène-oxy, 1 groupe urée, 1 groupe thio-urée, 1 - 2 groupe(s) carboxyalkylimino, 1 - 2 groupe(s) ester, 1 - 10 atome (s) d'oxygène, 1 - 5 atome(s) de soufre et/ou 1 - 5 atome(s) d'azote et/ou éventuellement substitué par 1 - 5 groupe(s) hydroxy, 1 - 2 groupe(s) mercapto, 1 - 5 groupe(s) oxo, 1 - 5 groupe(s) thioxo, 1 - 3 groupe(s) carboxy, 1 - 5 groupe(s) carboxyalkyle, 1 - 5 groupe(s) ester et/ou 1 - 3 groupe(s) amino, les groupes phénylène éventuellement présents pouvant être substitués par 1 - 2 groupe(s) carboxy, 1 - 2 groupe(s) sulfone ou 1 - 2 groupe(s) hydroxy,

T représente un groupe -CO-$\alpha$, -NHCO-$\alpha$ ou -NHCS-$\alpha$ et

$\alpha$ représente la position de liaison aux atomes d'azote terminaux de la dernière génération du motif de reproduction W.

**5.** Complexes polymères en cascade selon la revendication 4, **caractérisés en ce que** la chaîne alkylène en $C_1$-$C_{20}$ représentant $U^7$ renferme les groupes
$-CH_2-$, $-CH_2-NHCO-$, $-NHCOCH_2O-$, $-NHCOCH_2OC_6H_4-$, $-N(CH_2CO_2H)-$, $-NHCOCH_2C_6H_4-$, $-NHCSNHC_6H_4-$, $-CH_2OC_6H_4-$, $-CH_2CH_2O-$, et/ou est substituée par les groupes $-COOH$, $-CH_2COOH$.

**6.** Complexes polymères en cascade selon la revendication 4, **caractérisés en ce que** $U^7$ représente un groupe $-CH_2-$, $-CH_2CH_2-$, $-CH_2CH_2CH_2-$, $-C_6H_4-$, $-C_6H_{10}-$, $-CH_2C_6H_5-$, $-CH_2NHCOCH_2CH(CH_2CO_2H)-C_6H_4-$, $-CH_2NHCOCH_2OCH_2-$, $-CH_2NHCOCH_2C_6H_4-$.

**7.** Complexes polymères en cascade selon la revendication 3, **caractérisés en ce que** le radical $U^3$ présent dans les motifs de reproduction en cascade X, Y, Z et W représente
$-CO-$, $-COCH_2OCH_2CO-$, $-COCH_2-$, $-CH_2CH_2-$, $-CONHC_6H_4-$, $-NHCOCH_2-$, $-COCH_2CH_2CO-$, $-COCH_2-CH_2CH_2CO-$, $-COCH_2CH_2CH_2CH_2CO-$, $-CONHCH_2CH_2NHCOCH_2CH_2CO-$, $-COCH_2CH_2NHCOCH_2CH_2CO-$,
le radical $U^4$ représente une liaison directe, $-CH_2CO-$,
le radical $U^5$ représente une liaison directe, $-(CH_2)_4-$, $-CH_2CO-$, $-CH(COOH)-$, $CH_2OCH_2CH_2-$, $-CH_2C_6H_4-$, $CH_2-C_6H_4OCH_2CH_2-$,
le radical E représente un groupe

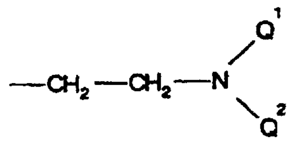

**8.** Complexes polymères en cascade selon la revendication 3, **caractérisés en ce que** les motifs de reproduction en cascade X, Y, Z et W représentent, indépendamment les uns des autres,

$-CH_2CH_2NH-$; $-CH_2CH_2N<$;
$-COCH(NH-)(CH_2)_4NH-$; $-COCH(N<)(CH_2)_4N<$;
$-COCH_2OCH_2CON(CH_2CH_2NH-)_2$; $-COCH_2OCH_2CON(CH_2CH_2N<)_2$;
$-COCH_2N(CH_2CH_2NH-)_2$; $-COCH_2N(CH_2CH_2N<)_2$;
$-COCH_2NH-$; $-COCH_2N<$;
$-COCH_2CH_2CON(CH_2CH_2NH-)_2$; $-COCH_2CH_2CON(CH_2CH_2N<)_2$;
$-COCH_2OCH_2CONH-C_6H_4-CH[CH_2CON(CH_2CH_2NH-)_2]_2$;
$-COCH_2OCH_2CONH-C_6H_4-CH[CH_2CON(CH_2CH_2N<)_2]_2$;
$-COCH_2CH_2CO-NH-C_6H_4-CH[CH_2CON(CH_2CH_2NH-)_2]_2$;
$-COCH_2CH_2CO-NH-C_6H_4-CH[CH_2CON(CH_2CH_2N<)_2]_2$;
$-CONHC_6H_4-CH[CH_2CON(CH_2CH_2NH-)_2]_2$;
$-CONHC_6H_4-CH[CH_2CON(CH_2CH_2NH<)_2]_2$;
$-COCH(NH-)CH(COOH)NH-$; $-COCH(N<)CH(COOH)N<$;

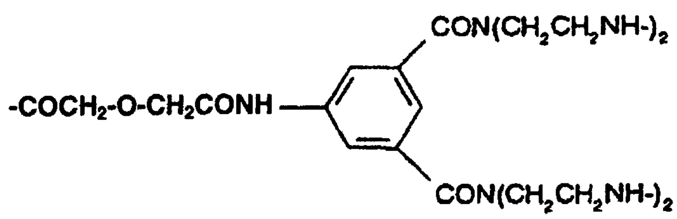

;

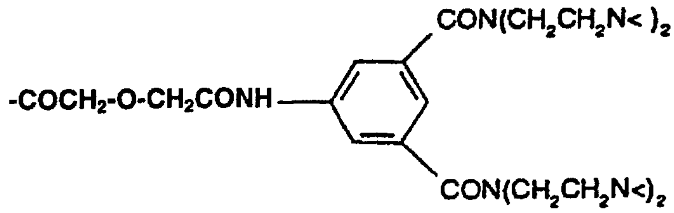

;

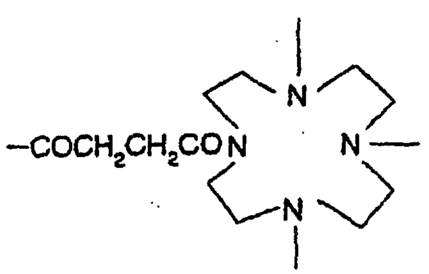

;

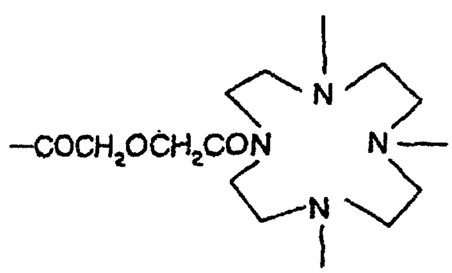

;

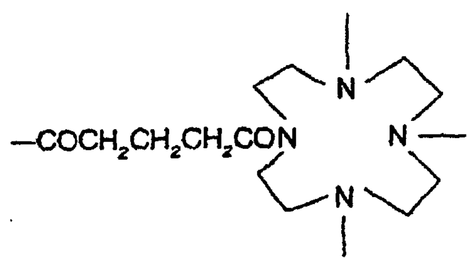
,

$-COCH(CH_2CH_2CH_2-N\langle)_{2}$; $-COCH(CH_2CH_2-N\langle)_{2}$;

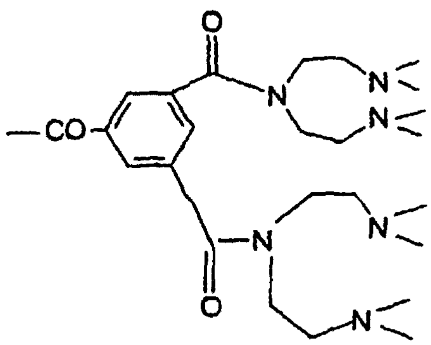
;

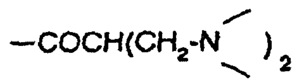
;

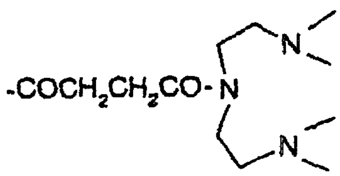
;

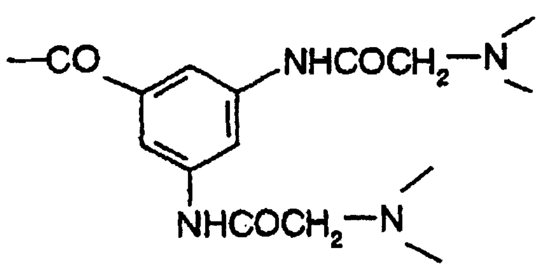

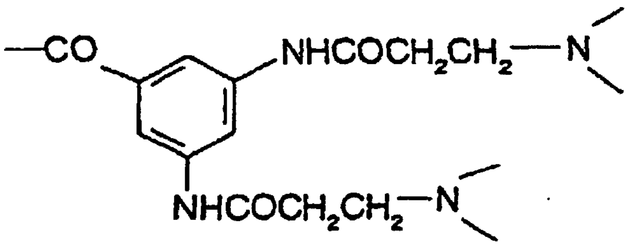

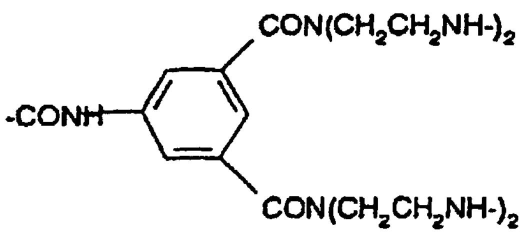

;

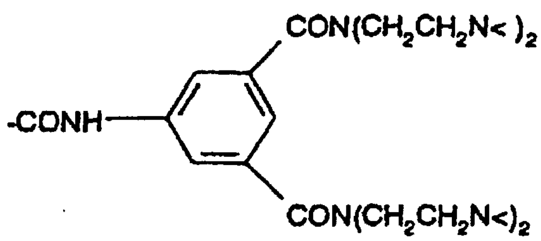

;

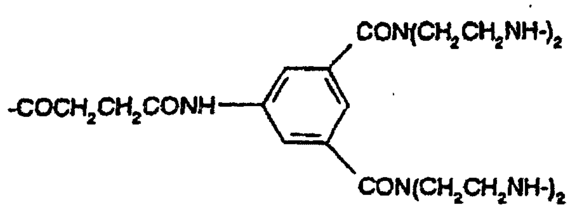

;

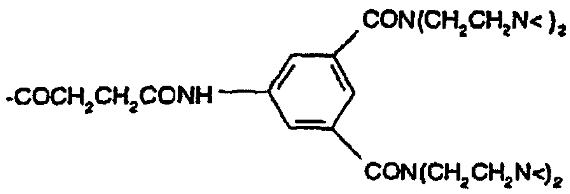

;

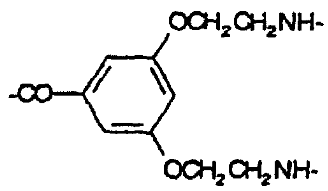

;

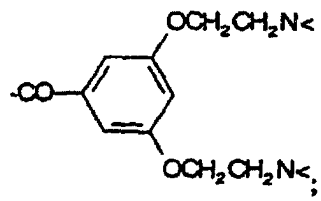

;

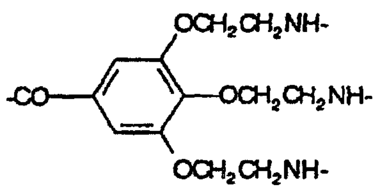

;

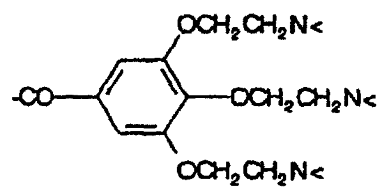

;

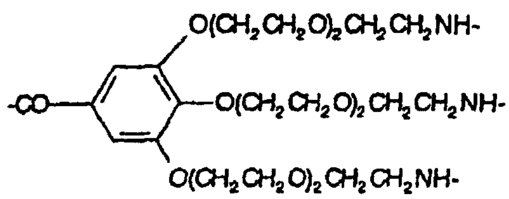

;

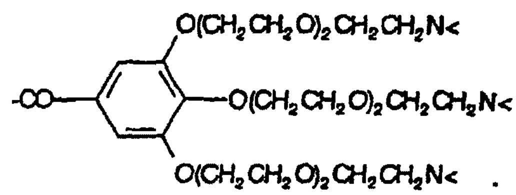

.

**9.** Complexes polymères en cascade selon la revendication 2, **caractérisés en ce que**

m représente les chiffres 1 - 3,

n représente les chiffres 1 - 3,

o représente le chiffre 1,

p représente les chiffres 0 - 3,

M représente un groupe $-CH_2$, -CO ou $-CH_2CO$ et

$R^0$ représente un groupe $-CH_2NU^1U^2$, $CH_3$ ou $NO_2$.

**10.** Compositions pharmaceutiques comprenant au moins un complexe polymère en cascade selon la revendication 1, éventuellement avec les additifs habituels en galénique.

**11.** Utilisation d'au moins un complexe polymère selon la revendication 1 pour la préparation d'agents destinés au diagnostic par RMN ou par rayons X.

**12.** Utilisation des complexes polymères en cascade selon la revendication 1 pour la préparation de compositions destinées à la différenciation de tumeurs bénignes et malignes dans des régions corporelles sans barrière hémato-encéphalique.

**13.** Procédé de préparation de complexes polymères en cascade selon les revendications 1 à 9, **caractérisé en ce que** l'on fait réagir des composés de formule générale I'

$$A\text{-}\{X\text{-}[Y\text{-}(Z\text{-}<W\text{-}\beta_w>_z)_y]_x\}_a \quad (I'),$$

dans laquelle

| | |
|---|---|
| A | représente un noyau en cascade azoté de multiplicité de base a, |
| X et Y | représentent, indépendamment l'un de l'autre, une liaison directe ou un motif de reproduction en cascade de multiplicité de reproduction x ou y respectivement, |
| Z et W | représentent, indépendamment l'un de l'autre, un motif de reproduction en cascade de multiplicité de reproduction z ou w respectivement, |
| a | représente les chiffres 2 à 12, |
| x, y, z et w | représentent, indépendamment les uns des autres, les chiffres 1 à 4, et |
| β | représente la position de liaison des groupes NH terminaux de la dernière génération du motif de reproduction W, |

à condition qu'au moins deux motifs de reproduction soient différents, que le produit des multiplicités satisfasse à

$$16 \leq a \cdot x \cdot y \cdot z \cdot w \leq 64,$$

et qu'au moins l'un des motifs de reproduction en cascade X, Y, Z, W représente un motif de reproduction 1,4,7,10-tétraazacyclododécane ou 1,4,8,11-tétraazacyclotétradécane,
avec un complexe ou un agent complexant K' de formule générale I'A, I'B ou I'C

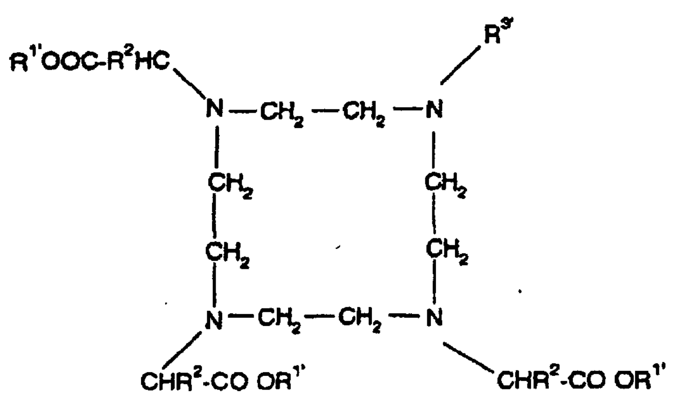

(I'A)

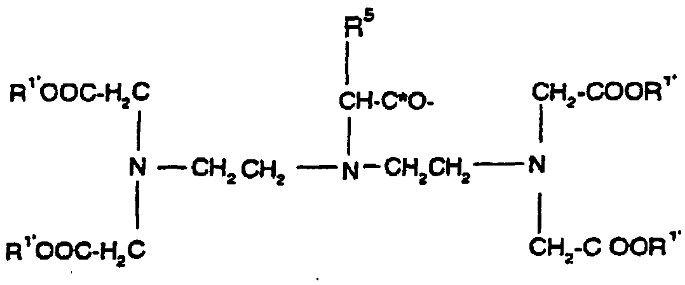

(I'B),

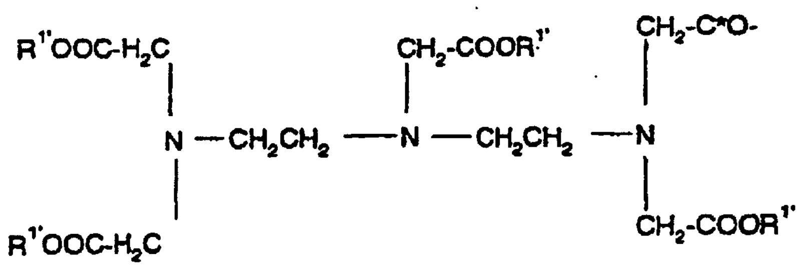

(I'C)

dans lesquelles

$R^{1'}$ représente indépendamment un atome d'hydrogène, un équivalent d'ion métallique des numéros atomiques 20 - 29, 39, 42 - 44 ou 57 - 83 ou un groupe protecteur d'acides,

$R^2$ représente un atome d'hydrogène, un radical méthyle ou éthyle qui est éventuellement substitué par 1 - 2 groupe(s) hydroxy ou 1 groupe carboxy,

$R^{3'}$ représente un groupe

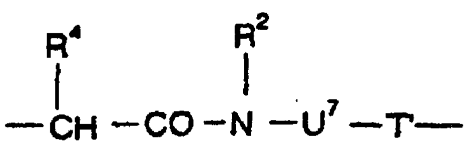

ou un groupe

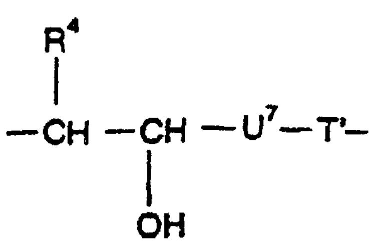

,

$R^4$ représente un atome d'hydrogène ou une chaîne alkyle en $C_1$-$C_{30}$ linéaire, ramifiée, saturée ou insaturée qui est éventuellement interrompue par 1 - 10 atomes d'oxygène, 1 groupe phénylène, 1 groupe phénylèneoxy et/ou éventuellement substituée par 1 - 5 groupe(s) hydroxy, 1 - 3 groupe(s) carboxy, 1 groupe phényle,

$R^5$ représente un atome d'hydrogène ou $R^4$,

$U^7$ représente un groupe alkylène en $C_1$-$C_{20}$ linéaire, ramifié, saturé ou insaturé, renfermant éventuellement 1 - 5 groupe(s) imino, 1 - 3 groupe(s) phénylène, 1 - 3 groupe(s) phénylène-oxy, 1 - 3 groupe(s) phénylène-imino, 1 - 5 groupe(s) amide, 1 - 2 groupe(s) hydrazide, 1 - 5 groupe(s) carbonyle, 1 - 5 groupe(s) éthylène-oxy, 1 groupe urée, 1 groupe thio-urée, 1 - 2 groupe(s) carboxyalkylimino, 1 - 2 groupe(s) ester, 1 - 10 atome(s) d'oxygène, 1 - 5 atome(s) de soufre et/ou 1 - 5 atome(s) d'azote et/ou éventuellement substitué par 1 - 5 groupe(s) hydroxy, 1 - 2 groupe(s) mercapto, 1 - 5 groupe(s) oxo, 1 - 5 groupe(s) thioxo, 1 - 3 groupe(s) carboxy, 1 - 5 groupe(s) carboxyalkyle, 1 - 5 groupe(s) ester et/ou 1 - 3 groupe(s) amino, les groupes phénylène éventuellement présents pouvant être substitués par 1 - 2 groupe(s) carboxy, 1 - 2 groupe(s) sulfone ou 1 - 2 groupe(s) hydroxy,

T' représente un groupe -C*O, -COOH, -N=C=O ou -N=C=S et

C*O représente un groupe carboxy activé,
à condition que - dans la mesure où K' représente un complexe - au moins deux (dans le cas de métaux bivalents) ou trois (dans le cas de métaux trivalents) des substituants $R^1$ représentent un équivalent d'ion métallique des éléments susmentionnés et que, si on le souhaite, d'autres groupes carboxy soient présents sous forme de leurs sels avec des bases inorganiques et/ou organiques, des aminoacides ou des aminoamides,

les groupes protecteurs éventuellement présents sont éliminés, les polymères en cascade ainsi obtenus - dans la mesure où K' représente un agent complexant - sont mis à réagir, de manière connue en soi, avec au moins un oxyde métallique ou sel métallique d'un élément des numéros atomiques 20 - 29, 39, 42, 44 ou 57 - 83 et ensuite, éventuellement, les atomes d'hydrogène acides encore présents dans les complexes polymères en cascade ainsi obtenus sont substitués, totalement ou partiellement, par des cations de bases inorganiques et/ou organiques, d'aminoacides ou d'aminoamides, et les groupes amino terminaux libres éventuellement encore présents sont acylés, si on le souhaite, avant ou après la complexation métallique.

**14.** Composés de formule générale I'A

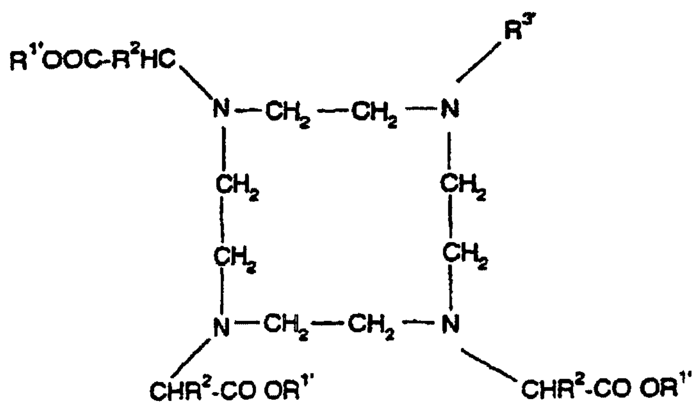

(I'A)

dans laquelle

$R^{1'}$ représente indépendamment un atome d'hydrogène, un équivalent d'ion métallique des numéros atomiques 20 - 29, 39, 42 - 44 ou 57 - 83 ou un groupe protecteur d'acides,

$R^2$ représente un atome d'hydrogène, un radical méthyle ou éthyle qui est éventuellement substitué par 1 - 2 groupe(s) hydroxy ou 1 groupe carboxy,

$R^{3'}$ représente un groupe

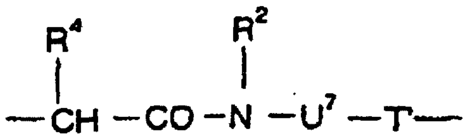

ou un groupe

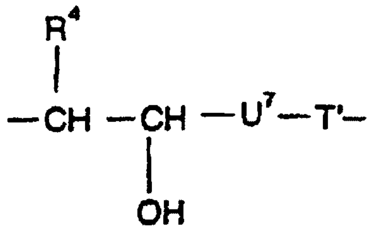

,

$R^4$ représente un atome d'hydrogène ou une chaîne alkyle en $C_1$-$C_{30}$ linéaire, ramifiée, saturée ou insaturée qui est éventuellement interrompue par 1 - 10 atomes d'oxygène, 1 groupe phénylène, 1 groupe phénylène-oxy et/ou éventuellement substituée par 1 - 5 groupe(s) hydroxy, 1 - 3 groupe(s) carboxy, 1 groupe phényle,

$U^7$ représente un groupe alkylène en $C_1$-$C_{20}$ linéaire, ramifié, saturé ou insaturé, renfermant éventuellement 1 - 5 groupe(s) imino, 1 - 3 groupe(s) phénylène, 1 - 3 groupe(s) phénylène-oxy, 1 - 3 groupe(s) phénylène-imino, 1 - 5 groupe(s) amide, 1 - 2 groupe(s) hydrazide, 1 - 5 groupe(s) carbonyle, 1 - 5 groupe(s) éthylène-oxy, 1 groupe urée, 1 groupe thio-urée, 1 - 2 groupe(s) carboxyalkylimino, 1 - 2 groupe(s) ester, 1 - 10 atome(s) d'oxygène, 1 - 5 atome(s) de soufre et/ou 1 - 5 atome(s) d'azote et/ou éventuellement substitué par 1 - 5 groupe(s) hydroxy, 1 - 2 groupe(s) mercapto, 1 - 5 groupe(s) oxo, 1 - 5 groupe(s) thioxo, 1 - 3 groupe(s) carboxy, 1 - 5 groupe(s) carboxyalkyle, 1 - 5 groupe(s) ester et/ou 1 - 3 groupe(s) amino, les groupes phénylène éventuellement présents pouvant être substitués par 1 - 2 groupe(s) carboxy, 1 - 2 groupe(s) sulfone ou 1 - 2 groupe(s) hydroxy,

T' représente un groupe -C-*O, -COOH, -N=C=O ou -N=C=S et

C*O représente un groupe carboxy activé.

**15.** Procédé de préparation des compositions pharmaceutiques selon la revendication 10, **caractérisé en ce que** le complexe polymère en cascade dissout ou mis en suspension dans de l'eau ou dans une solution de sel physiologique, éventuellement avec les additifs habituels en galénique, est mis sous une forme appropriée pour l'application par voie entérale ou parentérale.